(19) Europäisches Patentamt
European Patent Office
Office européen des brevets

(11) EP 1 514 597 A1

(12) **EUROPEAN PATENT APPLICATION**
published in accordance with Art. 158(3) EPC

(43) Date of publication:
**16.03.2005 Bulletin 2005/11**

(21) Application number: **03730731.1**

(22) Date of filing: **30.05.2003**

(51) Int Cl.[7]: **B01J 20/26**, B01D 71/82, B01D 69/08, G01N 30/48

(86) International application number:
**PCT/JP2003/006840**

(87) International publication number:
**WO 2003/101611 (11.12.2003 Gazette 2003/50)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **31.05.2002 JP 2002159728**

(71) Applicants:
- **Japan Envirochemicals, Ltd.**
  **Osaka-shi, Osaka 541-0045 (JP)**
- **ENVIRONMENT PURIFICATION RESEARCH INSTITUTE INC.**
  **Takasaki-shi, Gunma 370-0833 (JP)**

(72) Inventors:
- **SAITO, Kyoichi**
  **Faculty of Engineering, Chiba Uni.**
  **Chiba-shi, Chiba 263-0022 (JP)**
- **SHIRAISHI, Tomofumi, Environment Purification**
  **Takasaki-shi Gunma 370-0833 (JP)**
- **GODA, Yasuhiro, Japan EnviroChemicals, Ltd.**
  **Osaka-shi, Osaka 532-0044 (JP)**

(74) Representative: **von Kreisler Selting Werner**
**Patentanwälte**
**P.O. Box 10 22 41**
**50462 Köln (DE)**

(54) **ISOLATION MATERIAL FOR HORMONE DISRUPTING SUBSTANCE, METHOD OF CONCENTRATING AND METHOD OF CLEAN-UP**

(57) The present invention relates to an isolation material for an endocrine disruptor, which is prepared by immobilizing an anti-endocrine disruptor antibody to a graft-chain-containing carrier made of a polymeric material via covalent binding. According to the present invention, an isolation material for an endocrine disruptor, which is capable of specific and efficient isolation of an endocrine disruptor, and an endocrine disruptor concentration and a clean-up method capable of specific and efficient concentration or clean-up of an endocrine disruptor at a high rate can be afforded.

FIG. 1

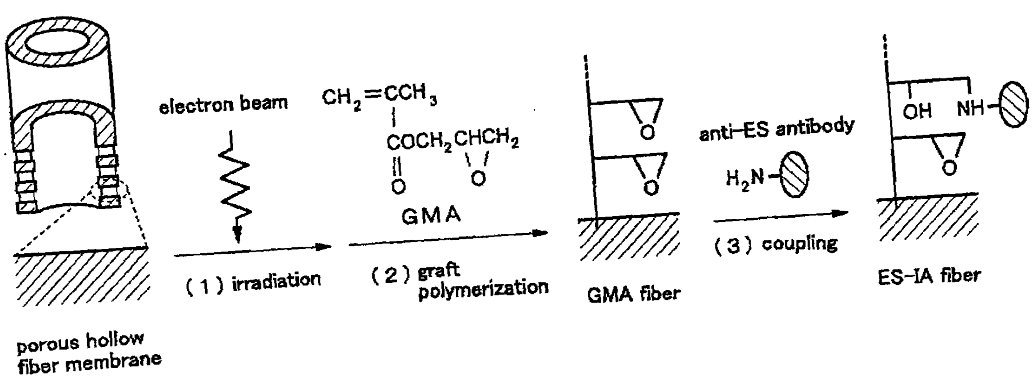


EP 1 514 597 A1

## Description

### Technical Field

The present invention relates to an isolation material for endocrine disruptor, concentration method and clean-up (isolation and removal) method.

### Background Art

Environmental hormones are also referred to as "endocrine disruptors" or "endocrine disrupting chemicals", and recently a social problem has been caused by environmental pollution due to them. Therefore, it is necessary to determine and analyze environmental pollutants and their degradation products in an environment, and to utilize the results for environmental preservation.

As methods of measuring and analyzing such endocrine disruptors (including degradation products thereof), various methods have conventionally been known. For example, high performance liquid chromatography (HPLC), gas chromatography (GC), gas chromatography-high resolution mass spectrometry (GC-HRMS), high performance liquid chromatography-high resolution mass spectrometry (HPLC-HRMS) and the like for quantitatively measuring endocrine disruptors in tap water, river water, lake water or sewage water are available.

Also, because endocrine disruptors occur only in very trace amounts, to quantify those substances, it is necessary to concentrate those substances at high rates, and it has been difficult to selectively and efficiently clean up those substances. However, it has been impossible to increase the concentration rate beyond some extent by conventional methods such as solvent extraction and solid phase extraction because of the abundance of impurities, and it has been impossible to selectively and efficiently clean up those substances by conventional clean-up methods using an adsorbent such as activated charcoal. In contrast, antibody-using concentration methods and clean-up methods have no such problems because they utilize antigen-antibody reaction.

However, to date, no isolation materials capable of selectively and efficiently isolating an endocrine disruptor have been obtained, and no immunological concentration methods or clean-up methods capable of selectively and efficiently concentrating an endocrine disruptor at a high rate have been established.

### Disclosure of the Invention

The object of the present invention is to provide an isolation material for endocrine disruptor capable of selectively and efficiently isolating an endocrine disruptor, and an endocrine disruptor concentration and clean-up method capable of selectively and efficiently concentrating or cleaning up an endocrine disruptor at high rate.

The present inventors conducted extensive investigations with the aim of solving the above-described problems, found that an endocrine disruptor can be isolated selectively and efficiently by an isolation material comprising a carrier made of a polymeric material carrying an antibody against an endocrine disruptor, and that by using said isolation material, an endocrine disruptor can be selectively and efficiently concentrated or cleaned up at high rates, and developed the present invention.

That is, the present invention is as follows.

[1] An isolation material for an endocrine disruptor, which comprises a carrier made of a polymeric material being bound covalently with an anti-endocrine disruptor antibody via a graft chain of the carrier made of a polymeric material.

[2] The isolation material described in [1] above, wherein the carrier made of a polymeric material is a plain membrane, a hollow fiber membrane, a non-woven fabric, a woven fabric, a fiber or a molded bead.

[3] The isolation material described in [2] above, wherein the carrier made of a polymeric material is a porous hollow fiber membrane or a non-woven fabric.

[4] The isolation material described in [3] above, wherein the carrier made of a polymeric material is a porous hollow fiber membrane.

[5] The isolation material described in [4] above, wherein the porous hollow fiber membrane has a pore diameter of 0.05 to 100 $\mu$m and a void ratio of 20 to 80%.

[6] The isolation material described in [3] above, wherein the carrier made of a polymeric material is a non-woven fabric.

[7] The isolation material described in [6] above, wherein the non-woven fabric has a fabric weight of 10 to 200 $g/m^2$.

[8] The isolation material described in any one of [1] to [7] above, wherein the graft ratio is 10 to 300%.

[9] The isolation material described in any one of [1] to [8] above, wherein the anti-endocrine disruptor antibody is a polyclonal antibody or recombinant antibody.

[10] The isolation material described in any one of [1] to [9] above, wherein the endocrine disruptor is an alkylphenol, an alkylphenol ethoxylate, a resin component, a resin plasticizer, a chlorophenol, a female sex hormone, a male sex hormone or a thyroid hormone.

[11] An endocrine disruptor concentration method comprising:

(1) a step wherein an endocrine disruptor-containing sample is brought into contact with the isolation material described in any one of [1] to [10] above to allow said endocrine disruptor to be captured by an anti-endocrine disruptor antibody, and
(2) a step wherein said endocrine disruptor captured by said anti-endocrine disruptor antibody is dissociated and recovered.

[12] The method described in [11] above, wherein the endocrine disruptor is an alkylphenol, an alkylphenol ethoxylate, a resin component, a resin plasticizer, a chlorophenol, a female sex hormone, a male sex hormone or a thyroid hormone.

[13] An endocrine disruptor clean-up method wherein an endocrine disruptor-containing sample is brought into contact with the isolation material described in any one of [1] to
[10] above to allow said endocrine disruptor to be captured by an anti-endocrine disruptor antibody.

[14] The method described in [13] above, wherein the endocrine disruptor is an alkylphenol, an alkylphenol ethoxylate, a resin component, a resin plasticizer, a chlorophenol, a female sex hormone, a male sex hormone or a thyroid hormone.

## Brief Description of the Drawings

Figure 1 shows a production scheme for an anti-ES antibody immobilized porous hollow fiber membrane.
Figure 2 shows an experimental apparatus for E2 recovery using ES-IA fiber.
Figure 3 shows the amount of anti-ES antibody immobilized versus coupling time.
Figure 4 shows a breakthrough curve of E2 for various amounts of anti-ES antibody immobilized.
Figure 5 shows the amount of E2 bound versus the amount of immobilized anti-ES antibody.
Figure 6 shows the dependency of the breakthrough curve upon the permeation rate of an E2 solution passing through the pores of ES-IA (4.8) fiber.
Figure 7 shows the elution rate as a function of the amount of anti-ES antibody immobilized on a porous hollow fiber membrane.
Figure 8 shows a production scheme for an anti-ES antibody immobilized non-woven fabric.
Figure 9 shows an outline of anti-ES antibody immobilization onto GMA non-woven fabric and a method of E2 recovery with an estrogen affinity column using a non-woven fabric.
Figure 10 shows the E2 elution pattern of each anti-estrogen antibody immobilized carrier.
Figure 11 shows an outline of a method of preparing a polyclonal antibody with reference to preparation of an anti-estrogen antibody as an example.
Figure 12 shows that the estradiol single-chain variable region antibody constructed in Reference Example 3 has bound to 17β-estradiol.
Figure 13 is a conceptual chart of the eight kinds of anti-endocrine disruptor single-chain variable region antibodies constructed in Reference Example 6.
Figure 14 is a Western blotting chart of the eight kinds of anti-endocrine disruptor single-chain variable region antibodies constructed in Reference Example 6.
Figure 15 is a chart comparing the performances of the eight kinds of anti-endocrine disruptor single-chain variable region antibodies constructed in Reference Example 6 by ELISA.

## Detailed Description of the Invention

The isolation material for endocrine disruptor of the present invention comprises a carrier made of a polymeric material being bound covalently with an anti-endocrine disruptor antibody via a graft chain of the carrier made of a polymeric material.

An endocrine disruptor in the present invention refers to an endogenous substance capable of influencing the normal. action of a hormone essentially carried on in the living body of an animal when incorporated therein. As examples of said endocrine disruptors, female sex hormone, male sex hormone, thyroid hormone, alkylphenol ethoxylate, alkylphenol, resin component, resin plasticizer, chlorophenol and other substances can be mentioned. In addition,

degradation products thereof are also included in the endocrine disruptors in the present invention, as long as they are adsorbed to the anti-endocrine disruptor antibody described below.

As examples of said female sex hormone, estrogen, estradiol (E2), estrone (E1), estriol (E3) and the like can be mentioned; as examples of said male sex hormone, androgen, testosterone, dehydroepiandrosterone, androstenedione and the like can be mentioned; as examples of said thyroid hormone, thyroxine (T3), triiodothyronine (T4) and the like can be mentioned; also, conjugates (for example, glucuronic acid conjugate, sulfuric acid conjugate and the like) that are in vivo metabolites thereof are also included therein.

As examples of said alkylphenol ethoxylate (APE), an alkylphenol ethoxylate (APE) represented by Formula (1):

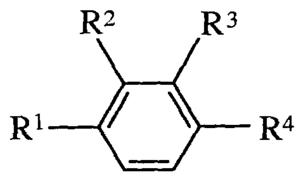

(1)

[wherein each of $R^1$, $R^2$ and $R^3$, whether identical or different, means H, a linear or branched (containing sec-, tert-, iso-) alkyl having 1 to 20, preferably 1 to 12, carbon atoms, $R^4$ means $(OC_2H_4)_mOH$ or $(OC_2H_4)_mCOOH$, and m means an average number of ethylene oxide chains of 1 to 70, preferably 1 to 15] [e.g., NPE (nonylphenol ethoxylate, for example, NP2EO (average number of ethylene oxide chains=2), NP5EO (average number of ethylene oxide chains=5), NP10EO (average number of ethylene oxide chains=10), NP10EC (average number of ethylene oxide chains=10, terminal OH→carboxylic acid), OPE (octylphenol ethoxylate) and the like] and the like can be mentioned.

As examples of said alkylphenol (AP), an alkylphenol (AP) represented by Formula (2):

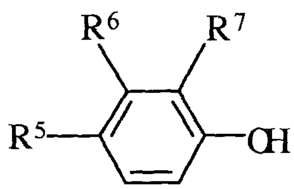

(2)

[wherein each of $R^5$, $R^6$ and $R^7$, whether identical or different, means H, a linear or branched (containing sec-, tert-, iso-) alkyl having 1 to 20, preferably 1 to 12, carbon atoms] [e.g., DP (4-dodecylphenol), EP (4-ethylphenol), HP (heptylphenol), IPP (4-isopentylphenol), 2-OP (2-octylphenol), 4-NP (4-nonylphenol), 4-OP (4-octylphenol), 4-sBP (4-sec-butylphenol), 4-tBP (4-t-butylphenol), 4-tPP (4-t-pentylphenol), 4-tOP (4-t-octylphenol) and the like] and the like can be mentioned.

As examples of said resin component (PRC), a resin component (PRC) represented by Formula (3):

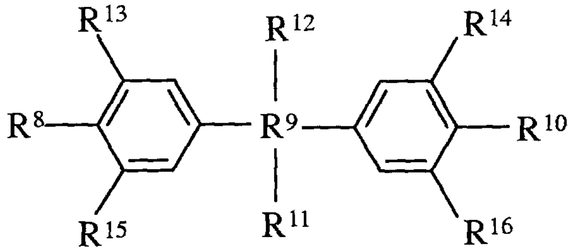

(3)

[wherein $R^9$ means C, CO or $SO_2$, each of $R^8$ and $R^{10}$, whether identical or different, means H, OH, $NH_2$ or $O(CH_2)_2OH$, each of $R^{11}$ and $R^{12}$, whether identical or different, means none, H, $CH_3$, $CH_2OH$ or $C_2H_4COOH$, and each of $R^{13}$, $R^{14}$, $R^{15}$ and $R^{16}$, whether identical or different, means H, OH, $CH_3$, Cl or Br] [e.g., BPA (bisphenol A), 4,4'-EBP (4,4'-ethylidenebisphenol), BHPM (bis(p-hydroxyphenyl)methane), 2,2'-BHPP (2,2'-bis(4-hydroxyphenyl)-1-propanol), 2,2'-BMHPP (2,2'-bis(m-methyl-p-hydroxyphenyl)propane), 4,4'-BHPP (4, 4'-bis (p-hydroxyphenyl)pentanoic acid), 4,4'-DDM (4,4'-diaminodiphenylmethane), 4,4'-DDE (4,4'-dihydroxydiphenyl ether), 4,4'-DOHDS (4,4'-dihydroxydiphenylsulfone), 4,4'-DClDS (4,4'-dichlorodiphenylsulfone), BHEDBrPS (bis[4-(2-hydroxyethoxy)-3,5-dibromophenyl]sulfone), BHEPS (bis[4-(2-hydroxyethoxy)phenyl]sulfone), 4, 4' -DDE (4,4'-dihydroxydiphenyl ether), p,p'-DBP (p,p'-dihydroxybenzophenone), HBP (4-hydroxybiphenyl) and the like] and the like can be mentioned.

As examples of said resin plasticizer (PP), a resin plasticizer represented by Formula (4):

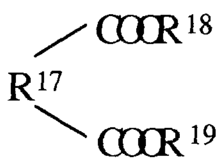

(4)

[wherein $R^{17}$ means o-phenylene or tetramethylene, each of $R^{18}$ and $R^{19}$, whether identical or different, means H, a linear or branched (containing sec-, tert-, iso-) alkyl having 1 to 20, preferably 1 to 12, carbon atoms, benzyl or cyclohexyl] [e.g., BBP (butylbenzyl phthalate), DBP (dibutyl phthalate), DCHP (dicyclohexyl phthalate), DEP (diethyl phthalate), DEHP (di(2-ethylhexyl)phthalate), DEHA (diethylhexyl adipate), DHP (dihexyl phthalate), DPP (di-n-pentyl phthalate), DPrP (dipropyl phthalate) and the like] and the like can be mentioned.

As examples of said chlorophenol (CP), a chlorophenol (CP) represented by Formula (5):

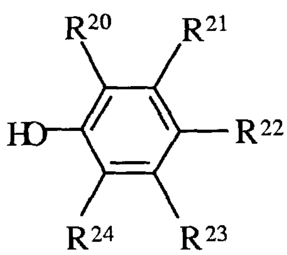

(5)

[wherein each of $R^{20}$, $R^{21}$, $R^{22}$, $R^{23}$ and $R^{24}$, whether identical or different, means H or Cl] [e.g., 2-CP (2-chlorophenol), 3-CP (3-chlorophenol), 4-CP (4-chlorophenol), 2,3-CP (2,3-dichlorophenol), 2,4-CP (2,4-dichlorophenol), 2,5-CP (2,5-dichlorophenol), 2,6-CP (2,6-dichlorophenol), 2,3,4-CP (2,3,4-trichlorophenol), 2,4,5-CP (2,4,5-trichlorophenol), 2,4,6-CP (2,4,6-trichlorophenol), 2,3,4,5-CP (2,3,4,5-tetrachlorophenol), 2,3,4,6-CP (2,3,4,6-tetrachlorophenol), PCP (pentachlorophenol) and the like] and the like can be mentioned.

As said other substances, polychlorodibenzo-p-dioxyn (PCDD), which is represented by 2,3,7,8-tetrachlorodibenzo-p-dioxyn (TCDD), polychlorodibenzofuran (PCDF), which is represented by 2, 3, 7, 8-tetrachlorodibenzofuran (TCDF), polychlorobiphenyl (PCB), benzophenone, benzopyran, chlorobenzene, bromonaphthol, nitrotoluene, tributyl tin, various agricultural chemicals, heavy metals (for example, Cd, Hg, Pb and the like), synthetic estrogens (e.g., centochromane, ethynylestradiol, diethylstilbestrol, hexestrol, 2-hydroxyestradiol, tamoxifen, raloxifene and the like), foods, food additives (for example, BHA (butylhydroxyanisole), equol, enterolactone, phytoestrogen, coumestrol, formononetin, daidzein, biochanin A, genistein and the like) and the like can be mentioned.

Referring to the above, as specific examples of the linear or branched alkyl having 1 to 20 carbon atoms, methyl, ethyl, propyl, iso-propyl, butyl, iso-butyl, sec-butyl, tert-butyl, n-pentyl, iso-pentyl, neo-pentyl, tert-pentyl, n-hexyl, iso-hexyl, n-heptyl, n-octyl, n-nonyl, n-decyl, undecyl, dodecyl, tridecyl, tetradecyl, pentadecyl, hexadecyl, heptadecyl, octadecyl, nonadecyl, icosyl and the like can be mentioned. As specific examples of the aforementioned alkyl having 2 to 18 carbon atoms, those other than methyl, nonadecyl and icosyl, among the above-described specific examples, can be mentioned.

An anti-endocrine disruptor antibody in the present invention refers to a protein possessing an activity to specifically bind to the above-described endocrine disruptor; for example, immunoglobulin (Ig), Ig-derived recombinant protein and the like can be mentioned. As examples of said Ig, those of mammals such as mouse, rat, rabbit, goat, sheep, human, cat, dog, guinea pig, horse, bovine, swine, deer and kangaroo, or of fowls such as chicken and turkey are preferred. Also, in the present invention, said anti-endocrine disruptor antibody may be any of monoclonal antibody, polyclonal antibody and recombinant antibody, and may be selected according to the purpose of use of the isolation

material of the present invention.

Also, as the above-described Ig, IgG, IgA, IgM, IgD, IgE and the like can be mentioned, and the above-described Ig may be a portion thereof; as examples of said portion, IgG (H+L), IgG (γ chain), IgG (Fab), IgG (Fc), IgG (Fab monomer), IgG (Fd), IgG1, IgG2, IgG3, IgG4, IgG2a, IgG2b, IgG2c, IgA (α chain), IgA1, IgA2, IgM (μ chain), IgD (δ chain), IgE (ε chain), κ light chain, κ (free) chain, λ light chain, λ (free) chain and the like can be mentioned.

Production of the above-described Ig, which is an anti-endocrine disruptor antibody, can be conducted by a method known per se, for example, a method described in Enzyme Immunoassay, pp. 46-71 and pp. 85-110 (written by P. TIJSSEN, translated by Eiji Ishikawa, Tokyo Kagaku Dozin (1989)) or a method based thereon. In these methods, as examples of the carrier protein for inoculation of a complex of an endocrine disruptor derivative which is an antigen and the carrier protein, bovine serum albumin (hereinafter abbreviated as BSA), ovalbumin (hereinafter abbreviated as OVA), keyhole limpet hemocyanin (hereinafter abbreviated as KLH), bovine thyroglobulin (hereinafter abbreviated as BTG) and the like can be mentioned.

Formation of a complex of an endocrine disruptor derivative and a carrier protein can, for example, be conducted by binding a compound represented by Formula (6):

$$A\text{-}R \qquad (6)$$

(wherein R represents COOH, $NH_2$ or SH, A represents a group that becomes an endocrine disruptors or a related compound thereof by eliminating of the R group.) to the carrier protein by a method known per se. The compound represented by Formula (6) can also be synthesized chemically by forming or introducing carboxyl group, amino group, or sulfhydryl group into an appropriate starting material by a method known per se.

For example, a compound represented by Formula (6) wherein R is COOH and A becomes a polyoxyethylenealkylphenyl ether can be produced by dehydrate-condensing (half esterifying) polyoxyalkylphenyl ether and succinic anhydride [Cancer Biochem. Biophys., 7, 175 (1984)]. A compound represented by Formula (6) wherein R is $NH_2$ and A becomes a polyoxyethylenealkylphenyl ether can be produced by chlorinating the hydroxyl group of the polyoxyalkylphenyl ether with thionyl chloride [Journal of American Chemical Society, 60, 2540 (1938)], and then treating with ammonia [Organic Functional Group Preparations, vol. 1, p. 382].

A compound represented by Formula (6) wherein R is SH and A becomes a polyoxyethylenealkylphenyl ether can be produced by chlorinating the hydroxyl group of the polyoxyalkylphenyl ether with thionyl chloride [Journal of American Chemical Society, 60, 2540 (1938)], and then reacting with sodium hydrosulfide [Journal of American Chemical Society, 72, 1843 (1950)].

The above-described Ig can be obtained by a method known per se, that is, when the above-described Ig is a polyclonal antibody, it can be obtained from an animal immunized with a complex of an endocrine disrupted derivative and a carrier protein. Also, when the above-described Ig is a monoclonal antibody, it can be obtained by allowing a monoclonal antibody-producing hybridoma obtained by fusing myeloma cells with splenocytes or lymph node cells from an animal immunized with the complex of an endocrine disruptor derivative and a carrier protein, to produce the monoclonal antibody.

For immunizing an animal, an endocrine disruptor or a complex obtained above is inoculated to the animal. As examples of the animal undergoing inoculation, goat, sheep, rabbit, rat, mouse, guinea pig, chicken and the like can be used; when a polyclonal antibody against an endocrine disruptor is desired, chicken and rabbit are particularly preferred; when a monoclonal antibody is desired, mouse is particularly preferably used. As the inoculation method, a commonly performed method can be used; for example, a method is used wherein about 1 to 100 μg, preferably about 50 to 100 μg, per inoculation, is emulsified with an equal volume (0.1 ml) of physiological saline and Freund's complete adjuvant or the RIBI Adjuvant System™, and inoculated subcutaneously into the back or abdomen intraperitoneally to a mouse 3 to 6 times every 2 to 3 weeks.

Here, when a polyclonal antibody is to be obtained, it can be collected from a serum of the animal or an egg of the fowl undergoing inoculation.

Also, to obtain a monoclonal antibody, the following procedure is further conducted.

From among these immunized animals, for example, mice, individuals with high antibody titers are selected; 3 to 5 days after final immunization, the spleen or a lymph node is collected, and antibody-producing cells contained therein are fused with myeloma cells.

As the method of immunization, known methods such as in vitro immunization and the Mouse Foot Pad method can be used to raise the antibody titer in a shorter period.

The fusion procedure can be performed according to a known method; as a fusogen, polyethylene glycol (hereinafter abbreviated as PEG), Sendai virus and the like can be mentioned, and PEG is preferably used.

As the myeloma cells, NS-1, P3U1, Sp2/O and the like can be used; particularly preferred is P3U1. For example, a preferable ratio of splenocytes and myeloma cells is about 1:1 to 10:1; it is recommended that a PEG of a

molecular weight of about 1,000 to 6,000 be added thereto at a concentration of about 10 to 80%, and incubation be conducted at about 20 to 37°C, preferably about 30 to 37°C, for about 3 to 10 minutes.

Monoclonal antibody production and purification using a hybridoma can also be conducted by a method known per se. The obtained monoclonal antibody serves as an antibody not only against an endocrine disruptor, but also against a compound represented by Formula (6).

As specific examples of the antibody production and purification method, methods described in the aforementioned "Enzyme Immunoassay", pp. 46-71 and pp. 85-110, such as salting-out ($Na_2SO_4$, $(NH_4)_2SO_4$), ion exchangers (DEAE, QAE, CM/cellulose, Sephadex, Sepharose, Servacel and the like), hydrophobicity chromatography (L-phenylalanyl-Sepharose and the like), gel filtration (Sephadex G-200, Bio-Gel P-300 and the like), electrophoresis (zone electrophoresis with agarose gel, isoelectric focusing, isotachophoresis and the like), ultracentrifugation (sucrose density gradient centrifugation), and affinity chromatography (immobilized protein A (Protein A Sepharose, Protein-A superose and the like)) can be mentioned.

Various methods can be used for screening of desired antibody-producing hybridoma; for example, an ELISA method wherein a hybridoma culture supernatant is added to a microplate to which an OVA coupled with an endocrine disruptor-like compound (hapten) has been adsorbed, then a horseradish peroxidase (hereinafter abbreviated as HRP)-labeled anti-mouse immunoglobulin antibody is added, and the antibody bound to the plate solid phase is detected, and the like can be mentioned. Antibody-activity-positive hybridomas are immediately subjected to cloning, and usually this is easily conducted by limiting dilution and the like.

By measuring the antibody titer of the cloned hybridoma supernatant by the above-described method, and selecting a hybridoma that stably produces an antibody of high titer, a desired monoclonal hybridoma can be obtained.

As examples of the hybridoma obtained by such a method, hybridomas that produce a monoclonal antibody against an endocrine disruptor, MOF3-139 (FERM BP-6059), AP-14 (FERM BP-6633), BP2-177 (FERM BP-6634), DF-34 (FERM BP-6635) and CP-8 (FERM BP-6636) (PCT International Publication No. WO99/43799), and hybridomas obtained in Reference Example 2 described below, E2-73 (FERM BP-7569), E1-420 (FERM BP-7568), EE2-227 (FERM BP-7567), EE2-3 and EE2-8-18, and the like, can be mentioned.

Also, in the present invention, it is also possible to use an Ig-derived recombinant protein as the anti-endocrine disruptor antibody. As said Ig-derived recombinant protein, a recombinant protein obtained by acquiring the gene for the above-described endocrine disruptor antibody, and creating various properties possessed by the original antibody such as the antigen affinity, antigen binding capability, cross reactivity, antigen-antibody reaction interrupter resistance, enzyme color developing reaction interrupter resistance and solvent resistance by modification technology of gene manipulation, and the like, can be mentioned. Because such a recombinant protein not only is capable of binding to an endocrine disruptor, but also possesses useful properties such as good sensitivity, little cross reactivity, unlikelihood of being affected by interrupters and unlikelihood of being affected by solvents, during endocrine disruptor measurement, quantitation and concentration, it is preferred from a practical viewpoint.

As examples of the above-described Ig-derived recombinant protein,

(1) a protein containing:

an anti-endocrine disruptor antibody heavy chain variable region having the amino acid sequence shown by SEQ ID NO:2, the amino acid sequence shown by SEQ ID NO:6, the amino acid sequence shown by SEQ ID NO:10, the amino acid sequence shown by SEQ ID NO:14, the amino acid sequence shown by SEQ ID NO: 18, the amino acid sequence shown by SEQ ID NO:22, or substantially the same amino acid sequence thereas, or an anti-endocrine disruptor antibody light chain variable region having the amino acid sequence shown by SEQ ID NO:4, the amino acid sequence shown by SEQ ID NO:8, the amino acid sequence shown by SEQ ID NO:12, the amino acid sequence shown by SEQ ID NO:16, the amino acid sequence shown by SEQ ID NO: 20, the amino acid sequence shown by SEQ ID NO:24, or the substantially the same amino acid sequence thereas, and obtained by gene recombination technology:

for example, the anti-estradiol monoclonal antibody variable region (scFv) obtained in Reference Example 3 described below, the anti-alkylphenol monoclonal antibody scFv obtained in Reference Example 6 described below, the anti-alkylphenol ethoxylate monoclonal antibody scFv and the like,

(2) a partial peptide of the protein described in (1), and the like can be mentioned.

The genes of the various antibody protein against the aforementioned endocrine disruptors can be obtained using a method known *per se* according to the disclosure in the present specification. For example, such gene can be obtained from a hybridoma that produces an anti-endocrine disruptor monoclonal antibody, which is not to be construed as limitative.

The N-terminal amino acid sequence of the antibody protein is determined, a primer having a base sequence deduced from this amino acid sequence is then prepared, mRNA is prepared from an antibody-producing hybridoma by a methods known *per se*, and single-stranded cDNA is synthesized on the basis of the mRNA using reverse transcriptase, after which the above-mentioned antibody protein gene of the present invention can be obtained selectively using PCR, hybridization, etc. on the basis of the amino acid sequence or base sequence of the variable region of a heavy chain or light chain of an anti-endocrine disruptor monoclonal antibody disclosed herein. Such methods are well-known; those skilled in the art can easily isolate the aforementioned antibody protein gene on the basis of the disclosure herein. As specific procedures for these methods, there may be mentioned, for example, the method described in Molecular Cloning, 3rd edition (J. Sambrook et. al., Cold Spring Harbor Lab. Press, 2001). Useful methods of mRNA extraction include the method described in the operating manual for the Amersham QuickPrep mRNA purification kit, and useful methods of cDNA synthesis and 5'-RACE include the methods described in the instruction manual for the CLONTECH Laboratories SMART RACE kit.

Regarding how to prepare recombinant antibodies, Chapter 2 of "RECOMBINANT ANTIBODIES" [edited by F. Breitling, John Wiley & Sons (USA), 1999] describes a method of preparing recombinant antibody fragments, a method of cloning antibody genes from hybridoma cell lines, a method of preparing antibody gene libraries, a method of selection of recombinant antibodies from gene libraries, a method of antibody gene engineering, etc., using which methods recombinant antibodies can be prepared.

Chapter 4 of the same book describes a method of producing recombinant antibodies and recombinant antibody fragments, and methods of their expression, including expression in rabbit reticulocyte lysate in vitro; expression in prokaryotes such as E. coli cytoplasm, soluble fraction of periplasm, inclusion body of periplasm, Bacillus and Streptomyces; and also described are methods of expression in eukaryotes such as Pichia, Saccharomyces, Schizosaccharomyces and other yeasts, Trichoderma and other fungi; expression in insect cells such as Baculovirus; expression in animal cells such as myeloma, CHO, and COS, transgenic plants such as of tobacco, and transgenic animals, using which methods transformants can be prepared.

Chapter 4 of the same book also describes methods of purifying recombinant antibodies and recombinant antibody fragments, wherein the desired product is first separated by a physical means, for example, cell harvest by centrifuging transformant, cell disruption by ultrasonication etc., mechanical milling, or enzymatic lysis. Subsequently, purification is conducted using a combination of ion exchange chromatography, size exclusion chromatography, thiophilic adsorption chromatography, affinity chromatography, etc. Affinity chromatography, in particular, is an efficient method; the desired product can be produced by purification using an antigen-specific method based on antigen recognition specificity, an antibody-specific method based on binding to the Fc or Fab' portion of protein A or protein G, or in the case of scFv without these portions, a method comprising expressing the ScFv as a fusion antibody having a small peptide fragment called "tag" and an affinity column specific for this tag is used (e.g., His-tag, c-myc tag, Strep tag, etc.).

First, a cDNA library of anti-endocrine disruptor monoclonal antibody-producing cells is constructed, and the cDNA library is then screened using a cDNA encoding the N-terminal sequence of the highly conservative constant region or the variable region of a heavy or light chain of an immunoglobulin as a probe; anti-endocrine disruptor monoclonal antibody light or heavy chain cDNA can be thus isolated. As specific procedures for these methods, there may be mentioned, for example, the method described in Molecular Cloning, 3rd edition (J. Sambrook et. al., Cold Spring Harbor Lab. Press, 2001).

The above-mentioned antibody protein gene may also be synthesized chemically using a well-known technique on the basis of the sequence described herein. Conversion of DNA base sequences can be achieved by a method known *per se* such as the ODA-LAPCR method, the Gupped duplex method, or the Kunkel method, or a method based thereon, using PCR or a known kit such as Mutan™-Super Express Km (Takara Shuzo Co., Ltd.) or Mutan™-K (Takara Shuzo Co., Ltd.) and the like. Depending on the purpose of use, the cloned antibody protein-encoding DNA can be used as is, or after being digested with a restriction enzyme or added with a linker as necessary. This DNA may have ATG as a translation initiation codon on the 5'-terminus thereof and TAA, TGA or TAG as a translation stop codon on the 3'-terminus thereof. These translation initiation codons and translation stop codons can also be added using an appropriate synthetic DNA adapter.

An expression vector for the above-mentioned antibody protein can, for example, be produced by (a) excising the desired DNA fragment from the DNA encoding the antibody protein of the present invention, and (b) joining the DNA fragment to downstream of a promoter in an appropriate expression vector.

[Methods of preparing recombinant antibodies]

Various forms of recombinant antibodies can be prepared and are exemplified by those described in Roland Kontermann's ANTIBODY ENGINEERING HOME PAGE (http://aximt1.imt.uni-marburg.de/~rek/AEP.html, February 25, 2002); for example, recombinant antibodies can be prepared in the form of Fab' fragments, F(ab') fragments, Fv

fragments (Fv), single-chain Fv fragments (scFv), bispecific-chimeric scFV (χ-scFv), tandem scFV (scFv)2, bispecific-(scFv)2, disulfide-linked scFv, disulfide-stabilized Fv fragments (dsFv), diabody, single-chain diabody (scDb), bivalent diabody, bispecific diabody, knob-into-hole stabilized diabody, disulfide-stabilized diabody, triabody, tetrabody, trispecific triabody, CL-dimerized scFv, CH1-CL-dimerized scFv, CH3-dimerized scFv, knob-into-hole CH3-dimerized scFv, CH3-dimerized bivalent diabody, Fc-dimerized scFv, Fab-scFv fusions, Ig-scFv fusions, leucine-zipper stabilized scFv dimers, helix-stabilized scFv dimers, 4 helix-bunde stabilized scFv tetramers, streptavidin-scFv, and intrabody. Methods wherein an antibody having a desired useful property is selected by shuffling mutated antibody genes also fall in the scope of the present invention.

[Recombinant antibody expression systems]

Any recombinant antibody expression system can be used, as long as it is capable of efficiently expressing a recombinant antibody; for example, as tabulated in Roland Kontermann's ANTIBODY ENGINEERING HOME PAGE (http://aximt1.imt.uni-marburg.de/~rek/AEP.html, February 25, 2002), expression of Fv, scFv and scFv derivatives, bivalent and bispecific scFv, scFv or Fab-fusion proteins, intrabodies, etc. in mammalian cells, expression of scFV, Fab, etc. in insect cells, expression of Fv, scFv, Fab, etc. in fungal cells, and expression of scFv in plant cells are known, and hence a variety of expression systems can be used.

[Methods of constructing a cDNA library]

Any method of constructing a cDNA library can be used, as long as it is capable of efficiently preparing a cDNA library; one of such methods is the phage display method described in Roland Kontermann's ANTIBODY ENGINEERING HOME PAGE (http://aximt1.imt.uni-marburg.de/~rek/AEP.html, February 25, 2002).

[Selection methods of recombinant antibody]

As useful methods of selecting the desired recombinant antibody from the library constructed, the protocol described in Roland Kontermann's ANTIBODY ENGINEERING HOME PAGE (http://aximt1.imt.uni-marburg.de/~rek/AEP.html, February 25, 2002), and the methods "Isolation of Recombinant Antibodies from Phagemid Libraries" and "Isolation of Peptides from fd Phage Libraries" are available.

The gene (DNA) used for expression may have substantially the same amino acid sequence as the anti-endocrine disruptor antibody protein expressed by the gene (DNA) obtained as described above, or a partial peptide thereof. Substantially the same amino acid sequence is exemplified by amino acid sequences with a partial modification, with a different amino acid sequence added, or with an amino acid sequence deleted. Depending on the purpose of use, the gene (DNA) can be used as is, or after being cleaved or added with another DNA as necessary. This DNA may have a translation initiation codon ATG on the terminal thereof. This can be achieved by a method known *per se*. Such methods include, for example, the method described in Molecular Cloning, 3rd edition (J. Sambrook et. al., Cold Spring Harbor Lab. Press, 2001).

By incorporating the thus-obtained DNA, a promoter, a translation initiation codon, the appropriate signal sequence, etc., by a methods known *per se,* into a vector, a recombinant vector can be produced. As such vectors, promoters and host strains, there may be mentioned, for example, the vectors, promoters and Escherichia strains described in Appendix 3 to Molecular Cloning, 3rd edition (J. Sambrook et. al., Cold Spring Harbor Lab. Press, 2001).

In addition to the aforementioned vectors, useful vectors include plasmids of *Escherichia coli* origin (pET-276, pCANTAB-5E, pUC19, pT7Bule T), plasmids of *Bacillus subtilis* origin (e.g., pUB110, pTP5, pC194), plasmids of yeast origin (e.g., pSH19, pSH15), λ phage, bacteriophages such as M13K07, and animal viruses such as retrovirus, vacciniavirus and vaculovirus, as well as pA1-11 pXT1, pRc/CMV, pRc/RSV, pcDNAI/Neo and the like.

Any promoter can be used, as long as it is suitable for the host used for gene expression. Preferred promoters include, for example, the trp promoter, the lac promoter, the recA promoter, the $\lambda P_L$ promoter, and the lpp promoter when the host is a bacterium of the genus *Escherichia;* the SPO1 promoter, the SPO2 promoter, and the penP promoter when the host is a bacterium of the genus *Bacillus;* and the PHO5 promoter, the PGK promoter, the GAP promoter, and the ADH promoter when the host is a yeast. When the host is an animal cell, the SRα promoter, the SV40 promoter, the LTR promoter, the CMV promoter, the HSV-TK promoter, etc. are preferred; when the host is an insect cell, the polyhedrin promoter, the P10 promoter, etc. are preferred.

In addition to the aforementioned promoters, the expression vector may contain an enhancer, splicing signal, polyA addition signal, selection marker, SV40 replication origin, etc. as necessary. Selection markers include, for example, the ampicillin resistance gene (hereinafter also referred to as $Amp^R$), the kanamycin resistance gene (hereinafter also referred to as $Km^R$), and the chloramphenicol resistance gene (hereinafter also referred to as $Cm^R$).

Where necessary, a signal sequence suitable for the host is added to the N-terminus side of the aforementioned antibody protein. Useful signal sequences include the phoA signal sequence, the ompA signal sequence and the like when the host is a bacterium of the genus *Escherichia;* the α-amylase signal sequence, the subtilisin signal sequence and the like when the host is a bacterium of the genus *Bacillus;* the MFα signal sequence, the SUC2 signal sequence and the like when the host is a yeast; and the insulin signal sequence, the α-interferon signal sequence, and the antibody molecule signal sequence and the like when the host is an animal cell. Using the thus-constructed vector harboring the DNA encoding the aforementioned antibody protein, a transformant can be produced.

Useful hosts include the genus *Escherichia,* the genus *Bacillus,* yeasts, insect cells, insects, animal cells and the like. Examples of useful bacteria of the genus *Escherichia* include *Escherichia coli* K12-DH1 [Proc. Natl. Acad. Sci. USA, vol. 60, 160 (1968)], JM103 [Nucleic Acids Research, vol. 9, 309 (1981)], JA221 [Journal of Molecular Biology, vol. 120, 517 (1978)], HB101 [Journal of Molecular Biology, vol. 41, 459 (1969)], C600 [Genetics, vol. 39, 440 (1954)], BL21DE3(pLysS), TG-1, and JM109. Useful bacteria of the genus *Bacillus* include, for example, *Bacillus subtilis* MI114 [Gene, vol. 24, 255 (1983)] and 207-21 [Journal of Biochemistry, vol. 95, 87 (1984)]. Useful yeasts include, for example, *Saccharomyces cerevisiae* AH22, AH22R$^-$, NA87-11A, DKD-5D, and 20B-12, *Schizosaccharomyces pombe* NCYC1913 and NCYC2036, and *Pichia pastoris.* When the virus is AcNPV, useful insect cells include, for example, established *Spodoptera frugiperda* larva cells (Sf cells), *Trichoplusia ni* midgut MG1 cells, *Trichoplusia ni* egg High Five™ cells, *Mamestrabrassicae* cells, *Estigmena acrea* cells and the like. When the virus is BmNPV, silkworm-derived established cells (*Bombyx mori* N; BmN cells) etc. are used. Such Sf cells include, for example, Sf9 cells (ATCC CRL1711) and Sf21 cells [both described by Vaughn, J.L. et al. In Vivo, 13, 213-217 (1977)]. Useful insects include, for example, silkworm larvae [Maeda et al., Nature, vol. 315, 592 (1985)]. Useful animal cells include, for example, simian COS-7 cells, Vero, Chinese hamster CHO cells, mouse L cells, mouse AtT-20, mouse myeloma cells, rat GH3 and human FL cells.

Transformation of bacteria of the genus Escherichia can, for example, be achieved according to the methods described in Proc. Natl. Acad. Sci. USA, vol. 69, 2110 (1972) and Gene, vol. 17, 107 (1982). Transformation of bacteria of the genus *Bacillus* can, for example, be achieved according to the method described in Molecular & General Genetics, vol. 168, 111 (1979). Transformation of yeasts can, for example, be achieved according to the methods described in Methods in Enzymology, vol. 194, 182-187 (1991) and Proc. Natl. Acad. Sci. USA, vol. 75, 1929 (1978). Transformation of insect cells or insects can, for example, be achieved according to the method described in Bio/Technology, vol. 6, 47-55 (1988). Transformation of an animal cell can, for example, be achieved using the method described in SAIBO KOGAKU (CELL TECHNOLOGY), Supplementary 8: *Shin Saibou Kougaku Jikken Protocol,* 263-267 (1995) (published by Shujunsha Co., Ltd.) and Virology, Vol. 52, 456 (1973). Thus, a transformant transformed with an expression vector harboring the DNA encoding an antibody protein is obtained.

Furthermore, the above-mentioned protein can be produced by cultivating the thus-obtained transformant and harvesting the resulting protein. As media to be used for culture, liquid media are suitable for the cultivation of a transformant whose host is a bacterium of the genus *Escherichia* or *Bacillus,* which media are supplemented with carbon sources, nitrogen sources, minerals, and other substances required for the growth of the transformant. Carbon sources include, for example, glucose, dextrin, soluble starch, sucrose and the like; nitrogen sources include, for example, inorganic or organic substances such as ammonium salts, nitrates, corn steep liquor, peptone, casein, meat extract, soybean flour, potato extract and the like; minerals include, for example, calcium chloride, sodium dihydrogen phosphate, magnesium chloride and the like. Yeast, vitamins, growth promoters, etc. may also be added. It is desirable that the pH of the medium be about 5 to 8 for bacteria and about 2 to 8 for fungi and yeast.

Preferred media for cultivating the genus *Escherichia* include, for example, M9 medium supplemented with glucose and casamino acid [Miller, Journal of Experiments in Molecular Genetics, 431-433, Cold Spring Harbor Laboratory, New York (1972)]. To increase promoter efficiency where necessary, an agent like 3β-indolylacrylic acid or isopropylthiogalactoside (IPTG), for example, may be added. When the host is a bacterium of the genus *Escherichia,* cultivation is normally carried out at about 15 to 43°C for about 3 to 24 hours, and aeration and stirring may be conducted as necessary. When the host is a bacterium of the genus *Bacillus,* cultivation is normally carried out at about 30 to 40°C for about 6 to 24 hours, and aeration and stirring may be conducted as necessary. Media for cultivating a transformant whose host is a yeast include, for example, Burkholder's minimum medium [Bostian, K.L. et al., Proc. Natl. Acad. Sci. USA, vol. 77, 4505 (1980)] and SD medium supplemented with 0.5% casamino acid [Bitter, G.A. et al., Proc. Natl. Acad. Sci. USA, vol. 81, 5330 (1984)]. It is preferable that the medium's pH be adjusted to about 2 to 8. Cultivation is normally carried out at about 20 to 35°C for about 24 to 72 hours, with aeration and stirring conducted as necessary.

Useful media for cultivating a transformant whose host is an insect cell or an insect include Grace's insect medium [Grace, T.C.C., Nature, 195, 788 (1962)] supplemented with additives such as inactivated 10% bovine serum as appropriate. It is preferable that the medium's pH be adjusted to about 6.2 to 6.4. Cultivation is normally carried out at about 27°C for about 3 to 5 days, with aeration and stirring conducted as necessary. Useful media for cultivating a transformant whose host is an animal cell include, for example, MEM medium supplemented with about 5 to 20% fetal bovine serum [Science, vol. 122, 501 (1952)], DMEM medium [Virology, vol. 8, 396 (1959)], RPMI1640 medium [The Journal of the American Medical Association], vol. 199, 519 (1967)], and 199 medium [Proceeding of the Society for

the Biological Medicine, vol. 73, 1 (1950)]. It is preferable that pH be about 6 to 8. Cultivation is normally carried out at about 30 to 40°C for about 15 to 60 hours, with aeration including carbon gas and stirring conducted as necessary. As described above, the above-mentioned antibody protein can be produced in the cells or cell membrane of the transformant or outside the cells.

Separation and purification of the above-mentioned subject antibody protein from the thus-obtained culture can, for example, be achieved by the method described below. When extracting the above-mentioned antibody protein from cultured fungi or cells, there may be used as appropriate, for example, a method wherein cultured fungi or cells are collected by a known means, suspended in an appropriate buffer solution, and disrupted by means of ultrasound, lysozyme and/or freeze-thawing etc., after which a crude extract of antibody protein is obtained by centrifugation or filtration. The buffer solution may contain a protein denaturant such as urea or guanidine hydrochloride and a surfactant such as Triton X-100™. If the antibody protein is secreted in the culture broth, it is treated by a method known *per se* to separate the fungi or cells and the supernatant after completion of cultivation and the supernatant is collected. Purification of the antibody protein contained in the thus-obtained culture supernatant or extract can be achieved by appropriately combining methods known *per se* of separation and purification. Useful known methods of separation and purification include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing.

The above-mentioned protein or a salt thereof can be produced by a method known *per se* of protein synthesis, or by cleaving the above-mentioned protein using an appropriate protease. This protein synthesis can, for example, be achieved by solid phase synthesis or liquid phase synthesis. Specifically, the desired protein can be produced by condensing the partial peptide or amino acids that constitute the above-mentioned protein and the remainder, and if the purified product has a protective group, removing the protective group. Known methods of condensation and the methods of protective group removal described below, for example, can be used.

(1) M. Bodanszky and M.A. Ondetti, Peptide Synthesis, Interscience Publishers, New York (1966)
(2) Schroeder and Luebke, The peptide, Academic Press, New York (1965)
(3) Nobuo Izumiya et al., *Peputido Dogei no Kiso to Jikken,* Maruzen Co., Ltd. (1975)
(4) Haruaki Yajima and Shunpei Sakakibara, *Seikagaku Jikken Kouza* 1, *Tanpakushitsu no Kagaku* IV, 205, (1977)
(5) Haruaki Yajima, supervisor, *Zoku Iyakuhin no Kaihatsu,* Vol. 14: Peputido Gosei, Hirokawa Publishing

After the reaction, the above-mentioned protein can be purified and isolated using ordinary purification methods such as solvent extraction, distillation, column chromatography, liquid chromatography, and recrystallization in combination. If the protein obtained by the method described above is a free from, it can be converted to an appropriate salt by a known method; if the protein obtained is a salt, it can be converted to a free form by a known method.
As the polymeric material that constitutes a carrier made of a polymeric material in the isolation material of the present invention known materials used in such carriers as an isolation membrane (isolation material), a filtration membrane (filtration material), a column packing and the like, for example,

(1) naturally occurring polymers or derivatives thereof: cellulose or cellulose derivative (for example, cellulose, regenerated cellulose, cellulose acetate, cellulose nitrate, nitrocellulose and the like), agarose and the like;
(2) synthetic polymers: polyolefin (for example, polyethylene, polypropylene and the like), halogenated polyolefin, polyester, polystyrene, polyether, polyamide (for example, nylon and the like), polyimide, polytetrafluoroethylene, polyvinyl alcohol, polyvinyl acetate, polycarbonate, polyacrylamide, ethylene-vinyl alcohol copolymer and the like;
(3) the polymer of (1) or (2) modified by cross-linking or surface treatment (for example, hydrophilicization treatment and the like); modified polystyrene, hydrophilicized polyethylene and the like;

and the like can be mentioned, but these are not to be construed as limiting.

Preferably, those that are highly sensitive to ionizing radiation and do not deteriorate can be mentioned; particularly preferred are polyolefin (particularly polyethylene) and cellulose.

As the carrier made of a polymeric material in the isolation material of the present invention, a carrier made of a polymeric material known as an isolation membrane (isolation material), a filtration membrane (filtration material), a column packing and the like can be mentioned, but these are not to be construed as limiting. Preferably, because many of the sample contain only a trace amount of endocrine disruptor to be isolated (for example, several ng/l or so), those enabling treatment at large flow volumes (high-speed treatment) can be mentioned. As examples of such carrier made of a polymeric materials, a plain membrane, a hollow fiber membrane, a non-woven fabric, a woven fabric, a fiber, a molded bead, general-purpose filter paper and the like can be mentioned. Particularly preferred are porous

carriers and non-woven fabrics such as a porous plain membrane and a porous hollow fiber membrane; particularly preferred are a porous hollow fiber membrane and a non-woven fabric. Because treatment at particularly high speed is possible when the carrier is a porous hollow fiber membrane, this carrier is suitable for cases wherein a particularly large number of samples are treated. On the other hand, when the carrier is a non-woven fabric, stable treatment is possible because not only high-speed treatment can be conducted, but also the occurrence of clogging caused by impurities present in the sample can be suppressed.

As a porous carrier, one having a large number of pores with a pore diameter of 0.05 to 100 μm (preferably 0.1 to 10 μm), and with a void ratio of 20 to 80% (preferably 40 to 80%).

The pore diameter of pores as mentioned herein refers to a value measured by the mercury press-fit method using a pore distribution measurement apparatus (porocimeter) (editing supervised by Hiroaki Yanagida, "Biryushi Kougaku Taikei (Volume I) Kihon Gijutsu", 1st edition, Fuji Technosystem K.K., October 31, 2001, pp. 442-444).

The void ratio as mentioned herein refers to a value obtained from the relative ratio of bulk density and true density of the material. Specifically, a porous carrier such as a porous hollow fiber membrane, dried, is immersed in methanol to wet the surfaces of inside pores, after which the methanol is replaced with water. The thus-obtained water-containing porous carrier is weighed and then dried, and the porous carrier is weighed. The difference in weight between the wet and dry states is converted to pore (void) volume using water density. By dividing the pore volume by the volume of the entire porous carrier, the void ratio can be calculated.

As the non-woven fabric, one having a fabric weight (density) of 10 to 200 $g/m^2$ is preferable, and one of 30 to 100 $g/m^2$ is more preferable.

The fabric weight of the non-woven fabric as mentioned herein refers to a value calculated by cutting out the non-woven fabric into an appropriate area, weighing the dry weight thereof, and then dividing the obtained weight by the area.

In the isolation material of the present invention, an anti-endocrine disruptor antibody is carried covalently via a graft chain by a carrier made of a polymeric material. A "graft chain" as mentioned herein refers to a polymer chain (branch polymer) resulting from polymerization of a reactive monomer, formed by graft polymerization in a carrier substrate (stem polymer). A reactive monomer as mentioned herein refers to a graft polymerizable monomer; for example, a known monomer usable for graft polymerization can be mentioned but is not subject to limitation. As the reactive monomer in the present invention, one having a polymerizable functional group (for example, vinyl group and the like), and also having a functional group that can be coupled to antibody (for example, epoxy group and the like) is preferable; as such, an epoxy group-containing vinyl monomer such as glycidyl methacrylate (GMA), glycidyl acrylate (AMA), glycidyl sorbate, glycidyl metaitaconate, ethylglycidyl maleate or glycidyl vinylsulfonate and the like can be mentioned. Particularly preferred is glycidyl methacrylate (GMA) because coupling with antibody is easy.

Although the method of producing an isolation material with an antibody carried by a carrier via a graft chain, which is the preferred isolation material of the present invention, is not subject to limitation, an isolation material according to the intended use can easily be obtained, it is preferable that the isolation material be produced by allowing a carrier substrate of an existing shape to form a side chain by graft polymerization using the above-described reactive monomer, and then coupling an antibody to said side chain.

The graft polymerization method for allowing a carrier substrate of an existing shape to form a graft chain is not subject to limitation; because the reaction conditions are mild so that the substrate can be allowed to form a graft chain without affecting the characteristics of the carrier substrate, the radiation graft polymerization method, which is a method wherein a carrier substrate (stem polymer) is irradiated with a radiation to produce a radical (reaction initiation point) and a reactive monomer is brought into contact therewith to form a branch polymer, is preferred (see steps (1) and (2) of Figure 1).

As the reactive monomer used for radiation graft polymerization in the present invention, those described above can be mentioned.

As examples of the above-described radiation used in radiation graft polymerization, α rays, β rays, γ rays, electron beams, accelerated electron beams, X rays and the like can be mentioned; although these may be appropriately selected according to the material, shape, thickness and the like of the carrier irradiated with radiation, γ rays or electron beams are normally used in the present invention.

As the γ ray source, cobalt, cesium, strontium and the like can be mentioned. Because a cobalt γ ray source has a great transmission depth and hence makes it possible to increase the thickness of the subject of irradiation compared to the use of an electron beam, it can preferably used in the method wherein a long article is made into a roll and irradiated by the batch method.

As the electron beam source, an accelerator such as the Van de Graaff accelerator, linac or cyclotron is used. Also, because the electron beam source has a small transmission depth, though it has an exposure rate of as high as 300-1000 times that of the γ ray source and hence permits short-time irradiation, it can suitable used in cases where a thin article is continuously irradiated using a transportation apparatus. Also, because the accelerated electron beam transmission depth depends upon acceleration voltage, the acceleration voltage can be appropriately set according

to the thickness of the subject of irradiation and the irradiation scale.

Radiation exposure is generally 10 to 500 kGy (preferably 50 to 100 kGy) and irradiation time is 1 to 50 hours (preferably 5 to 10 hours) when the $\gamma$ ray is used and 1 to 500 seconds (preferably 5 to 10 second) when the electron beam is used.

Also, although radiation irradiation is normally conducted at room temperature (10 to 40°C), it can be conducted in an inert gas (for example, nitrogen, argon and the like) as necessary to suppress the attenuation of the radical produced.

In radiation graft polymerization in the present invention, the above-described reactive monomer may be co-present during radiation irradiation of the carrier substrate (simultaneous irradiation method), and the above-described reactive monomer may be brought into contact with the radiation-irradiated substrate after radiation irradiation of the substrate (pre-irradiation method). Preferably, in the simultaneous irradiation method, because monomer homopolymerization is likely to occur simultaneously with graft polymerization, it is preferable that the pre-irradiation method be used.

Because the simultaneous irradiation method involves an exothermic reaction, $\gamma$ ray polymerization is safer than short-time polymerization using the electron beam; this method is normally conducted by irradiating the $\gamma$ ray in an inert gas atmosphere at room temperature for 1 to 2 hours.

The pre-irradiation method includes a step wherein radiation is irradiated to a carrier substrate to allow the carrier substrate to produce a radical and then the above-described reactive monomer is brought into contact therewith to cause a graft polymerization reaction. Although said a graft polymerization reaction step is not subject to limitation, it can usually be conducted by immersing a substrate with a radical produced by radiation irradiation in a solution containing a reactive monomer.

As the solvent used in the above-described solution containing a reactive monomer, methanol, propanol, butanol, acetone, N,N-dimethylformamide (DMF) and the like can be used; preferably, methanol is used. Also, the reactive monomer concentration in said solution is normally 10 to 60% by weight, preferably 20 to 50% by weight.

Reaction temperature and reaction time in the graft polymerization reaction step are not subject to limitation, and can be appropriately set considering the extent of radical production in the substrate, the reactive monomer used, the desired graft ratio and the like; the reaction can normally be conducted at room temperature to 80°C (preferably 40 to 50°C).

In the isolation material of the present invention, it is preferable that the graft ratio, which indicates degree of graft polymerization, be 10 to 300%, more preferably 50 to 250%.
The graft ratio (dg) as mentioned herein is expressed by Formula (i) below:

$$dg\ (\%) = 100 \times \frac{\text{Weight gain due to graft polymerization}}{\text{Stem polymer weight}}$$

By coupling the graft chain of a carrier obtained as described above and an anti-endocrine disruptor antibody having a functional group, the preferred isolation material of the present invention can be obtained (see step (3) of Figure 1) .

It is important that the coupling reaction in the present invention should be conducted under conditions that do not denature the antibody; as examples of such conditions, a reaction in a buffer solution (for example, phosphate buffer solution, borate buffer solution, acetate buffer solution, carbonate buffer solution, citrate buffer solution, Tris-HCl buffer solution and the like) of pH 2.0 to 11.0 (preferably 6.0 to 10.5) at a temperature of 0 to 40°C (preferably 4 to 30°C) for 0.1 to 72 hours (preferably 0.5 to 24 hours) can be mentioned.

The amount of antibody carried (density) in the isolation material of the present invention is not subject to limitation, and is preferably 0.1-20 mg-antibody/g-isolation material, more preferably 0.5-10 mg-antibody/g-isolation material.

The isolation material of the present invention, because an anti-endocrine disruptor antibody is stably carried by a carrier made of a polymeric material, is capable of selectively and efficiently isolating an endocrine disruptor from a sample containing only an extremely trace amount thereof (for example, river water, lake water, seawater, ground water and the like). Also, when the isolation material of the present invention is allowed to capture an endocrine disruptor and then eluted with an eluent, the captured endocrine disruptor can be eluted with a very small amount of eluent. That is, the efficiency of concentrating an endocrine disruptor with the isolation material of the present invention is extremely high. Therefore, said isolation material can be preferably used for endocrine disruptor concentration, clean-up and the like.

The endocrine disruptor concentration method of the present invention is a method comprising:

(1) a step wherein an endocrine disruptor-containing sample is brought into contact with the above-described isolation material for endocrine disruptor to allow said endocrine disruptor to be captured by an anti-endocrine

disruptor antibody, and
(2) a step wherein said endocrine disruptor captured by said anti-endocrine disruptor antibody is dissociated and recovered.

The sample containing an endocrine disruptor in the present method is not subject to limitation, as long as it contains the above-described endocrine disruptor; for example, river water, lake water, seawater, ground water, tap water, sewage, soil samples and the like can be mentioned. These may be applied as is to the present method, and may also be applied as samples prepared for analysis therefrom by a method known *per se* (for example, those with insoluble matter removed by filtration, centrifugation and the like). Also, to avoid the denaturation and the like of the anti-endocrine disruptor antibody carried by the isolation material, it is also preferable that the sample pH and salt concentration be adjusted as necessary.
In the concentration method of the present invention, the isolation material described above can be used with appropriate selection according to the kind and quantity of sample, subject endocrine disruptor and the like.
Step (1) above is a step wherein by bringing a sample containing the above-described endocrine disruptor into contact with an isolation material with anti-endocrine disruptor comprising the above-described carrier made of a polymeric material and an anti-endocrine disruptor antibody carried by said carrier made of a polymeric material, the endocrine disruptor in said sample is allowed to be captured by the anti-endocrine disruptor antibody contained in said isolation material, utilizing an antigen-antibody reaction.
The method of bringing a sample into contact with the isolation material is not subject to limitation; for example, a method wherein a sample is passed through the isolation material (for example, a method wherein a sample is introduced to a column packed with an isolation material, a method wherein a sample is introduced to the membrane inner face of an isolation material when using a hollow isolation material (for example, hollow fiber membrane) is used, and the like), a method wherein a sample and an isolation material are mixed (for example, a method wherein a sample is stirred with an isolation material added thereto) and the like can be mentioned.
Although contact temperature for the sample and the isolation material is not subject to limitation, it is normally 0 to 40°C (preferably 4 to 30°C) for avoiding the denaturation of the antibody carried by the isolation material. Also, contact time may be appropriately set according to the capability of the isolation material used.
Step (2) above is a step wherein the endocrine disruptor captured by an anti-endocrine disruptor antibody in step (1) above is dissociated and recovered from said anti-endocrine disruptor antibody.
As examples of the method of dissociating and recovering an endocrine disruptor from said anti-endocrine disruptor antibody, a method wherein the endocrine disruptor is dissociated and recovered from the anti-endocrine disruptor antibody by a known method such as an eluent nature change such as a change in pH (pH lowered to acidity (2.5 to 3 and the like), pH raised to alkalinity (11.5 and the like) and the like), a change in ionic strength (addition of NaCl and the like, salt concentration change, and the like), a change in polarity (for example, addition, concentration change and the like of an organic solvent (methanol, dioxane, ethylene glycol and the like), a chaotropic salt ($SCN^-$, $CCl_3COO^-$, $I^-$) and the like), and addition of a protein denaturant (urea, guanidine hydrochloride and the like), or electrophoresis, and the like can be used, but these are not to be construed as limiting.
By the concentration method of the present invention described above, endocrine disruptors, which occur only in very trace amounts in the environment, can be concentrated at much higher rates (several thousands to several tens of thousands of times) and more efficiently compared to conventional concentration methods such as solvent extraction and solid phase extraction, in a condition with low abundance of immunological impurities.
The endocrine disruptor clean-up method of the present invention is a method including bringing a sample containing an endocrine disruptor into contact with the above-described isolation material to allow said endocrine disruptor to be captured by an anti-endocrine disruptor antibody. This method can be conducted in the same manner as step (1) of the above-described endocrine disruptor concentration method.
Because the clean-up method of the present invention is capable of selectively and efficiently cleaning up endocrine disruptors, which occur only in very trace amounts in the environment, it is much more economical than conventional methods.
When bases, amino acids and the like are shown in abbreviations in the present specification and Figures, they are based on the abbreviations by IUPAC-IUB Commission on Biochemical Nomenclature or abbreviations conventionally used in the pertinent field. Examples thereof are given in the following. When an amino acid can have an optical isomer, it is an L form, unless particularly indicated.

DNA : deoxyribonucleic acid
cDNA : complementary deoxyribonucleic acid
a,A : Adenine
t,T : Thymine
g,G : Guanine

c,C : Cytosine
n : unidentified
RNA : ribonucleic acid
mRNA : messenger ribonucleic acid

Abbreviations of amino acids

3 letters:1 letter:name
Gly : G :glycine
Ala : A :alanine
Val : V :valine
Leu : L :leucine
Ile : I :isoleucine
Ser : S :serine
Thr : T :threonine
Cys : C :cysteine
Met : M :methionine
Glu : E :glutamic acid
Asp : D :aspartic acid
Lys : K :lysine
Arg : R :arginine
His : H :histidine
Phe : F :phenylalanine
Tyr : Y : tyrosine
Trp : W :tryptophan
Pro : P :proline
Asn : N : asparagine
Gln : Q :glutamine
Asx : B :Asn+Asp
Glx : Z :Gln+Glu
Xaa : unknown or different amino acid

## Examples

The present invention is explained in more detail by illustrating Examples in the following, which are not to be construed as limitative.
Note that measurements of the pore diameters and void ratios of the pores of porous carriers and the fabric weights of non-woven fabrics in the Examples below were conducted as described below.

(Pore diameter of pores of porous carrier)

Measured by the mercury press-fit method using a pore distribution measurement apparatus (porocimeter) (editing supervised by Hiroaki Yanagida, "Biryushi Kougaku Taikei (Volume I) Kihon Gijutsu", 1st edition, Fuji Technosystem K.K., October 31, 2001, pp. 442-444).

(Void ratio)

A dry porous carrier was immersed in methanol to wet the surfaces of inside pores, after which the methanol was replaced with water. The thus-obtained water-containing porous carrier was weighed and then dried, and the porous carrier was weighed. The difference in weight between the wet and dry states was converted to pore (void) volume using water density, and by dividing the pore volume by the volume of the entire porous carrier, the void ratio was calculated.

(Fabric weight of non-woven fabric)

Calculated by cutting out the non-woven fabric into an appropriate area, weighing the dry weight thereof, and

then dividing the obtained weight by the area.

Example 1: Preparation of immunoaffinity porous hollow fiber membrane

In preparing an immunoaffinity porous hollow fiber membrane, the following materials were used. Also, the reagents used other than those shown below were of analytical grade or higher.

- Carrier substrate:
  As the carrier substrate, a porous hollow fiber membrane of polyethylene (manufactured by Asahi Kasei Corporation) was used. The inside diameter and outside diameter of said hollow fiber were 1.8 mm and 3.1 mm, respectively. Also, said hollow fiber had a void ratio of 70%, and had formed a three-dimensional pore network with a pore diameter of 0.4 μm.
- Reactive monomer:
  As the reactive monomer, glycidyl methacrylate (GMA, $CH_2=CCH_3COOCH_2CHOCH_2$) was obtained from Tokyo Kasei Co., Ltd., and used without further purification.
- 17β-Estradiol (hereinafter also referred to as E2):
  Obtained from Wako Pure Chemical Industries, Ltd.
- Anti-estrogen polyclonal antibody (hereinafter also referred to as anti-estrogen antibody or anti-ES antibody)

One produced according to Reference Example 1 described below was used.
An immunoaffinity porous hollow fiber membrane was prepared through the following three steps (Figure 1).

(1) Radical formation in carrier substrate by electron beam irradiation:

The above-described hollow fiber membrane of 10 cm length was irradiated with an electron beam in a nitrogen atmosphere at room temperature using a cascade type acceleration apparatus (Dynamitron 1EA 3000-25-2, Radiation Dynamics Inc., New York). Exposure was 200 kGy (10 kGy×20 minutes).

(2) Graft polymerization of epoxy group-containing vinyl monomer (GMA):

The above-described hollow fiber membrane irradiated with the electron beam was immersed in a 10% (v/v) GMA/methanol solution. After 12 minutes, said hollow fiber membrane was taken out and thoroughly washed to remove the remaining GMA and polyGMA homopolymer. The GMA graft ratio (dg: defined by Formula (i) above) in the obtained GMA graft porous hollow fiber membrane (hereinafter referred to as GMA fiber) was 170%.

(3) Coupling of epoxy group of GMA graft porous hollow fiber membrane and antibody:

The above-described GMA fiber was immersed in a 0.025M borate buffer solution (pH 10) wherein anti-ES antibody was dissolved at 1.75 mg/mL, at 277 K, so that coupling reaction time would be 4-36 hours. After coupling, by washing with boric acid, a porous hollow fiber membrane containing anti-ES antibody (hereinafter referred to as ES-IA (q) fiber; q indicates ligand density (that is, the amount of coupled anti-ES antibody)) was obtained.
Ligand density (that is, the amount of anti-ES antibody immobilized), as a function of reaction time in the coupling reaction of the epoxy group of the polyGMA chain graft-polymerized to the porous hollow fiber membrane and the anti-ES antibody at 277 K, is shown in Figure 3. said ligand density increased as coupling time increased. The ligand density became 5.8 mg/g-GMA fiber with a coupling time of 36 hours.

**Example 2**

(1) Binding of E2 to immunoaffinity porous hollow fiber membrane

Various pieces of ES-IA (q) fiber (effective length 2.0 cm) having a ligand density (q) in the 3.1 to 5.8 mg/g-GMA fiber range were placed in the experimental apparatus, as shown in Figure 2. Next, 1.0 μg/L of E2-containing 10% (v/v) methanol/water was passed to the membrane outer face through the pores from the membrane inner face of the ES-IA (q) fiber at room temperature and at a constant permeation rate in the 30 to 120 mL/hour range. The E2 concentration in the effluent passed to the membrane outer face of the ES-IA (q) fiber was continuously quantified using an estrogen (Estrogen; ES) ELISA kit (Takeda Chemical Industries, Ltd.). Changes in the E2 concentration in the effluent versus the volume of the effluent were plotted to obtain a breakthrough curve. Also, the equilibrated binding capacity (EBC) of E2 was calculated as shown in Formula (ii) below.

$$EBC\ (\mu g/g) = ?\ _0^{V_e}(C_0\text{-}C)\ dV/W \qquad \text{(ii)}$$

[wherein $C_0$ and C indicate the E2 concentrations in the influent and effluent, respectively, and V, $V_e$ and W indicate the volume of effluent, the volume of effluent at the time C has reached $C_0$, and the weight of GMA fiber, respectively.].

E2 breakthrough curve s for each ES-IA (q) fiber at a constant permeation rate (60 mL/hour) at room temperature are shown in Figure 4. Also, the equilibrated binding capacity (EBC) of E2 in each ES-IA (q) fiber, along with binding ratio (this is defined as the molar ratio of the amount of E2 bound to the amount of immobilized anti-ES antibody), are shown in Figure 5. The EBC of E2 increased linearly as ligand density (q) increased. Also, the binding ratio was constant at 0.085.

Breakthrough curves obtained when the E2 solution was fed at various permeation rates (retention times) to ES-IA (4.8) fiber (ligand density 4.8 mg/g-GMA fiber) are shown in Figure 6. The obtained breakthrough curve overlapped, irrespective of the permeation rate of the E2 solution. That is, as the E2 solution permeation rate was increased, the speed of E2 binding to the ES-IA (4.8) fiber could be increased. It was considered that this was because the diffusional mass transfer limitation of E2 to the immobilized antibody was negligible. Also, this is suitable for cleaning up E2 at a high speed.

(2) Elution of E2 bound to immunoaffinity porous hollow fiber membrane

By passing 3 to 5 mL of methanol to the entire ES-IA (q) fiber at room temperature at the permeation rates corresponding to respective cases of E2 binding, the E2 bound to ES-IA (q) fiber was eluted. The E2 concentration in the effluent of the membrane outer face was continuously quantified using an ES ELISA kit. The elution rate, obtained by dividing the amount of E2 eluted by the amount of E2 bound, as shown in Figure 7, irrespective of ligand density, was almost 100%.

**Example 3**: Recovery of E2 using hollow fiber membrane estrogen affinity column

(1) Preparation of affinity column using hollow fiber membrane

A GMA hollow fiber membrane prepared by Example 1 was immersed in each of methanol and distilled water for 30 to 60 minutes. Next, the anti-estrogen antibody (2 mg/ml in 30mM borate buffer solution (pH 10)) obtained in Reference Example 1 described below was added at 1 ml per 2 cm of the hollow fiber membrane, and reacted at 4°C overnight. By washing the GMA membrane with the above-described borate buffer solution using a peristaltic pump, and measuring the amount of antibody in the washings, the amount of antibody bound to the membrane was calculated.

(2) Addition and recovery of estrogen using affinity column

After the above-described affinity column was washed with 10 ml of PBS, an estrogen (17β-estradiol (E2) or estrone (E1)) dissolved in 10% methanol was applied to the prepared affinity column. After being washed with 10 ml of PBS, the affinity column was eluted with 30% methanol (in the case of reuse of antibody column) or 100% methanol. The 30% methanol-eluted sample was diluted with distilled water to obtain a methanol at concentration of 10%, and the 100% methanol-eluted sample, after being evaporated to dryness, was re-dissolved in 10% methanol. This 10% methanol sample was measured by E2 ELISA (17β-Estradiol ELISA kit, manufactured by Takeda Chemical Industries, distributed by Wako Pure Chemical Industries, Code Number 300-07771) or by E1 ELISA (Hirobe et al., Proceedings of the 36th Annual Meeting of the Japan Society on Water Environment, p. 433 (2002)).

Note that the liquid feed procedure was performed using a peristaltic pump at a flow rate of 1 ml/min.

(3) Effects of column length on the amount of E2 recovered

As shown in Table 1 below, the amount of E2 recovered increases in proportion to column length but the amount of liquid recovered does not increase; it was confirmed that the present method was an efficient recovery method because concentration can be achieved without an increase in the amount of liquid recovered, which is problematic in subsequent concentration and the like.

Table 1

| Membrane length (cm) | Amount of antibody immobilized (mg) | Amount of E2 added (ng) | Amount of liquid recovered (ml) | Amount of E2 recovered (ng) |
|---|---|---|---|---|
| 2 | 0.34 | 50 | 2 | 38 |
| 10 | 1.95 | 250 | 2 | 211 |

(4) Investigation of acceptability of affinity column reuse

The results of an investigation of the acceptability of column reuse are shown in Table 2. It was confirmed that by using 30% methanol as the column eluent, the column could be reused and could be used efficiently. The column had a GMA membrane length of 2 cm, and the column after 30% methanol elution was regenerated by washing with 10 ml of PBS.

Table 2

| Frequency of column use | Amount of E2 added (ng) | Amount of E2 recovered (ng) |
|---|---|---|
| 1 | 50 | 31.1 |
| 2 | 50 | 36.6 |
| 3 | 50 | 28.3 |
| 4 | 50 | 31.4 |
| 5 | 50 | 30.0 |
| 6 | 50 | 29.9 |
| 7 | 50 | 28.9 |
| 8 | 50 | 27.9 |
| 9 | 50 | 33.4 |
| 10 | 50 | 31.5 |

(5) Investigation of acceptability of application to low-concentration samples

For the purpose of directly concentrating environmental water using an affinity column, an addition/recovery experiment was conducted on E1 at a sewage influent level (10 ng/L, 100 ml). The results are shown in Table 3. Even for a sample of a concentration of as low as 10 ng/L, a good result of a recovery rate of 85% was obtained. From this, it was confirmed that this affinity column was usable for direct concentration of an estrogen at a sewage influent level.

Table 3

| Subject of measurement | Concentration (ng/l) | Liquid volume (ml) | Amount added (ng) | Recovery rate (%) |
|---|---|---|---|---|
| E1 | 10 | 100 | 1 | 85 |

Example 4: Recovery of E2 with estrogen affinity column using non-woven fabric

(1) Preparation of affinity column using non-woven fabric

After a GMA non-woven fabric (graft ratio 207%, fabric weight 30 g/$m^2$) prepared using a non-woven fabric of high-density polyethylene (HDPE high-density polyethylene 100%, 3.3 dtex) in place of the hollow fiber membrane of Example 1 was cut (diameter 0.8, 1.2, 2.6 cm), it was filled in a reservoir (3, 6, 60 ml capacity: manufactured by GL Sciences) by being vertically sandwiched with frits (manufactured by GL Sciences). After immersion in each of methanol and distilled water for 30-60 minutes, 2 ml of the anti-estrogen antibody (2 mg/ml in 30mM borate buffer solution (pH 10)) obtained in Reference Example 1 described below was added, and the reaction was carried out at 4°C overnight. By measuring the amount of unbound antibody, the amount of antibody bound to the non-woven fabric was measured. The outlines of the above procedures are shown in Figures 8 and 9.

(2) Estrogen addition/recovery experiment using affinity column

After the above-described affinity column was washed with 10 ml of PBS, E2 dissolved in 10% methanol was applied to the affinity column. After being washed with 10 ml of PBS, the affinity column was eluted with 8 ml of 100% methanol. After the effluent was evaporated to dryness, the residue was re-dissolved in 10% methanol and measured by E2 ELISA. Note that the above-described procedure was performed using a commercially available solid phase extraction apparatus (VAC ELUTE-24: manufactured by GL Sciences) at a flow rate of 3-4 ml/min. An outline of the procedure is shown in Figure 9.

(3) Effects of the number of laminated layers and diameter of GMA non-woven fabric

The effects of the number of laminated layers and diameter of GMA non-woven fabric on E2 recovery rate were examined (E2: 1 ng/L, concentrated from 300 ml to 2 ml). Note that the non-woven fabric of the 1.2 cm diameter was laminated in four levels comprised of 10, 20, 30 and 40 layers, and the non-woven fabrics of the 0.8 and 2.6 cm diameters were laminated in 20, 40, 60 and 80 layers, and in 3, 6, 9 and 12 layers, respectively, so that the non-woven fabric weight would be equal to that at each level for the non-woven fabric of the 1.2 cm diameter. Note that the graft ratio of the GMA non-woven fabric was 207% (fabric weight 30 g/$m^2$).

The results are shown in Table 4. The graft ratio was good at 86% for the 1.2 cm diameter (40 layers) and 90% for the 2.6 cm diameter (12 layers), whereas it was 65% for the 0.8 cm diameter (80 layers). Because the amounts of anti-estrogen antibody bound at the respective diameters were almost equal, it was found that the diameter of GMA non-woven fabric affected the recovery rate, and that the diameter was preferably 1.2 cm or more.

Note that the present experiment was performed at E2: 1 ng/L, which corresponds to a concentration in sewage treatment water, and it was also confirmed that even when a non-woven fabric was used, very low concentrations of estrogen can directly be concentrated.

Table 4

| Diameter (cm) | Number of laminated layers | Non-woven fabric weight (g) | Antibody binding (mg) | E2 recovery rate (0.15 μg/L: 100%) | |
|---|---|---|---|---|---|
| | | | | E2-T measured value (μg/L) | Recovery rate (%) |
| 0.8 | 20 | 0.11 | 1.7 | <0.05 | <36 |
| | 40 | 0.22 | 2.2 | 0.08 | 50 |
| | 60 | 0.34 | 2.5 | 0.09 | 59 |
| | 80 | 0.45 | 3.2 | 0.10 | 65 |
| 1.2 | 10 | 0.13 | 1.7 | 0.05 | 36 |
| | 20 | 0.26 | 1.9 | 0.08 | 56 |
| | 30 | 0.39 | 2.2 | 0.10 | 68 |
| | 40 | 0.52 | 3.1 | 0.13 | 86 |
| 2.6 | 3 | 0.14 | 2.3 | 0.06 | 40 |
| | 6 | 0.29 | 2.8 | 0.09 | 62 |
| | 9 | 0.43 | 2.9 | 0.12 | 82 |
| | 12 | 0.58 | 3.1 | 0.14 | 90 |

(4) Effects of non-woven fabric density and graft ratio

The effects of non-woven fabric density and graft ratio (amount of GMA introduced/substrate membrane weight ×100 (%)) on E2 recovery rate were examined (E2: 3 ng/L, concentrated from 100 ml to 2 ml). Note that all non-woven fabrics were laminated in 30 layers. The results are shown in Table 5. At constant graft ratios (50 to 150%), the recovery rate increased as the membrane density increased. On the other hand, it was found that even with the membrane of the lowest density of 30 g/$m^2$, a sufficient recovery rate could be obtained, provided that the graft ratio was 200% or so.

Table 5

| Effects of density and graft ratio | | | | |
|---|---|---|---|---|
| Density (g/m$^2$) | Graft ratio (%) | Amount of antibody bound (mg) | E2 measured value (µg/L) | E2 recovery rate (%) |
| 100 | 58<br>93<br>148 | 2.5<br>2.4<br>2.9 | 0.117<br>0.122<br>0.118 | 78<br>81<br>79 |
| 65 | 47<br>97<br>154 | 1.3<br>1.6<br>1.5 | 0.089<br>0.091<br>0.095 | 59<br>61<br>63 |
| 30 | 145<br>207 | 1.2<br>1.6 | 0.073<br>0.153 | 49<br>102 |

Example 5: Recovery of DBP with resin plasticizer affinity column using non-woven fabric

(1) Preparation of affinity column using non-woven fabric

After a GMA non-woven fabric produced according to Example 4(graft ratio 207%, fabric weight 30 g/m$^2$) was cut (diameter 1.2 cm), it was filled in a reservoir (6 ml capacity: manufactured by GL Sciences) by being vertically sandwiched with frits (manufactured by GL Sciences). After immersion in each of methanol and distilled water for 30 to 60 minutes, 2 ml of the anti-resin plasticizer antibody (2 mg/ml in 30mM borate buffer solution (pH 10)) obtained in Reference Example 1 described below was added, and the reaction was carried out at 4°C overnight. By measuring the amount of unbound antibody, the amount of antibody bound to the non-woven fabric was measured.

(2) Dibutyl phthalate (DBP) addition/recovery experiment using affinity column

After the above-described affinity column was washed with 10 ml of PBS, dibutyl phthalate (DBP) dissolved in 10% methanol was applied to the affinity column. After being washed with 10 ml of PBS, the affinity column was eluted with 8 ml of 100% methanol. After the effluent was evaporated to dryness, the residue was re-dissolved in 10% methanol and measured by DBP ELISA. Note that the above-described procedure was performed using a commercially available solid phase extraction apparatus (VAC ELUTE-24: manufactured by GL Sciences) at a flow rate of 3 to 4 ml/min.

As shown in Table 6, when 300 ng of DBP was applied to the column, 167 ng was recovered. From this, it was found that by changing the kind of the anti-endocrine disruptor polyclonal antibody to be carried, the present technique could be applied to recovery of various substances.

Table 6

| Investigation of DBP affinity column | | | | |
|---|---|---|---|---|
| Non-woven fabric diameter (cm) | Number of laminated layers (layers) | Amount of antibody bound (mg) | Amount of DBP added (ng) | Amount of DBP recovered (ng) |
| 1.2 | 40 | 3 | 300 | 167 |

Example 6: Recovery of bisphenol A with resin component affinity column using non-woven fabric

(1) Preparation of affinity column using non-woven fabric

After a GMA non-woven fabric produced according to Example 4 (graft ratio 170-190%, fabric weight 65 g/m$^2$) was cut (diameter 1.2 cm), it was filled in a reservoir (6 ml capacity: manufactured by GL Sciences) by being vertically sandwiched with frits (manufactured by GL Sciences). After immersion in each of methanol and distilled water for 30-60 minutes, 2 ml of the anti-resin component antibody obtained in Reference Example 1 described below (5 mg/ml in PBS (pH 7.4)) was added, and the reaction was carried out at 4°C overnight. By measuring the amount of unbound antibody, the amount of antibody bound to the non-woven fabric was measured.

(2) Bisphenol A (BPA) addition/recovery experiment using affinity column

After the above-described affinity column was washed with 10 ml of PBS, 10 ng of bisphenol A (BPA) dissolved in distilled water (concentration 10 ng/l) was applied to the affinity column. After being washed with 10 ml of PBS, the affinity column was eluted with 8 ml of 100% methanol. After the effluent was evaporated to dryness, the residue was re-dissolved in 10% methanol and measured by BPA ELISA. Note that the above-described procedure was performed using a commercially available solid phase extraction apparatus (VAC ELUTE-24: manufactured by GL Sciences) at a flow rate of 3 to 4 ml/min.

(3) Results

As shown in Table 7, when 10 ng (concentration 10 ng/l) of BPA was applied to the affinity column, 8.5 ng (concentration 1.06 ng/ml×8 ml) of BPA was recovered. From this, it was shown that even in BPA, BPA could be recovered by the present technique, provided that an anti-resin component antibody is used as the antibody to be carried.

Table 7

| Investigation of BPA affinity column | | | | |
|---|---|---|---|---|
| Non-woven fabric diameter (cm) | Number of laminated layers (layers) | Amount of antibody bound (mg) | Amount of BPA added (ng) | Amount of BPA recovered (ng) |
| 1.2 | 30 | 3.4 | 10 | 8.5 |

Hence, from the results of Examples 4, 5 and 6, it was confirmed that by changing the kind of the anti-endocrine disruptor antibody to be carried, the present technique could be applied to recovery of various substances.

**Example 7**

With the anti-estrogen (ES) antibody produced in Reference Example 1 described below as the model antibody, an E2 binding/elution test of an anti-endocrine disruptor antibody-immobilized carrier was performed using an anti-endocrine disruptor antibody-immobilized carrier according to the present invention and a commercially available carrier.

[Methods]

[Method of antibody binding to column]

(1) Commercially available columns:

Affigel-10 (manufactured by Bio-Rad, 153-6099),
Affigel-hydrazide (Hz) (manufactured by Bio-Rad, 153-6060)

In accordance with the instruction manuals for the columns, anti-ES antibody-immobilized columns were prepared.

(2) Grafted hollow fiber membrane (GMA fiber)

After a GMA fiber produced according to Example 1 was washed with methanol and distilled water, an anti-ES antibody (anti-ES-1 rabbit polyclonal antibody) dissolved in PBS was reacted overnight to introduce the antibody to the graft polymerized epoxy group.

[Sample application to column and elution]

As the sample, 17β estradiol (E2) dissolved in distilled water was used. For elution from the column, 100%

methanol was used.

[E2 measurement in eluate]

After the eluate was evaporated to dryness under nitrogen purge, E2 in the sample was measured using a commercially available E2 ELISA kit (manufactured by Takeda Chemical Industries).

[Results]

[Amount of E2 bound to column]

The amount of E2 adsorbed per 1 ml for the commercially available columns, and the amount of E2 adsorbed per 20 mm for the grafted hollow fiber membrane, were examined. As a result, all carriers had 33-35 ng or so of E2 bound thereto, and were judged to be equivalent in terms of the amount of E2 bound (Table 8).

Table 8

| Amounts of E2 adsorbed to individual columns | |
|---|---|
| Carrier | Amount of E2 adsorbed (ng) |
| Affigel-10 (1 ml) | 35 |
| Affigel-Hz (1 ml) | 36 |
| GMA fiber (20 mm) | 33 |

[Comparison of patterns of E2 elution from respective columns]

The E2 effluent from each column (100% methanol) was fractionated in 1 ml increments, and the pattern of E2 elution from the column was examined. As a result, it was found that compared to commercially available columns (Affigel-10, Affigel-Hz), elution could be conducted with a smaller amount of eluent using GMA fiber (Figure 10).

From the results above, it was shown that the E2 adsorption capabilities of the individual anti-ES antibody immobilized carriers were similar, but when viewed from the elution patterns, GMA fiber produced the sharpest pattern compared to the other carriers and hence exhibited a remarkably high concentration efficiency. Furthermore, GMA fiber was also excellent in handling aspects such as antibody binding procedural simplicity.

**Reference Example 1:** Preparation of anti-endocrine disruptor polyclonal antibody

In Figure 11, an outline of a method of preparation with reference to preparation of an anti-estrogen antibody as an example, is shown.

(1) Preparation of hapten

i) Preparation of hapten (E3-16G) for acquisition of estrogen polyclonal antibody

Estriol 16α-(β-D-glucuronide) (manufactured by SIGMA, Product No. E-1877) (hereinafter abbreviated as E3-16G) was purchased and used as the hapten (E3-16G).

ii) Preparation of hapten (DEHP-1) for acquisition of resin plasticizer polyclonal antibody

One synthesized as described below was used as the hapten (DEHP-1).

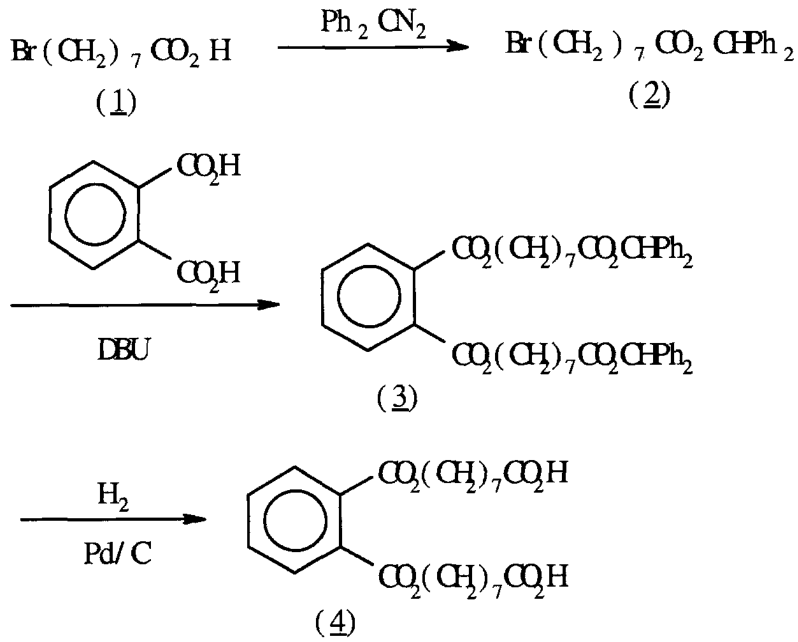


[First step: synthesis of diphenylmethyl ester]

10 g of 8-bromooctanoic acid (1) was dissolved in 300 ml of tetrahydrofuran; under ice cooling, about 20 ml of a diphenyldiazomethane solution was added and the reaction was carried out for 1 hour; further the reaction was carried out at room temperature with stirring overnight; the reaction product was concentrated under reduced pressure, applied to a silica gel column (300 g), and eluted with hexane-ethyl acetate (9:1) to yield 20 g of a light-yellow oily substance (crude product 2) .

[Second step: phthalic acid esterification using DBU]

20 g of 8-bromooctanoic acid diphenylmethyl ester (crude product 2), 2.42 g of phthalic acid and 1,8-diazabicyclo[5,4,0]-7-undecene (DBU) were reacted with refluxing under heating in 60 ml of benzene overnight, further 2.2 g of DBU was added, and the reaction mixture was refluxed under heating for 6 hours and cooled to room temperature, after which water and chloroform were added and stirring was conducted, after which the liquid portion was separated, washed with water, and dried with Glauber's salt, after which the reduced-pressure concentrate was applied to a silica gel column (300 g) and eluted with hexane-ethyl acetate (9:1) and then with hexane-ethyl acetate (2:1) to yield 8.2 g of phthalic acid ester (3).

[Third step: de-diphenylmethylation by catalytic reduction]

4.1 g of phthalic acid ester (3) was dissolved in 100 ml of THF, 0.4 g of 10% Pd/C (50% wet product) was added, $H_2$ was sparged at 0.3 ml/min for 5 hours, further 1.2 g of 10% Pd/C (50% wet product) was added, and $H_2$ was sparged at 0.5 ml/min for 2 hours. After the catalyst was filtered off, the reduced-pressure concentrate was applied to an ODS column (Daiso sp-120 30/50 μm; 3×50 cm) and eluted with 75% MeOH to yield 2.1 g of a light-yellow oily substance. This was dissolved in ethyl acetate and filtered, after which it was concentrated under reduced pressure, dissolved in ether, and concentrated under reduced pressure to yield light-yellow oily substance (4). The desired structure was identified by 1H-NMR, and the HPLC purity was 99% or higher.

iii) Preparation of hapten (BPA-2) for acquisition of resin component polyclonal antibody

One synthesized as described below was used as the hapten (BPA-2).

[First step]

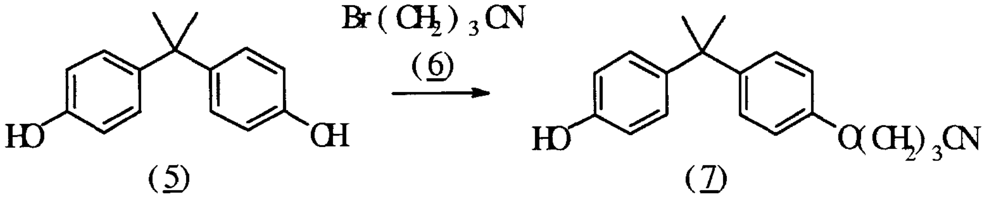


1.5 g (6.57 mmol) of bisphenol A (5), 0.97 g (6.57 mmol) of 4-bromobutyronitrile (6) and 0.65 g (6.57 mmol) of potassium carbonate were dissolved in 20 ml of dry dimethylformamide (DMF), and stirred at room temperature overnight. The obtained reaction product was extracted with 50 ml of methyl-t-butyl ether (MTBE), 50 ml of saturated brine and 50 ml of water, washed with saturated brine (50 ml×3), dried with sodium sulfate, and concentrated to yield a colorless oil (2.23 g). This was subjected to silica gel (Daisogel IR-60; 100 g) column chromatography with chloroform/methanol=10:1 as the developing solvent, and further subjected to silica gel (Merck Kieselgel 60; 50 g) column chromatography with chloroform/methanol=10:1 as the developing solvent. Next, this was subjected to silica gel (Merck Kieselgel 60; 50 g) column chromatography with chloroform as the developing solvent to yield 0.6 g of white crystal (7) (2.03 mmol, percent yield 31%).

[Second step]

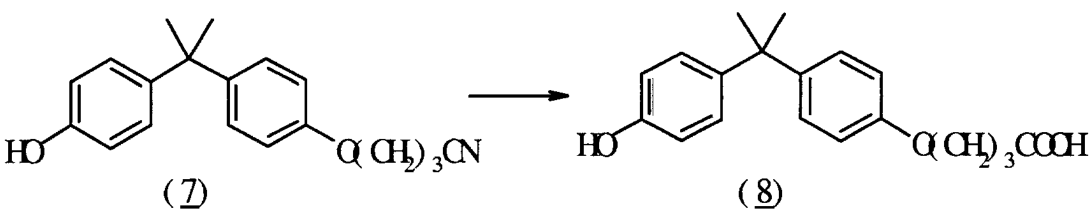


0.6 g (2.03 mmol) of bisphenol A derivative (7), 0.57 g (10.2 mmol) of potassium hydroxide, 10 ml of ethanol and 10 ml of water were mixed and stirred at 80°C overnight. After the ethanol was distilled off, the solution was acidified with 1N hydrochloric acid and extracted with 20 ml of ethyl acetate. The extract was washed with saturated brine (50 ml×2), dried with sodium sulfate, and concentrated to yield a white crystal (0.5 g). After being washed with isopropyl ether and hexane, the white crystal was subjected to silica gel (Daisogel IR-60; 50 g) column chromatography with chloroform/methanol=10/1 as the developing solvent, then subjected to silica gel (Daisogel IR-60; 50 g) column chromatography with chloroform/methanol=1/0→10/1 as the developing solvent, and washed with hexane, to yield 159 mg of white crystal (8) (0.50 mmol, percent yield 25%, HPLC purity 97.8%).

(2) Preparation of immunogen

0.10 mmol of hapten was dissolved in 1 ml of dimethylsulfoxide (DMSO) (Solution A). 0.14 mmol of 1-ethyl-3-(3-dimethylaminopropyl)carbodiimide hydrochloride (WSC) and 0.14 mmol of N-hydroxysuccinimide (NHSI) were dissolved in 1 ml of DMSO (Solution B). Solution A was added drop by drop to Solution B, and these were reacted at room temperature with stirring overnight to prepare a hapten activated ester.

10 mg (0.145 μmol) of Keyhole Limpet Hemocyanin (KLH) was dissolved in 1 ml of 0.13 mol/L $NaHCO_3$, the above-described hapten activated ester was added to have a molar ratio of hapten:KLH=50:1, and these were reacted at 4°C with stirring overnight. Next, the reaction mixture was dialyzed at 4°C against PBS; the obtained antigen for immunization, after the protein concentration was measured using the BCA Protein Assay Kit (manufactured by PIERCE), was dispensed at 0.5 mg/tube and stored at -20°C.

(3) Preparation of labeled antigens

5 mg (0.118 μmol) of Horse Radish Peroxidase (HRP) (for EIA, Boehringer Mannheim)) was dissolved in 5 ml of 0.13M $NaHCO_3$, and the above-described hapten-activated ester was added to obtain a molar ratio of hapten: enzyme=5:1. After an overnight reaction at 4°C, the reaction mixture was concentrated by ultrafiltration using Centricon YM-30 (Amicon), which has a molecular weight cutoff value of 30,000, washed with PBS thee times, to yield an enzyme-labeled antigen of each endocrine disruptor.

(4) Preparation of antigen for antibody titer measurement

10 mg (0.145 μmol) of ovalbumin (Ovalbumin, OVA) was dissolved in 1 ml of 0.13 mol/L $NaHCO_3$, the above-described hapten activated ester was added to have a molar ratio of hapten:OVA=50:1, and these were reacted at 4°C with stirring overnight. Next, the reaction mixture was dialyzed at 4°C against PBS; the obtained antigen for antibody titer measurement, after the protein concentration was measured using a BCA Protein Assay Kit (PIERCE), was dispensed at 0.5 mg/tube, and stored at -20°C.

(5) Preparation of polyclonal antibody

1) Immunization

0.5 mg of the immunogen (hapten-KLH) was dissolved in 1 ml of phosphate-buffered physiological saline (PBS), this solution was mixed with an equal volume of Freund's complete Adjuvant (FCA) (manufactured by Difco), and the entire volume was subcutaneously injected in several sites in one rabbit. Subsequently, the same immunization was conducted at 2-week intervals. Freund's complete adjuvant was used for second immunization, and Freund's incomplete Adjuvant (FIA) was used for third immunization and beyond.

2) Serum sampling

Following third immunization, a serum was sampled from the marginal ear vein from the rabbit one week after immunization. To the sampled serum, sodium azide was added to obtain a 0.1% concentration, and the sample was stored under refrigeration until the time of antibody titer measurement.

3) Measuring antibody titer

[Preparation of assay plate]

Hapten-OVA (500 μg) was diluted with 50 ml of PBS. This was added to a 96-well microplate (Costar) at 100 μl/well. After storage at 4°C overnight, the microplate was twice washed with 300 μl/well of PBS. Block Ace (Dainippon Pharmaceutical, product number UK-B25) (containing 0.01% thimerosal), 4-fold diluted with PBS, was added at 200 μl/well. This was used after being stored at 4°C overnight or more.

[Dilution of enzyme-labeled secondary antibody]

An enzyme-labeled secondary antibody (PEROXIDASE-CONJUGATED GOAT IGG FRACTION TO MOUSE IGG (WHOLE MOLECULE, ICN Pharmaceutical, Inc. Catalog # 55550), 50 μl portions, were dispensed to tubes and stored at -20°C (enzyme-labeled secondary antibody stock solution). One tube was dissolved in 2.5 ml of PBS, and 100 μl portions were dispensed to tubes and stored at -20°C (enzyme-labeled secondary antibody diluted solution).

[Color development substrate]

As the substrate kit for HRP, a peroxidase substrate kit (Bio-Rad, Cat. No. 172-1067) was used.

[Measuring antibody titer]

Using a microplate for dilution (Nunc), the serum sample was serially diluted with PBS (0.05% Tween 20 added), started at the 200 fold rate (120 μl/well), and added to a microplate for assay washed with 300 μl/well of PBS three times, at 100 μl/well. A plate seal (Sumitomo Bakelite, product number MS-300020) was applied and the reaction was carried out at room temperature for 1 hour. After the microplate was washed with 300 μl/well of PBS three times,

100 μl of the enzyme-labeled secondary antibody diluted solution was dissolved in 10 ml of PBS, and 5000-fold diluted enzyme-labeled secondary antibody was added at 100 μl/well. After a plate seal was applied and the reaction was carried out at room temperature for 1 hour, the microplate was washed with 300 μl/well of PBS three times, a color was developed by the method described in the substrate kit, and the absorbance was measured using a plate reader.

4) Antibody purification from rabbit antiserum

[Ammonium sulfate precipitation]

After whole blood was drawn from rabbits with elevated antibody titers, an antiserum was collected and 2 fold diluted with physiological saline, after which saturated ammonium sulfate (100%) was added to obtain a 40% concentration. After mixing at room temperature for 1 hour, centrifugation (10000 rpm, 20 min) was conducted and a precipitate was collected. After the precipitate was dissolved in physiological saline in a manner not causing bubbling, it was dialyzed sequentially against water, physiological saline, and 10mM phosphate buffer solution (pH 8.0).

[Purification by ion exchange chromatography]

Per 30 ml of serum, 60 ml of DE-52 cellulose (manufactured by Whatman) was packed, and the dialysate was applied. About 1 L of 10mM phosphate buffer solution (pH 8.0) was fed over 24 hours, and the IgG fraction was collected. The collected IgG fraction, after being dispensed at 5 mg/tube, was stored at -20°C.
An outline of the results of the above steps is as follows.

Table 9

| Immunogen | Immunization number | Antibody titer | Amount of purified antibody (mg/rabbit) |
|---|---|---|---|
| E3-16G-KLH | 3 | +++ | 168 |
| DEHP-1-KLH | 3 | ++ | 252 |
| BPA-2-KLH | 6 | ++ | 178 |

In the E3-16G-KLH-immunized rabbits, third immunization (immunization number 3) led to an equilibrium of antibody titer, and 168 mg of purified anti-estrogen polyclonal antibody was obtained from one rabbit. When assuming E2=100% for this purified antibody, cross reactivity was as shown in Table 10; as cross reactivity was found to 17β-estradiol (E2), estrone (E1), estriol (E3) and the like, it was proven that all estrogens (female sex hormones) can be concentrated or cleaned up.

Table 10

| Compound | Cross reactivity (%) |
|---|---|
| 17β-Estradiol (E2) | 100 |
| Estrone (E1) | 100 |
| Estriol (E3) | 69 |
| Ethynylestradiol (EE2) | 7 |
| Bisphenol A (BPA) | <0.02 |
| t-Nonylphenol (NP) | <0.02 |
| Nonylphenol ethoxylate (NP10EO) | <0.02 |
| Diethylhexyl phthalate (DEHP) | <0.02 |
| 2,4-Dichlorophenol (2,4DCP) | <0.02 |

Also, even in the DEHP-1-KLH-immunized rabbits, third immunization (immunization number 3) led to an equilibrium of antibody titer, and 252 mg of purified anti-resin plasticizer antibody was obtained from one rabbit. When assuming DBP=100% for this purified antibody, cross reactivity was as shown in Table 11; as cross reactivity was found to various resin plasticizers, from those with 2 alkyl chains (ethyl) to those with 7 alkyl chains (hexyl), it was proven that many resin plasticizers can be concentrated or cleaned up.

Table 11

| Resin plasticizer | Abbreviation | Cross reactivity (%) |
|---|---|---|
| Diethyl phthalate | DEP | 7 |
| Dipropyl phthalate | DPrP | 27 |
| Dibutyl phthalate | DBP | 100 |
| Butylbenzyl phthalate | BBP | 11 |
| Dipentyl phthalate | DPnP | 10 |
| Dicyclohexyl phthalate | DCHP | < 1 |
| Dihexyl phthalate | DHP | 4 |
| Diethylhexyl phthalate | DEHP | < 1 |
| Diethylhexyl adipate | DEHA | < 1 |
| Diisononyl phthalate | DINP | < 1 |

Also, even in the BPA-2-KLH-immunized rabbits, sixth immunization (immunization number 6) led to an equilibrium of antibody titer, and 178 mg of purified anti-resin component antibody was obtained from one rabbit. When assuming BPA=100% for this purified antibody, cross reactivity was as shown in Table 12; as specificity for BPA was high, it was proven that BPA only can be specifically concentrated or cleaned up.

Table 12

| Compound | Cross reactivity (%) |
|---|---|
| Bisphenol A (BPA) | 100 |
| Bisphenol E (BPE) | 6.6 |
| Bis(p-hydroxyphenyl)methane | 1.0 |
| Bisphenol B (BPB) | 22.5 |
| 2,2'-Bis(4-hydroxyphenyl)-1-propanol | 9.3 |
| BPA diacetate | 11.1 |
| 1,2-Bis(4-hydroxyphenyl)-2-propanol | <0.4 |
| 4,4'-Bis(p-hydroxyphenyl)pentanoic acid | <0.4 |
| 4,4'-Dihydroxydiphenyl ether | <0.4 |
| p,p'-Dihydroxybenzophenone | <0.4 |
| Bisphenol S (BPS) | <0.4 |
| Bis[4-(2-hydroxyethoxy)phenyl]sulfone | <0.4 |
| BPA dimethacrylate | 2.7 |
| BPA diglycidyl ether | <0.4 |
| BPX-33 | <0.4 |

**Reference Example 2**

1. Acquisition of monoclonal antibodies

1?1 Production of hapten

(1) Production of hapten for ethynyl estradiol antibody

Ethynyl estradiol (EE2; 1.0 g) and sodium methylate (0.76 g) were dissolved in ethanol (35 ml). After adding

monochloracetic acid (0.41g), the solution was refluxed under heating for 22 hr. After concentration under reduced pressure, water and ethyl acetate (about 100 ml each) were added to partition the solution. The aqueous layer was washed with ethyl acetate (50 ml) and acidified (pH 1 to 2) with conc. hydrochloric acid. After extracting with ethyl acetate (100 ml and 50 ml), the organic layer was washed with saturated brine (30 ml) and dried over anhydrous sodium sulfate. The residue was concentrated under reduced pressure and stood at -20°C for 2 days to allow a part thereof to be crystallized. This part was taken and used as a seed crystal. The concentrated solution was dissolved in a small amount of acetone, and hexane and the seed crystal were added to allow precipitation of crystals. The crystals were collected by filtration and vacuum dried to yield an object product, EE2-3-carboxymethylether (EE2-3CME).

(2) Production of hapten for 17β-estradiol antibody

17β-Estradiol (E2; 1.0 g) and sodium methylate (0.82 g) were dissolved in ethanol (35 ml). After adding monochloracetic acid (0.45 g), the solution was refluxed under heating for 2 days. After concentration under reduced pressure, water (about 300 ml) and ethyl acetate (about 200 ml) was added to partition the mixture. The aqueous layer was washed with ethyl acetate (50 ml) and acidified (pH 1 to 2) with concentrated hydrochloric acid. After extracting twice with ethyl acetate (100 ml), the organic layer was washed with saturated brine (30 ml) and dried over anhydrous sodium sulfate. The residue was concentrated under reduced pressure and dissolved in a small amount of acetone. Isopropyl ether (IPE) was added to allow precipitation of crystals. The crystals were collected by filtration, washed with IPE and vacuum dried to yield an object product, E2-3-carboxymethylether (E2-3CME).

(3) Production of hapten for estrone antibody

Estradiol-3,17-diacetate (E2-3,17-diAce, 20 g) was dissolved in acetic acid (25 ml). While cooling the solution, 4/5 of a mixture of anhydrous chromic acid (9 g), acetic acid (27 ml) and water (4 ml) was gradually added. After stirring overnight, water was added and the mixture was extracted with chloroform, washed with potassium carbonate solution, dried over sodium sulfate and concentrated under reduced pressure. The residue was dissolved in benzene, applied to column chromatography (Wako gel 2w) and eluted with hot benzene to give crude 6-oxo-E2-3,17-diAce (starting material was removed while confirming by TLC). This substance was dissolved in 2-methoxy-ethanol, the same amount of 20% NaOH was added and reacted at 100°C for 1 hr. After acidifying with dilute hydrochloric acid, the reaction mixture was eluted with ethyl acetate, dried over sodium sulfate, and concentrated under reduced pressure to allow recrystallization (6oxo-E2) from methanol. 6oxo-E2 (300 mg) was dissolved in ethanol (2 ml) and potassium carbonate fine powder (50 mg) was added. Benzyl bromide (50 μl) was added, the mixture was stirred (70°C, 2 hr) and concentrated, which was recrystallized from methanol (6-oxo- E2-3-benzyl ether). 6-oxo-E2-3-benzyl ether was dissolved in methanol, and carboxymethoxylamine?1/2 HCl and sodium methoxide (1.2 molar amount) were dissolved in a small amount of water, diluted with methanol and added. After reacting at room temperature for 1 hr, 2/3 was evaporated, and the resulting solution was acidified with water and HCl, extracted with ethyl acetate, and dried over sodium sulfate. The residue was dissolved in hexane, purified by silica gel column eluting with methanol and recrystallized from methanol. A recrystallized substance (0.6 g) was dissolved in acetone (2 ml) and a half of a mixture of anhydrous chromic acid (1 g), acetic acid (5 ml) and water (1 ml) was added. After generation of heat, the mixture was extracted with ethyl acetate and evaporated to dryness (6-oxo-E1-3-benzyl ether). 6-Oxo-E1-3-benzyl ether (400 mg) was dissolved in methanol (5 ml), 5% Pd?C (100 mg) was added and the mixture was stirred while blowing hydrogen. After reacting for 1 hr, the reaction mixture was evaporated to dryness, which was recrystallized from methanol to yield an object product, 6-oxo-E1-6carboxymethyloxime (E1-6CMO).

1-2 Preparation of immunogen

For each of 3 kinds of haptens obtained in 1-1, hapten (0.1 mol), water-soluble carbodiimide (0.14 mol) and N-hydroxysuccinimide (0.14 mol) were reacted in dimethyl sulfoxide (2 ml) overnight to produce an activated ester. Then, keyhole limpet hemocyanin (KLH; 10 mg) was dissolved in 0.13 M sodium bicarbonate ($NaHCO_3$) solution. After adding said activated ester (200 μl), the mixture was reacted at 4°C overnight. Unreacted reagents were removed by dialysis against Dulbecco's phosphate buffer (PBS), and the resultant was stored in a frozen state as immunogen.

1-3 immunization

The immunogen obtained in 1-2 was dissolved in PBS to be at concentration of 500 μg/ml, and added to an equal amount of Freund's adjuvant or RIBI adjuvant system. The mixture was sufficiently emulsified, and subcutaneously administered to a BALB/C mouse (female) at 100 μg/mouse. Additional immunizations were performed at 2 weeks (Freund's) or 3 weeks (RIBI) interval. After 5-6 additional immunizations, the immunogen (50 μg/0.1 ml PBS/mouse) was intravenously administered to the individual showing the highest serum antibody titer as final immunization.

1-4 Cell fusion

A spleen was removed from the mouse in which the final immunization was performed in 1-3 (3 days after the final immunization), and spleen cells were prepared. Separately cultured mouse myeloma cells and the spleen cells were contacted in the proportion of 1:5 in the presence of polyethylene glycol (average molecular weight 4,000) and carried cell fusion to give fused cells (hybridoma). These hybridomas were suspended in HAT medium, plated into 96-well microplate and cultured in a carbonic acid gas incubator (37°C, 5% $CO_2$).

1-5 Screening of hybridomas

(1) Production of assay plate

A goat anti-mouse IgAGM antibody (manufactured by ICN/Cappel, #55461) was dissolved in PBS at concentration of 50 µg/ml, and added into microplate at concentration of 100 µl/well. The plate was reacted at 4°C overnight, and washed with PBS containing 0.05% Tween20 (T-PBS) (300 µl/well, twice). Then, BlockAce (Snow Brand Milk Products Co., Tokyo) 4-fold diluted with PBS was added (200 µl/well). The plate was reacted at 4°C overnight or more, and cold-stored until it was used.

(2) Production of antigen-enzyme conjugate (labeled antigen)

For each of 3 kinds of haptens produced in 1-1, hapten (0.1 mol), water-soluble carbodiimide (0.14 mol) and N-hydroxysuccinimide (0.14 mol) were reacted in dimethyl sulfoxide (2 ml) overnight to produce an activated ester. Then, a horseradish peroxydase (HRP; 10 mg) was dissolved in 0.13 M sodium bicarbonate ($NaHCO_3$) solution (10 ml), said activated ester (15 µl) was added, and the mixture was reacted at 4°C overnight. Unreacted reagents were removed by ultrafiltration, and the resultant was cold-stored at concentration of 3 mg/ml.

(3) Primary screening (antigen binding ability test)

In the microplate into which the cells were plated in 1-4, the culture solution (100 µl) in the well in which cell growth was confirmed was added to the assay plate produced in (1) (after washing with PBS (or T-PBS) (300 µl/well, twice)). The plate was reacted at room temperature for 1 hr, and washed with T-PBS (300 µl/well, 3 times). The labeled antigen against the objective antibody produced in 1-5(2) was 5000-fold diluted with T-PBS, and added to the microplate. The plate was reacted at room temperature for 1 hr, and washed with T-PBS (300 µl/well, 3 times). TMB peroxydase substrate kit (Bio-Rad Japan, Tokyo, #172-1066: hereinafter, chromogeic substrate) (100 µl/well) was added, and the mixture was reacted at room temperature for 30 min. Then, 1 N phosphoric acid (100 µl/well) was added to stop the chromogenic reaction. The absorbance at wavelength of 450 nm was read off. The cells showing absorbance over 1 were scaled-up to 24-well microplate.

(4) Secondary screening (female sex hormone inhibitory test)

For the cells scaled-up to 24-well microplate in 1-5(3), the culture solution (100 µl each) in the well in which sufficient cell growth was confirmed was added into plural wells of the assay plate produced in (1) (after washing with PBS (or T-PBS) (300 µl/well, twice)). The plate was reacted at room temperature for 1 hr, and washed with T-PBS (300 µl/well, 3 times). The measuring object female sex hormone (1 ng/ml in 10% methanol (hereinafter, MeOH)) or 10% MeOH alone (control) and the labeled antigen against the objective antibody (5000-fold diluted with T-PBS) were mixed in equal amounts and added to the microplate. The plate was reacted at room temperature for 1 hr, and washed with T-PBS (300 µl/well, 3 times). The chromogeic substrate (100 µl/well) was added, and the mixture was reacted at room temperature for 30 min. Then, 1 N phosphoric acid (100 µl/well) was added to stop the chromogenic reaction. The absorbance at wavelength of 450 nm was read off. A cloning was carried out for the cells that reduction of absorbance by not less than 20% in comparison to the control, in the presence of female sex hormone, was confirmed according to a conventional method, resulting in obtaining hybridoma lines producing the objective antibody as follows.

(1) Anti-estradiol monoclonal antibody-producing mouse hybridoma cell E2-73 line (FERM BP-7569),
(2) Anti-estrone monoclonal antibody-producing mouse hybridoma cell E1-420 line (FERM BP-7568),

(3) Anti-ethynyl estradiol monoclonal antibody-producing mouse hybridoma cell
EE2-227 line (FERM BP-7567),
(4) Anti-ethynyl estradiol monoclonal antibody-producing mouse hybridoma cell
EE2-3 line
(The resistance to a detergent of the antibody obtained from this line is the same as that of one from EE2-277 line.)
(5) Ethynyl estradiol anti-monoclonal antibody producing mouse hybridoma cell
EE2-8-18 line
(The resistance to a detergent of the antibody obtained from this line is slightly lower than that of one from EE2-277 line.)

The above-mentioned mouse hybridoma cell E2-73 line (FERM BP-7569), mouse hybridoma cell E1-420 line (FERN BP-7568), and mouse hybridoma cell EE2-227 line (FERM BP-7567) have been deposited at National Institute of Advanced Industrial Science and Technology, International Patent Organism Depository (Central No. 6, 1-1, Higashi 1-chome, Tsukuba-shi, Ibaragiken, Japan) under the Budapest treaty since April 25, 2001 with the above-mentioned deposit numbers.

Reference Example 3

(1) Determination of N-terminal amino acid sequence of anti-estradiol monoclonal antibody

The antibody protein purified from the culture supernatant of anti-estradiol monoclonal antibody-producing mouse hybridoma cells (E2-73 line) (FERM BP-7569) using Protein A column (PROSEP-A, purchased from Air Brown Co.) was separated and purified to heavy chain and light chain by SDS-polyacrylamide gel electrophoresis, and transferred onto PVDF membrane. After staining with Ponso-S, protein bands were excised. The excised proteins on the membrane were treated with Pfu Pyroglutamate Aminopeptidase (purchased from Takara Shuzo Co.) (2 mU) at 50°C for 17 hr, and their N-terminal amino acid sequences were determined with gas phase protein sequencer (Model 494 Applied Biosystems Japan). The following results were obtained.

E2-73 heavy chain: VQLQQSGAELARPGASVKLS

E2-73 light chain: DIVMTQSPTFLSTSLGDKIT

(2) Purification of anti-estradiol monoclonal antibody mRNA

Anti-estradiol monoclonal antibody producing mouse hybridoma cells (E2-73 line) (FERM BP-7569) ($1X10^7$ cells/ml) were suspended in RNA preparation solution (ISOGEN solution, purchased from Wako Pure Chemical Industries, Ltd.) (1 ml) and disrupted, and RNA was extracted from the cells according to the manual. The concentration of RNA was determined from absorbance at 260nm. From the obtained RNA (about 100 μg), mRNA (about 1.5 μg) was obtained using mRNA purified kit (Oligotex-dT30 <super> mRNA Purification kit, purchased from Takara Shuzo Co.).

(3) Acquisition of cDNA fragments of anti-estradiol monoclonal antibody heavy chain and light chain variable regions and determination of their base sequences as well as deduction of amino acid sequences of heavy chain and light chain variable regions

The synthesis of cDNAs was carried out using about 100 ng of mRNA each for light chain and heavy chain, with First Strand cDNA Synthesis Kit ReverTra Ace -α™ (purchased from Toyobo Co.). Then, amplification of each cDNA fragment was carried out by PCR reaction in Personal cycler (purchased from Biometra) (94°C 30 sec, 55°C 30 sec, 72°C 2 min; Repeat number: 30 cycles), using primers for heavy chain and primers for light chain shown in the following, which were devised from their N terminal amino acid sequences obtained in Example 1, and Pfu Turbo (purchased from Toyobo Co.) enzyme.
Primers for E2-73 heavy chain <B: C, G, T>

Forward 5'- CTATGCGGCCCAGCCGGCCATGGTBCAGCTGCAGCAGTCTGG -3'
(SEQ ID NO: 25)

Back 5'- ACCACTCTCACAGTCTCCTCGG -3' (SEQ ID NO: 26)

Primes for E2-73 light chain <Y: C, T>

Forward 5'- CTCAGTCTCCAGAYATCGTGATGACYCAGTCT -3' (SEQ ID NO: 27)

Back 5'- GGGACCAAGCTGGAAATAAAACGGA -3' (SEQ ID NO: 28)

Thus, cDNA fragments for variable regions of heavy chain and light chain of anti-estradiol monoclonal antibody were obtained. Each fragment was purified from 2% agarose after electrophoresis using a micro spin column (purchased from Amersham Pharmacia Biotech Co.). Then, using dideoxy method, the base sequence of each variable region cDNA was determined, and the amino acid sequence was deduced therefrom. The base sequences were determined by DNA sequencer 310 (Applied Biosystems Japan), after the reaction in a Personal cycler (purchased from Biometra) with Big Dye Terminator Cycle Sequencing Ready Reaction Kit (purchased from Applied Biosystems Japan) using the above-mentioned PCR primers as sequencing primers.

(4) Conversion of anti-estradiol monoclonal antibody heavy chain and light chain variable region cDNAs to single chain (single chain variable region antibody; scFv) and construction of plasmid containing scFv fragment

The obtained fragments of heavy chain and light chain were ligated with a peptide linker ((GGGGS)X3) to give a single chain (scFv). The scFv DNA fragment was ligated to pUC19 cut with *Hinc*II using a ligation kit (purchased from Takara shuzo). After the reaction, the mixture was added to JM109 competent cells (purchased from Toyobo) to allow transformation. The plasmid was purified from the transformant, the complete base sequence of the cDNA insert was determined using dideoxy method. Then, the identity to the base sequences of the above-mentioned variable regions was confirmed (SEQ ID NOs: 1 and 3), and the amino acid sequences were determined (SEQ ID NOs: 2 and 4).

Namely, SEQ ID NO: 1 depicts the base sequence of gene encoding the heavy chain variable region of the antibody produced by mouse hybridoma cell line E2-73, and SEQ ID NO: 2 depicts the amino acid sequence corresponding thereto. SEQ ID NO: 3 depicts the base sequence of gene encoding the light chain variable region of the antibody produced by mouse hybridoma cell line E2-73, and SEQ ID NO: 4 depicts the amino acid sequence corresponding thereto.(5) Expression of anti-estradiol monoclonal antibody variable region A recombinant phage production system (purchased from Amersham Pharmacia Biotech) was used to express the cDNA of the anti-estradiol monoclonal antibody variable region. To utilize this system, the scFv, which was excised from the above-mentioned plasmid with restriction enzymes *Sfi*I and *Not*I and pCANTAB 5E phagemid (purchased from Amersham Pharmacia Biotech) were ligated using Quick Ligation Kit (purchased from NEB) to yield a plasmid pCANTAB-5E/E2-73scFvHL. TG1 competent cells (*Escherichia coli* TG-1) (purchased from Toyobo) were transformed with the obtained plasmid by electroporation to produce a transformant *Escherichia coli* TG/pCAN-E2scFvHL harboring the plasmid pCANTAB-5E/E2-73scFvHL. The *Escherichia coli* transformant has been deposited at National Institute of Advanced Industrial Science and Technology, International Patent Organism Depository (Central No. 6, 1-1, Higashi 1-chome, Tsukuba-shi, Ibaragi-ken, Japan) under the Budapest treaty. The acceptance number and acceptance date thereof are shown below.

Escherichia coli TG/pCAN-E2scFvHL

Accession number: FERM BP-7912

Acceptance date: February 22, 2002

Furthermore, an antibody expressing phage was obtained using the transformant obtained in this way and a helper phage M13KO7 (purchased from Amersham Pharmacia Biotech). Then, the reactivity of the scFv expressed on this phage against estradiol was confirmed by enzyme immunoassay (ELISA). In ELISA, at first, a hapten-protein complex (β-Estradiol 6-(o-carboxy-methyl)oxime:BSA; purchased from SIGMA)(100 μl) was added to a plate, and stood at 4°C overnight. Then, blocking with 3% skim milk was carried out at 37°C for 1.5 hr. The plate was washed with PBS twice. The expressed scFv was mixed with a free antigen (final concentration 1 mg/ml, 0.037 mg/ml or 0 mg/ml) and the mixture (100 μl) was added to the plate and stood at room temperature for 3 hr. Then, the plate was washed with

0.5% Tween/PBS (twice) and PBS (once), 500-fold diluted HRP/Anti-M13: secondary antibody (purchased from Amersham Pharmacia Biotech) (100 µl) was added, and the mixture was stood at room temperature for 2 hr. Then, the plate was washed with 0.5% Tween/PBS (twice) and PBS (once), a substrate solution (0.2% o-phenylenediamine solution, 0.003% $H_2O_2$) was added and the mixture was reacted at room temperature for 20 min. A reaction termination solution (4N $H_2SO_4$) was added and the absorbance (492 nm) was measured (Fig. 12). The obtained results confirmed that this scFv has a binding ability to a female sex hormone.

Reference Example 4

(1) Determination of N terminal amino acid sequence of anti-estrone monoclonal antibody

The antibody protein purified from the culture supernatant of anti-estrone monoclonal antibody-producing mouse hybridoma cells (E1-420 line) (FERM BP-7568) using Protein A column (PROSEP-A, purchased from Air Brown Co.) was separated and purified to heavy chain and light chain by SDS-polyacrylamide gel electrophoresis, and transferred onto PVDF membrane. After staining with Ponso-S, protein bands were excised. The excised proteins on the membrane were treated with Pfu Pyroglutamate Aminopeptidase (purchased from Takara Shuzo Co.) (2 mU) at 50°C for 17 hr, and N terminal amino acid sequences were determined with gas phase protein sequencer (Model 494 Applied Biosystems Japan). The following results were obtained.

E1-420 heavy chain: IQLVESGPELKKPGETVKIS

E1-420 light chain: DVLMTQTPLTLSVTIGQPA

(2) Purification of anti-estrone monoclonal antibody mRNA

Anti-estrone monoclonal antibody producing mouse hybridoma cell (E1-420 line) (FERM BP-7568) ($1X10^7$ cells/ml) were suspended in RNA preparation solution (ISOGEN solution, purchased from Wako Pure Chemical Industries, Ltd.) (1 ml) and disrupted, and RNA was extracted from the cells according to the manual. The concentration of RNA was determined from absorbance at 260nm. From the obtained RNA (about 100 µg), mRNA (about 1.5 µg) was obtained using mRNA purified kit (Oligotex-dT30 <super> mRNA Purification kit, purchased from Takara Shuzo Co.).

(3) Acquisition of cDNA fragments of anti-estrone monoclonal antibody heavy chain and light chain variable regions and determination of their base sequences as well as deduction of amino acid sequences of heavy chain and light chain variable regions

The synthesis of cDNAs was carried out using about 100 ng of mRNA each for light chain and heavy chain, with First Strand cDNA Synthesis Kit ReverTra Ace -α™ (purchased from Toyobo Co.). Then, amplification of each cDNA fragment was carried out by PCR reaction in Personal cycler (purchased from Biometra) (94°C 30 sec, 55°C 30 sec, 72°C 2 min; Repeat number: 30 cycles), using primers for heavy chain and primers for light chain shown in the following, which were devised from their N terminal amino acid sequences obtained in (1) above, and Pfu Turbo (purchased from Toyobo Co.) enzyme.
Primers for E1-420 heavy chain

Forward 5'- CTATGCGGCCCAGCCGGCCATGATCCAGTTGGTGCAGTCTGGA-3' (SEQ ID NO: 29)

Back 5'- CGAGGAGACTGTGAGAGTGGT-3' (SEQ ID NO: 30)

Primers for E1-420 light chain

Forward 5'- CTCAGTCTCCAGATGTTTTGATGACCCAAACT-3' (SEQ ID NO: 31)

Back 5'- CCGTTTCAGCTCCAGCTTGGTCCC-3' (SEQ ID NO: 32)

Thus, cDNA fragments for variable regions of heavy chain and light chain of anti-estradiol monoclonal antibody were obtained. Each fragment was purified from 2% agarose after electrophoresis using a micro spin column (purchased from Amersham Pharmacia Biotech Co.).

Then, using dideoxy method, the base sequence of each variable region cDNA was determined, and the amino acid sequence was deduced therefrom. The base sequences (SEQ ID NOs: 5 and 7) were determined by DNA sequencer 310 (Applied Biosystems Japan) and amino acid sequences (SEQ ID NOs: 6 and 8) were determined therefrom, after the reaction in a Personal cycler (purchased from Biometra) with Big Dye Terminator Cycle Sequencing Ready Reaction Kit (purchased from Applied Biosystems Japan) using the above-mentioned PCR primers as sequencing primers.

Namely, SEQ ID NO: 5 depicts the base sequence of cDNA containing the heavy chain variable region of the antibody produced by mouse hybridoma cell line E1-420, SEQ ID NO: 6 depicts the amino acid sequence corresponding thereto.

SEQ ID NO: 7 depicts the base sequence of cDNA containing the light chain variable region of the antibody produced by mouse hybridoma cell line E1-420, SEQ ID NO: 8 depicts the amino acid sequence corresponding thereto.

Reference Example 5

(1) Determination of N terminal amino acid sequence of anti-ethynyl estradiol monoclonal antibody

The antibody protein purified from the culture supernatant of anti- ethynyl estradiol monoclonal antibody-producing mouse hybridoma cells (EE2-3 line or EE2-8-18 line) obtained in Reference Example 2 using Protein A column (PROSEP-A, purchased from Air Brown Co.) was separated and purified to heavy chain and light chain by SDS-polyacrylamide gel electrophoresis, and transferred onto PVDF membrane. After staining with Ponso-S, protein bands were excised. The excised proteins on the membrane were treated with Pfu Pyroglutamate Aminopeptidase (purchased from Takara Shuzo Co.) (2 mU) at 50°C for 17 hr, and N terminal amino acid sequences were determined with gas phase protein sequencer (Model 494 Applied Biosystems Japan). The following results were obtained.

EE2-3 heavy chain: VTLKESGPGILQPSQTLSLT

EE2-3 light chain: DIVMSQSPSSLAVSVGEKVT

EE2-8-18 heavy chain: VQVKESGPGLVAPSQSLL

EE2-8-18 light chain: DIQMTQSPASL?A?VG?TVT

(2) Purification of anti-ethynyl estradiol monoclonal antibody mRNA

Anti-ethynyl estradiol monoclonal antibody producing mouse hybridoma cells (EE2-3 line or EE2-8-18 line) ($1X10^7$ cells/ml) obtained in Reference Example 2 were suspended in RNA preparation solution (ISOGEN solution, purchased from Wako Pure Chemical Industries, Ltd.) (1 ml) and disrupted, and RNA was extracted from the cells according to the manual. The concentration of RNA was determined from absorbance at 260 nm. From the obtained RNA (about 100 µg), mRNA (about 1.5 µg) was obtained using mRNA purification kit (Oligotex-dT30 <super> mRNA Purification kit, purchased from Takara Shuzo Co.).

(3) Acquisition of cDNA fragments of anti-ethynyl estradiol monoclonal antibody heavy chain and light chain variable regions and determination of their base sequences as well as deduction of amino acid sequences of heavy chain and light chain variable regions

The synthesis of cDNAs was carried out using about 100 ng of mRNA each for light chain and heavy chain, with First Strand cDNA Synthesis Kit ReverTra Ace -α™ (purchased from Toyobo Co.). Then, amplification of each cDNA fragment was carried out by PCR reaction in Personal cycler (purchased from Biometra) (94°C 30 sec, 55°C 30 sec, 72°C 2 min; Repeat number: 30 cycles), using primers for heavy chain and primers for light chain shown in the following, which were devised from their N terminal amino acid sequences obtained in Example 1, and Pfu Turbo (purchased from Toyobo Co.) enzyme.
Primers for EE2-3 heavy chain <S:C,G R:A,G>

Forward 5'- CTATGCGGCCCAGCCGGCCCAGGTTACTCTRAAASARTC -3' (SEQ ID NO: 33)

Back 5'- CGCAGAGACAGTGACCAGAGT-3' (SEQ ID NO: 34)

Primers for EE2-3 light chain

Forward 5'- CTCAGTCTCCAGACATTGTGATGTCACAGTC-3' (SEQ ID NO: 35)

Back 5'- GCGGCCGCCTCATTCCTGTTGAAGCTCTTGAC-3' (SEQ ID NO: 36)

Primers for EE2-8 - 18 heavy chain

Forward 5'- CTATGCGGCCCAGCCGGCCATGGTGCAGGTGAAGGAGTCAGGA-3' (SEQ ID NO: 37)

Back 5'- CGAGGAGACGGTGACTGAGGT-3' (SEQ ID NO: 38)

Primers for EE2-8 - 18 light chain <Y:C,T D:A,G,T>

Forward 5'- CTCAGTCTCCAGAYATTCAGATGAYDCAGTC-3' (SEQ ID NO: 39)

Back 5'- CCGTTTGATTTCCAGCTTGGTGCC-3' (SEQ ID NO: 40)

Thus, cDNA fragments for variable regions of heavy chain and light chain of anti-ethynyl estradiol monoclonal antibody were obtained. Each fragment was purified from 2% agarose after electrophoresis using a micro spin column (purchased from Amersham Pharmacia Biotech Co.).

Then, using dideoxy method, the base sequence of each variable region cDNA was determined, and the amino acid sequence was deduced therefrom. The base sequences (SEQ ID NOs: 9,11,13 and 15)were determined by DNA sequencer 310 (Applied Biosystems Japan) and amino acid sequences (SEQ ID NOs: 10,12,14 and 16) were determined therefrom, after the reaction in a Personal cycler (purchased from Biometra) with Big Dye Terminator Cycle Sequencing Ready Reaction Kit (purchased from Applied Biosystems Japan) using the above-mentioned PCR primers

as sequencing primers.

Namely, SEQ ID NO: 9 depicts the base sequence of cDNA containing the heavy chain variable region of the antibody produced by mouse hybridoma cell line EE2-3, SEQ ID NO: 10 depicts the amino acid sequence corresponding thereto.
SEQ ID NO: 11 depicts the base sequence of cDNA containing the light chain variable region of the antibody produced by mouse hybridoma cell line EE2-3, SEQ ID NO: 12 depicts the amino acid sequence corresponding thereto.
SEQ ID NO: 13 depicts the base sequence of cDNA containing the heavy chain variable region of the antibody produced by mouse hybridoma cell line EE2-8-18, SEQ ID NO: 14 depicts the amino acid sequence corresponding thereto.
SEQ ID NO: 15 depicts the base sequence of cDNA containing the light chain variable region of the antibody produced by mouse hybridoma cell line EE2-8-18, SEQ ID NO: 16 depicts the amino acid sequence corresponding thereto.

Reference Example 6

(1) Cloning of cDNA encoding heavy chain and light chain of an anti-alkylphenol monoclonal antibody and an anti-alkylphenolethoxyate monoclonal antibody using 5'-Rapid Amplification of cDNA Ends (RACE) method

Mouse hybridoma cell AP-14 (FERM BP-6633) producing an anti-alkylphenol monoclonal antibody, and mouse hybridoma cell MOF3-139 (FERM BP-6059) producing an anti-alkylphenol ethoxylate monoclonal antibody (they were produced by the method described in Examples of PCT international publication WO99/43799) were used as starting materials. Firstly, about 2 μg of mRNAs were respectively extracted from $2x10^7$ of each hybridoma cells cultured in DMEM medium (purchased from ICN) containing 10% fetal calf serum (purchased form JRH) using Quick-Prep Micro mRNA Purification Kit (purchased from Amersham Pharmacia Biotech) according to attached instructions. Using these mRNAs as templates and SMART-RACE cDNA Amplification Kit (purchased from Clontech) according to attached instructions, reverse transcription reactions were carried out to synthesize single strand cDNAs respectively. Since it was identified that subclass of AP-14 was IgG2b and ? chain and subclass of MOF3-139 was IgG1 and ?? chain, Cgamma1 primer specific for constant region of heavy chain IgG1, Cgamma2b primer specific for constant region of heavy chain IgG2b, and Ckappa primer specific for constant region of light chain were synthesized. cDNAs encoding the light chain and the heavy chain of AP-14 and MOF3-139 were respectively isolated by 5'-RACE method using these three kinds of primers and SMART-RACE cDNA Amplification Kit.

Cgamma1 primer (SEQ ID NO:41):

5'-GACAGATGGGGGTGTCGTTTTGGC-3'

Cgamma2b primer (SEQ ID NO:42):

5'-GGAGGAACCAGTTGTATCTCCACACC-3'

Ckappa primer (SEQ ID NO:43):

5'-AGATGGATACAGTTGGTGCAGCATCAGC-3'

Composition of PCR reaction mixture for amplifying RACE product was 34.5 μL of sterilized water, 5 μL of 10 X Advantage 2 PCR Buffer (attached with SMART-RACE cDNA Amplification Kit), 1 μL of 10 mM dNTP Mix (purchased from Clontech), 1 μL of 50 X Advantage 2 Polymerase Mix (attached with SMART-RACE cDNA Amplification Kit), 2.5 μL of cDNA samples and 5 μL of Universal Primer Mix (attached with SMART-RACE cDNA Amplification Kit), as well as 1 μL of 10 μM Cgamma1 primers, 10 μM Cgamma2b primers or 10 μM Ckappa primers in 50 μL of total volume. In PCR, reaction of 30 seconds at 94°C, 30 seconds at 68°C and 3 minutes at 72°C was repeated 30 times. The reaction solutions were electrophoresed on 1.5% agarose gel. As a result, about 600 bp of cDNA fragment deduced to comprise a portion encoding the variable region of the light chain, and about 600 bp of cDNA fragment deduced to comprise a portion encoding the variable region of the heavy chain were amplified.

(2) Determination of base sequence of an anti-alkylphenolmonoclonal antibody and an anti-alkylphenol ethoxylate monoclonal antibody

cDNA fragments encoding the variable regions of the heavy chains and the light chains prepared in the above-mentioned (1) were inserted into cloning vector pT7Blue T-vector (purchased from Novagen), and introduced to Escherichia coli JM109. Recombinant pT7Blue T-vectors were extracted from grown Escherichia coli, base sequences of the variable regions of the heavy chains and the light chains of AP-14 and MOF3-139 were determined with dideoxy method. The base sequences are shown as SEQ ID NO:17, SEQ ID NO:19, SEQ ID NO:21 and SEQ ID NO:23. Amino acid sequences corresponding to them are shown as SEQ ID NO:18, SEQ ID NO:20, SEQ ID NO:22 and SEQ ID NO:24.

Specifically, SEQ ID NO:17 shows base sequence of heavy chain variable region gene of antibody produced by mouse hybridoma cell line AP-14, and SEQ ID NO:18 shows amino acid sequence corresponding thereto.
SEQ ID NO:19 shows base sequence of light chain variable region gene of antibody produced by mouse hybridoma cell line AP-14, and SEQ ID NO:20 shows amino acid sequence corresponding thereto.

SEQ ID NO:21 shows base sequence of heavy chain variable region gene of antibody produced by mouse hybridoma cell line MOF3-139, and SEQ ID NO:22 shows amino acid sequence corresponding thereto.

SEQ ID NO:23 shows base sequence of light chain variable region gene of antibody produced by mouse hybridoma cell line MOF3-139, and SEQ ID NO:24 shows amino acid sequence corresponding thereto.

(3) Synthesis of primers

Using base sequence information of the variable regions of the heavy chains and the light chains of AP-14 and MOF3-139 determined in the above-mentioned (2), 12 kinds of primers indicated below were synthesized for constructing AP-14scFv and MOF3-139scFv genes and chimera gene.

primer 01 (SEQ ID NO:44, sequence length: 23)
5'-CCATGGATCAGGTCCAGCTCCAGCAGTCT-3'

primer 02 (SEQ ID NO:45, sequence length: 52)
5'-AGAGCCACCTCCGCCTGAACCGCCTCCACCTGAGGAGACGGTGACTGAGGTT-3'

primer 03 (SEQ ID NO:46, sequence length: 52)
5'-GGCGGAGGTGGCTCTGGCGGTGGCGGATCGGATATTGTGATGACGCAGGCTG-3'

primer 04 (SEQ ID NO:47, sequence length: 29)
5'-GCTAGCCCGTTTTATTTCCAGCTTGGTC-3'

primer 05 (SEQ ID NO:48, sequence length: 28)
5'-CCATGGATCAGGTTCAGCTGCAGCAGTCT-3'

primer 06 (SEQ ID NO:49, sequence length: 53)
5'-AGAGCCACCTCCGCCTGAACCGCCTCCACCTGAGGAGACTGTGAGAGTGGTGC-3'

primer 07 (SEQ ID NO:50, sequence length: 30)
5'-CCATGGATGATATTGTGATGACGCAGGCTG-3'

primer 08 (SEQ ID NO:51, sequence length: 52)
5'-AGAGCCACCTCCGCCTGAACCGCCTCCACCCCGTTTTATTTCCAGCTTGGTC-3'

primer 09 (SEQ ID NO:52, sequence length: 51)
5'-GGCGGAGGTGGCTCTGGCGGTGGCGGATCGCAGGTCCAGCTCCAGCAGTCT-3'

primer 10 (SEQ ID NO:53, sequence length: 28)
5'-GCTAGCTGAGGAGACGGTGACTGAGGTT-3'

primer 11 (SEQ ID NO:54, sequence length: 51)
5'-GGCGGAGGTGGCTCTGGCGGTGGCGGATCGCAGGTCCAGCTCCAGCAGTCT-3'

primer 12 (SEQ ID NO:55, sequence length: 29)
5'-GCTAGCTGAGGAGACTGTGAGAGTGGTGC-3'

(4) Construction of single chain variable fragment antibody (scFv) gene of an anti-alkylphenol monoclonal antibody and an anti-alkylphenolethoxylate monoclonal antibody

1) Construction of AP-14scFvHL gene

AP-14scFvHL gene was constructed using primer 01 and primer 02 for amplifying AP-14 VH gene, primer 03 and primer 04 for amplifying AP-14 VL gene and primer 01 and primer 04 for constructing its scFv gene in accordance with following procedures.

2) Construction of MOF3-139scFvHL gene

MOF3-139scFvHL gene was constructed using primer 05 and primer 06 for amplifying MOF3-139 VH gene, primer 03 and primer 04 for amplifying MOF3-139 VL gene and primer 05 and primer 04 for constructing its scFv gene in accordance with following procedures.

3) Construction of AP-14scFvLH gene

AP-14scFvLH gene was constructed using primer 07 and primer 08 for amplifying AP-14 VL gene, primer 09 and primer 10 for amplifying AP-14 VH gene and primer 07 and primer 10 for constructing its scFv gene in accordance with following procedures.

4) Construction of MOF3-139scFvLH gene

MOF3-139scFvLH gene was constructed using primer 07 and primer 08 for amplifying MOF3-139VL gene, using primer 11 and primer 12 for amplifying MOF3-139 VH gene and primer 07 and primer 12 for constructing its scFv gene in accordance with following procedures.

5) Construction of AP-14VH/MOF3-139VL gene

AP-14VH/MOF3-139VL gene was constructed using primer 01 and primer 02 for amplifying AP-14 VH gene, primer 03 and primer 04 for amplifying MOF3-139 VL gene and primer 01 and primer 04 for constructing its scFV gene in accordance with following procedures.

6) Construction of MOF3-139VH/AP-14VL gene

AP-14VH/MOF3-139VL gene was constructed using primer 05 and primer 06 for amplifying MOF3-139 VH gene, primer 03 and primer 04 for amplifying AP-14 VL gene and primer 05 and primer 04 for constructing its scFv

gene in accordance with following procedures.

7) Construction of AP-14VL/MOF3-139VH gene

AP-14VL/MOF3-139VH gene was constructed using primer 07 and primer 08 for amplifying AP-14 VL gene, primer 11 and primer 12 for amplifying MOF3-139 VH gene and primer 07 and primer 12 for constructing its scfv gene in accordance with following procedures.

8) Construction of the MOF3-139VL/AP-14VH gene

MOF3-139VL/AP-14VH gene was constructed using primer 07 and primer 08 for amplifying MOF3-139 VL gene, primer 09 and primer 10 for amplifying AP-14 VH gene, and primer 07 and primer 10 for constructing its scFv gene in accordance with following procedures.

The genes were constructed by PCR. Firstly, PCR reactions were carried out using plasmid inserted with cDNA encoding the variable regions of the heavy chain or the light chain of AP-14 and MOF3-139, and primers corresponding to each plasmid in order to amplify cDNA fragments encoding the variable regions of the heavy chain and the light chain. For example, composition of PCR reaction mixture was 1 μL of 50 X Advantage cDNA Polymerase Mix (purchased from Clontech), 5 μL of 10 X cDNA PCR Reaction Buffer (purchased from Clontech), 1 μL of 50 X dNTP Mix (purchased from Clontech), 0.5 μL of recombinant plasmid (in this case, recombinant pT7Blue T-vector inserted with cDNA fragment encoding the variable region of the heavy chain of AP-14) (100 ng), 0.5 μL of primer 01 (50 pmol), 0.5 μL of primer 02 (50 pmol) and 41.5 μL of sterilized water in 50 μL of the total volume, when the heavy chain of AP-14 was amplified. Reaction was repeated 30 times under PCR condition of 30 seconds at 94°C, 30 seconds at 63°C and 1 minute at 72°C. cDNAs encoding the heavy chain and the light chain of AP-14 and MOF3-139 were amplified respectively, then reaction solution were electrophoresed on 1.5% agarose gel. As a result, amplification of about 400 bp of cDNA fragment encoding the variable region of the light chain, and about 400 bp of cDNA fragment encoding the variable region of the heavy chain was confirmed respectively. cDNA fragments were purified respectively, and then used for constructing scFv gene in which the variable regions of the heavy chain and the light chain is linked. PCR reaction was carried out using cDNA fragments encoding the variable regions of the heavy chain and the light chain. For example, composition of PCR reaction mixture was 21.5 μL of lysis buffer for cDNA encoding the variable region of the heavy chain of AP-14, 21.5 μL of lysis buffer for cDNA encoding the variable region of the light chain of AP-14, 5 μL of 10 X cDNA PCR Reaction Buffer, 1 μL of 50 X d NTP Mix, 1 μL of 50 X Advantage cDNA Polymerase Mix in 50 μL of total volume, when the heavy chain of AP-14 was amplified. Reaction was repeated 7 times under PCR condition of 30 seconds at 94°C, 30 seconds at 55°C and 1 minute at 72°C. Thereafter, 1 μL of 50 X Advantage cDNA Polymerase Mix, 5 μL of 10 X cDNA PCR Reaction Buffer, 1 μL of 50 X dNTP Mix, 0.5 μL of primer 01 (50 pmol), 0.5 μL of primer 06 (50 pmol) and 42 μL of sterilized water was used to 50 μL of total amount, and added to the aforementioned PCR reaction mixture. Reaction was repeated 30 times under PCR condition of 30 seconds at 94°C, 30 seconds at 63°C and 1 minute at 72°C. The reaction solution was electrophoresed on 1.5% agarose gel, amplification of about 750 bp of AP-14scFv and MOF3-139scFv gene fragment was confirmed, and purified.

A summary of 8 kinds of anti-endocrine disruptor single chain variable region antibodies prepared by the above procedures is shown in Fig. 13.

(5) Construction of scFv expression plasmids of anti-alkylphenol monoclonal antibody and anti-alkylphenol ethoxylate monoclonal antibody

The eight kinds of genes prepared in the above-mentioned (4) were each inserted in cloning vector pT7 Blue T-vector to transform Escherichia coli JM109 strain. The introduced recombinant plasmid was extracted from the grown Escherichia coli, and the base sequence of the gene was confirmed. The recombinant plasmid in which the gene had been inserted and the Escherichia coli expression vector pET-27b were digested using restriction enzymes *Nco*I and *Nhe*I respectively, and the gene and the Escherichia coli expression vector pET-27b was ligated to construct the following expression plasmids

pET-27b/AP-14scFvHL,
pET-27b/AP-14scFvLH,
pET-27b/MOF3-139scFvHL,
pET-27b/MOF3-139scFvLH,
pET-27b/AP-14VH/MOF3-139VLscFv,
pET-27b/MOF3-139VL/AP-14VHscFv,
pET-27b/MOF3-139VH/AP-14VLscFv and

pET-27b/AP-14VL/MOF3-139VHscFv.

(6) Preparation of scFvs of anti-alkylphenol monoclonal antibody and anti-alkylphenol ethoxylate monoclonal antibody

The eight kinds of expression plasmids prepared in the above-mentioned (5) were each introduced in Escherichia coli BL21DE3 (pLysS) strain to produce eight strains of transformant in which an expression plasmid had been introduced.

A strain in which AP-14scFvHL expression plasmid pET-27b/AP-14 scFvHL has been introduced in Escherichia coli BL21DE3 (pLysS) strain is referred to as Escherichia coli BL/pET-APscFvHL, and a strain in which MOF3-139scFvHL expression plasmid pET-27b/MOF3-139scFvHL has been introduced in Escherichia coli BL21DE3 (pLysS) strain is referred to as Escherichia coli BL/pET-MOFscFvHL. Both have been deposited at the National Institute of Advanced Industrial Science and Technology, International Patent Organism Depositary (Central No. 6, 1-1, Higashi 1-chome, Tsukuba-shi, Ibaragi-ken, Japan) under the Budapest treaty. The accession numbers and acceptance dates thereof are shown below.

(a) Escherichia coli BL/pET-APscFvHL

Accession number: FERM BP-7910
Acceptance date: February 22, 2002

(b) Escherichia coli BL/pET-MOFscFvHL

Accession number: FERM BP-7911
Acceptance date: February 22, 2002

The eight kinds of transformed colonies obtained as above were each seeded on 2 X YT medium (5 mL, comprising 1.7% (w/v) of Bacto-trypton (purchased from Nacalai Tesque, Inc.), 1% (w/v) of Bacto-yeast extract (purchased from Nacalai Tesque, Inc.) and 0.5% of (w/v) NaCl (purchased from Nacalai Tesque, Inc.)) comprising 25 μg/mL of kanamycin (purchased from Nacalai Tesque, Inc.) and 33 μg/mL of chloramphenicol (purchased from Nacalai Tesque, Inc.) and cultured at 23°C overnight with shaking. The suspension was suspended in SB medium (800 mL, 3.5% (w/v) of Bacto-trypton, 2% (w/v) of Bacto-yeast extract and 0.5% (w/v) of NaCl) and cultured at 23°C overnight with shaking. To the suspension was added isopropylthiogalactoside (IPTG) (purchased from Nacalai Tesque, Inc.) so that the final concentration became 1 mM and further cultured at 23°C for 6 hr with shaking. The suspension was separated by centrifugation at 1500 g for 20 min and the supernatant was removed. The precipitated fungus body was suspended in ice-cooled TES buffer (32 mL, comprising 0.2 M Tris-HCl (pH 8.0), 0.5 mM EDTA and 0.5 M sucrose (each was purchased from Nacalai Tesque, Inc.)). To the suspension was further added ice-cooled 0.2X TES buffer (48 mL, comprising 0.04 M Tris-HCl (pH 8.0), 0.1 mM EDTA and 0.1 M sucrose) and stirred sufficiently. The suspension was cooled with ice for 30 min and separated by centrifugation at 12000 g for 10 min, and the obtained supernatant was used for AP-14scFv and MOF3-139scFv. Expression of the desired protein was confirmed by Western Blot method using an anti-HSV tagged antibody (mentioned below). The results are shown in Fig. 14. It was confirmed that eight kinds of scFv antibodies (about 30 kDa) were each expressed in *Escherichia coli.*
Western Blot method:

About 10 μg of scFv was subjected to 10% dodecyl sodium sulfate-polyacrylamide electrophoresis (SDS-PAGE) and the scFv in the gel was transferred to a polyvinylidene difluoride (PVDF) membrane. Blocking was carried out using Block Ace, and the PVDF membrane was soaked in a solution of anti-HSV antibody (0.1 μg/mL) and shaken at room temperature for 1 hr. The PVDF membrane was washed, soaked in an antibody solution comprising alkaline phosphatase-labeled anti-mouse antibody and shaken at room temperature for 30 min. The PVDF membrane was washed, and coloring reaction was carried out by soaking the membrane in a solution of a chromogen substrate (nitroblue tetrazolium/5-bromo-4-chloro-3-indolylphosphoric acid).

(7) Comparison of performance of antibody by indirect competitive ELISA

100 μL of a conjugate (5 μg/mL) of hapten and bovine serum albumin (BSA) was added to each well in a 96-well microtiter plate and stood at 4°C overnight. After removing the liquid from the well, 300 μL of Block Ace (purchased from Dainippon Pharmaceutical Co., Ltd.) 4-fold diluted with distillated water was added to each well and stood at 4°C overnight. After removing the liquid from the well, a standard solution (50 μL) of nonylphenol decaethoxylate stepwise diluted with phosphate buffer (10 mM sodium phosphate (purchased from Nacalai Tesque, Inc.), 150 mM NaCl, pH 7.2) was added to each well. AP-14scFv or MOF3-139scFv prepared in Example 6 was diluted with a phosphate buffer and the diluted solution (50 µL) was added to each well. The mixture was incubated at 25°C for 1 hr and each well was washed 4 times with a phosphate buffer. Then, 100 µL of mouse anti-HSV tagged antibody (purchased from Amersham Pharmacia Biotech), diluted with a phosphate buffer to 0.1 µg/mL, was added to each well and the mixture was incubated at 25°C for 1 hr. After washing each well 4 times with a phosphate buffer, 100 µL of a peroxydase-labeled goat antibody against mouse antibody (purchased from Pierce), 2,000-fold diluted with a phosphate buffer, was added to each well and the mixture was incubated at 25°C for 1 hr. After washing each well 3 times with a phosphate buffer, 100 µL of chromogeic substrate solution (100 mg/L tetramethylbenzidine (purchased from Wako Pure Chemical Industries, Ltd.) and 0.006% (v/v) aqueous hydrogen peroxide solution were added into 0.1 M acetate buffer (pH 5.5)) was added to each well. The mixture was incubated at room temperature for 10 min. Then, 1 N sulfuric acid (50 µL/well) was added to stop the enzyme reaction. The absorbance at wavelength of 450 nm was measured.

As a control, indirect competitive ELISA was conducted by using a monoclonal antibody from hybridoma. As described above, 50 µL of a standard solution of nonylphenol decaethoxylate, serially diluted with a phosphate buffer, was added to each well in a 96-well microtiter plate having a conjugate of hapten and BSA fixed thereto. AP-14 or MOF3-139 was diluted with a phosphate buffer, and 50 µL of the diluted solution was added to each well. The mixture was incubated at 25°C for 1 hr and each well was washed 3 times with a phosphate buffer. Then, 100 µL of a peroxydase-labeled goat antibody against mouse antibody (purchased from Pierce) 2,000-fold diluted with a phosphate buffer was added to each well and the mixture was incubated at 25°C for 1 hr. After washing each well 3 times with a phosphate buffer, 100 µL of the above-mentioned chromogeic substrate solution was added to each well. The mixture was incubated at room temperature for 10 min. Then, 1N sulfuric acid (50 µL/well) was added to stop the enzyme reaction. The absorbance at wavelength of 450 nm was measured.

The results are shown in Fig. 15. As is clear from Fig. 15, the prepared anti-environmental single chain variable fragment antibody could be used as an antibody.

## Industrial Applicability

Because the isolation material of the present invention is capable of selectively and efficiently isolating an endocrine disruptor, by using this, endocrine disruptors, which occur only in very trace amounts in the environment, can be concentrated at high rates and efficiently in a condition with low abundance of immunological impurities.

Sequence Listing Free Text

SEQ ID NO: 25; oligonucleotide designed for acting as a PCR primer.
SEQ ID NO: 26; oligonucleotide designed for acting as a PCR primer.
SEQ ID NO: 27; oligonucleotide designed for acting as a PCR primer.
SEQ ID NO: 28; oligonucleotide designed for acting as a PCR primer.
SEQ ID NO: 29; oligonucleotide designed for acting as a PCR primer.
SEQ ID NO: 30; oligonucleotide designed for acting as a PCR primer.
SEQ ID NO: 31; oligonucleotide designed for acting as a PCR primer.
SEQ ID NO: 32; oligonucleotide designed for acting as a PCR primer.
SEQ ID NO: 33; oligonucleotide designed for acting as a PCR primer.
SEQ ID NO: 34; oligonucleotide designed for acting as a PCR primer.
SEQ ID NO: 35; oligonucleotide designed for acting as a PCR primer.
SEQ ID NO: 36; oligonucleotide designed for acting as a PCR primer.
SEQ ID NO: 37; oligonucleotide designed for acting as a PCR primer.
SEQ ID NO: 38; oligonucleotide designed for acting as a PCR primer.
SEQ ID NO: 39; oligonucleotide designed for acting as a PCR primer.
SEQ ID NO: 40; oligonucleotide designed for acting as a PCR primer.
SEQ ID NO: 41; oligonucleotide designed for acting as a PCR primer.
SEQ ID NO: 42; oligonucleotide designed for acting as a PCR primer.
SEQ ID NO: 43; oligonucleotide designed for acting as a PCR primer.
SEQ ID NO: 44; oligonucleotide designed for acting as a PCR primer.
SEQ ID NO: 45; oligonucleotide designed for acting as a PCR primer.
SEQ ID NO: 46; oligonucleotide designed for acting as a PCR primer.
SEQ ID NO: 47; oligonucleotide designed for acting as a PCR primer.
SEQ ID NO: 48; oligonucleotide designed for acting as a PCR primer.
SEQ ID NO: 49; oligonucleotide designed for acting as a PCR primer.

SEQ ID NO: 50; oligonucleotide designed for acting as a PCR primer.
SEQ ID NO: 51; oligonucleotide designed for acting as a PCR primer.
SEQ ID NO: 52; oligonucleotide designed for acting as a PCR primer.
SEQ ID NO: 53; oligonucleotide designed for acting as a PCR primer.
SEQ ID NO: 54; oligonucleotide designed for acting as a PCR primer.
SEQ ID NO: 55; oligonucleotide designed for acting as a PCR primer.

## Claims

1. An isolation material for an endocrine disruptor, which comprises a carrier made of a polymeric material being bound covalently with an anti-endocrine disruptor antibody via a graft chain of the carrier made of a polymeric material.

2. The isolation material of claim 1, wherein the carrier made of a polymeric material is a plain membrane, a hollow fiber membrane, a non-woven fabric, a woven fabric, a fiber or a molded bead.

3. The isolation material of claim 2, wherein the carrier made of a polymeric material is a porous hollow fiber membrane or a non-woven fabric.

4. The isolation material of claim 3, wherein the carrier made of a polymeric material is a porous hollow fiber membrane.

5. The isolation material of claim 4, wherein the porous hollow fiber membrane has a pore diameter of 0.05 to 100 µm and a void ratio of 20 to 80%.

6. The isolation material of claim 3, wherein the carrier made of a polymeric material is a non-woven fabric.

7. The isolation material of claim 6, wherein the non-woven fabric has a fabric weight of 10 to 200 $g/m^2$.

**8.** The isolation material of any of claims 1 to 7, wherein the graft ratio is 10 to 300%.

**9.** The isolation material of any of claims 1 to 8, wherein the anti-endocrine disruptor antibody is a polyclonal antibody or recombinant antibody.

**10.** The isolation material of any of claims 1 to 9, wherein the endocrine disruptor is an alkylphenol, an alkylphenol ethoxylate, a resin component, a resin plasticizer, a chlorophenol, a female sex hormone, a male sex hormone or a thyroid hormone.

**11.** An endocrine disruptor concentration method including:

(1) a step wherein an endocrine disruptor-containing sample is brought into contact with the isolation material of any of claims 1 to 10 to allow said endocrine disruptor to be captured by an anti-endocrine disruptor antibody, and
(2) a step wherein said endocrine disruptor captured by said anti-endocrine disruptor antibody is dissociated and recovered.

**12.** The method of claim 11, wherein the endocrine disruptor is an alkylphenol, an alkylphenol ethoxylate, a resin component, a resin plasticizer, a chlorophenol, a female sex hormone, a male sex hormone or a thyroid hormone.

**13.** An endocrine disruptor clean-up method wherein an endocrine disruptor-containing sample is brought into contact with the isolation material of any of claims 1 to 10 to allow said endocrine disruptor to be captured by an anti-endocrine disruptor antibody.

**14.** The method of claim 13, wherein the endocrine disruptor is an alkylphenol, an alkylphenol ethoxylate, a resin component, a resin plasticizer, a chlorophenol, a female sex hormone, a male sex hormone or a thyroid hormone.

FIG. 1

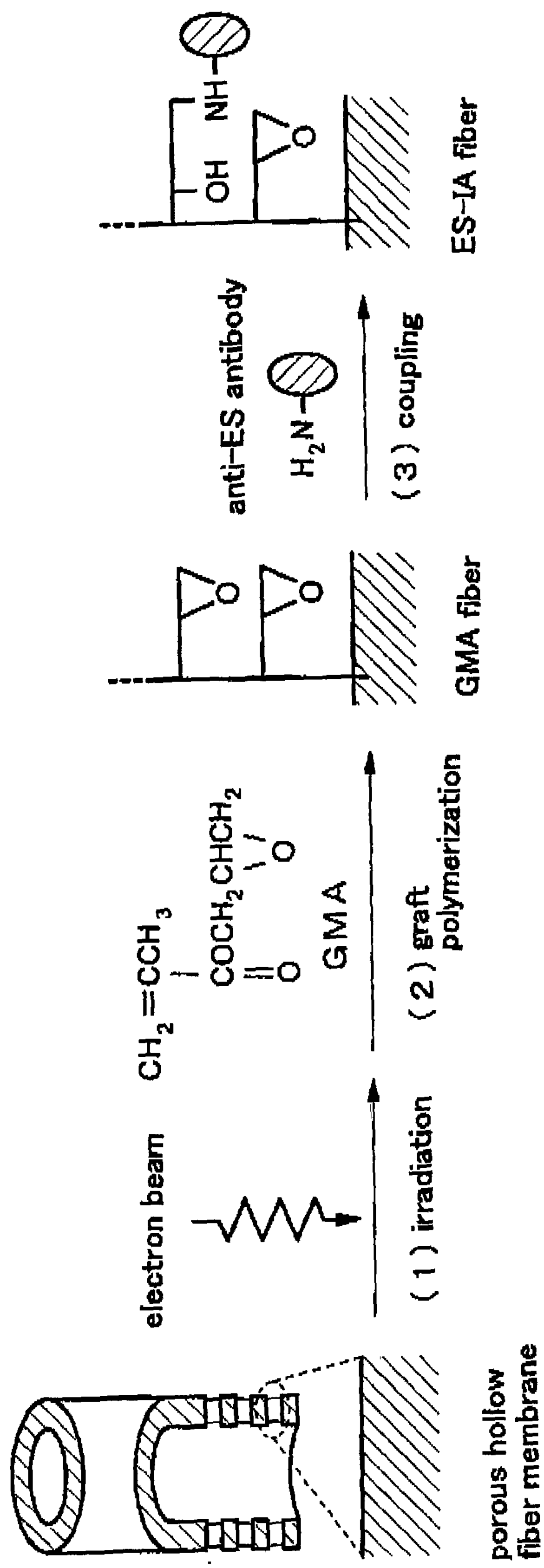
porous hollow fiber membrane
electron beam
(1) irradiation
GMA
(2) graft polymerization
GMA fiber
anti-ES antibody
(3) coupling
ES-IA fiber

FIG. 2

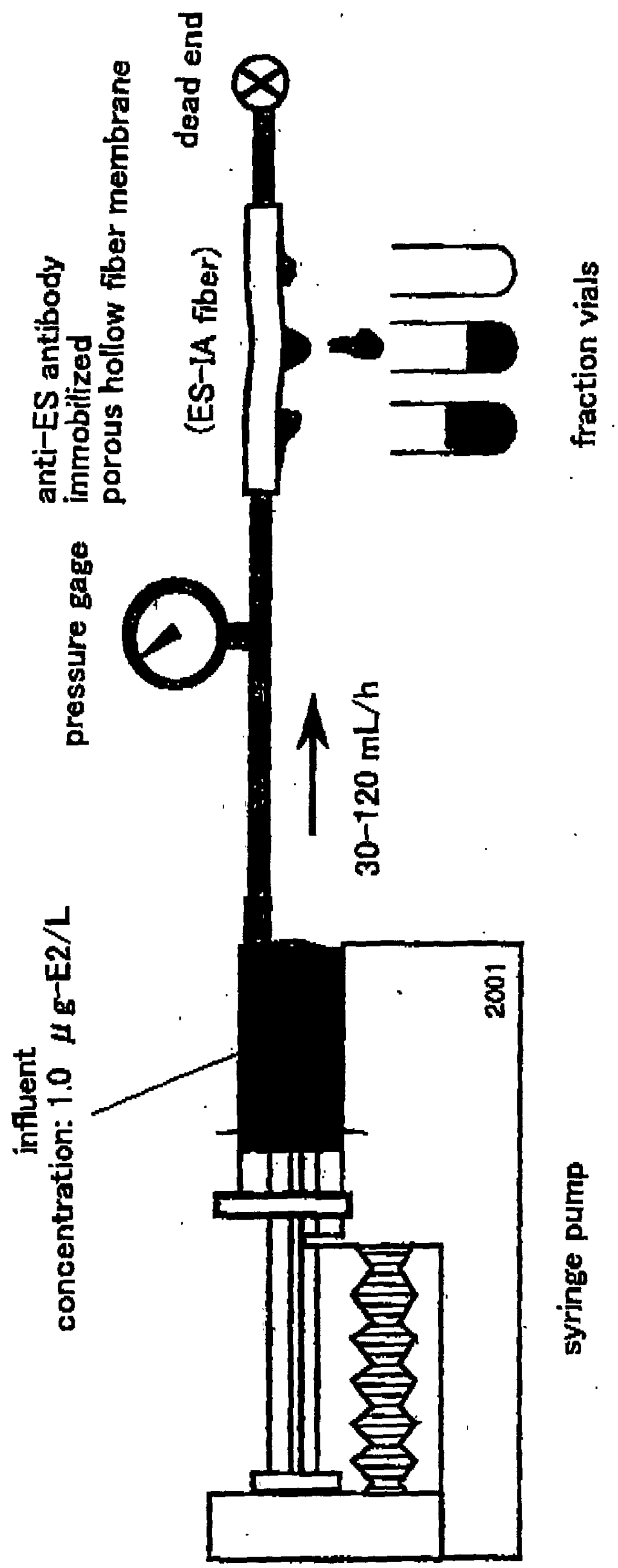
dead end
anti-ES antibody
immobilized
porous hollow fiber membrane
(ES-1A fiber)
fraction vials
pressure gage
30-120 mL/h
influent
concentration: 1.0 μg-E2/L
2001
syringe pump

FIG. 3

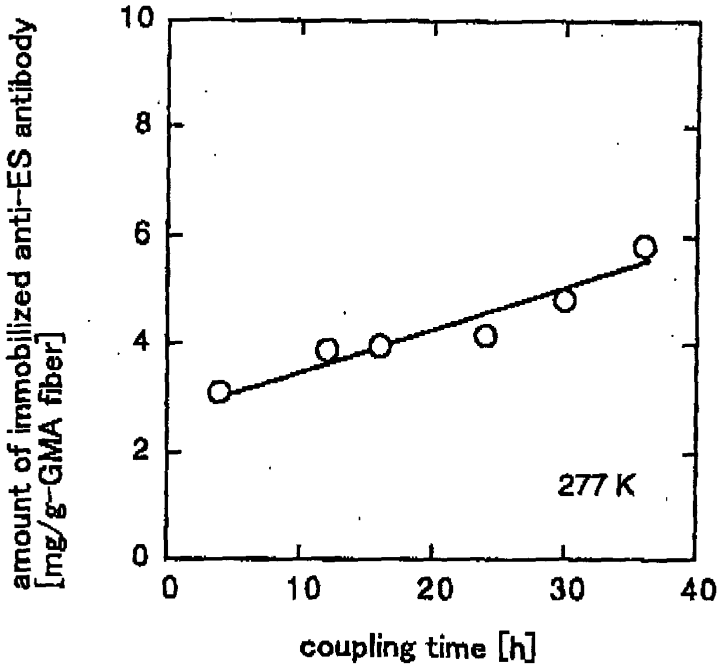
10
8
6
4
2
0
amount of immobilized anti-ES antibody [mg/g-GMA fiber]
277 K
0
10
20
30
40
coupling time [h]

FIG. 4

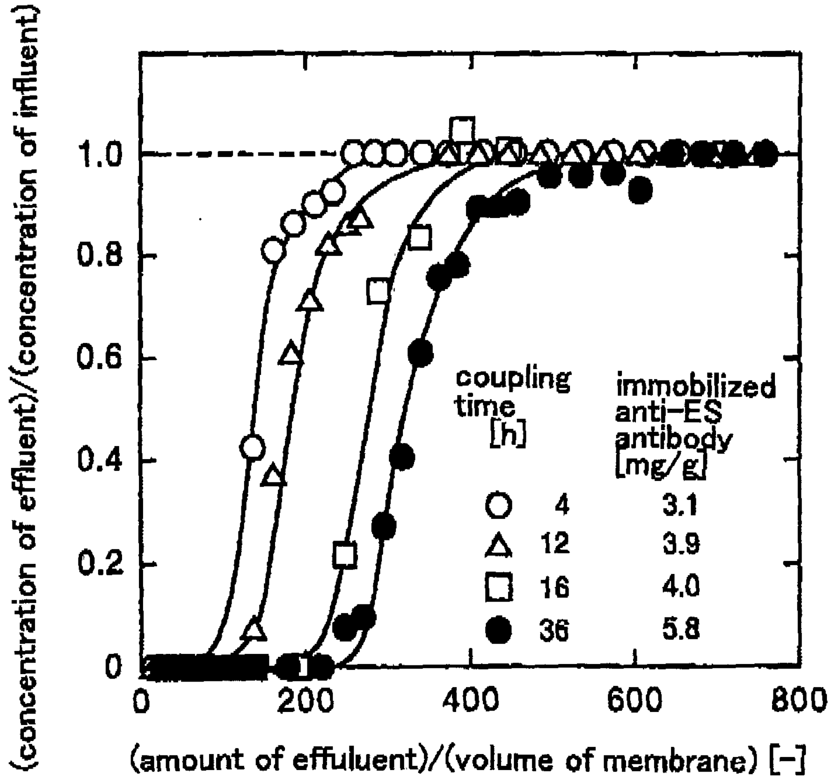
(concentration of effluent)/(concentration of influent) [–]
1.0
0.8
0.6
0.4
0.2
0
coupling time [h]
immobilized anti-ES antibody [mg/g]
4 3.1
12 3.9
16 4.0
36 5.8
0
200
400
600
800
(amount of effuluent)/(volume of membrane) [–]

FIG. 5

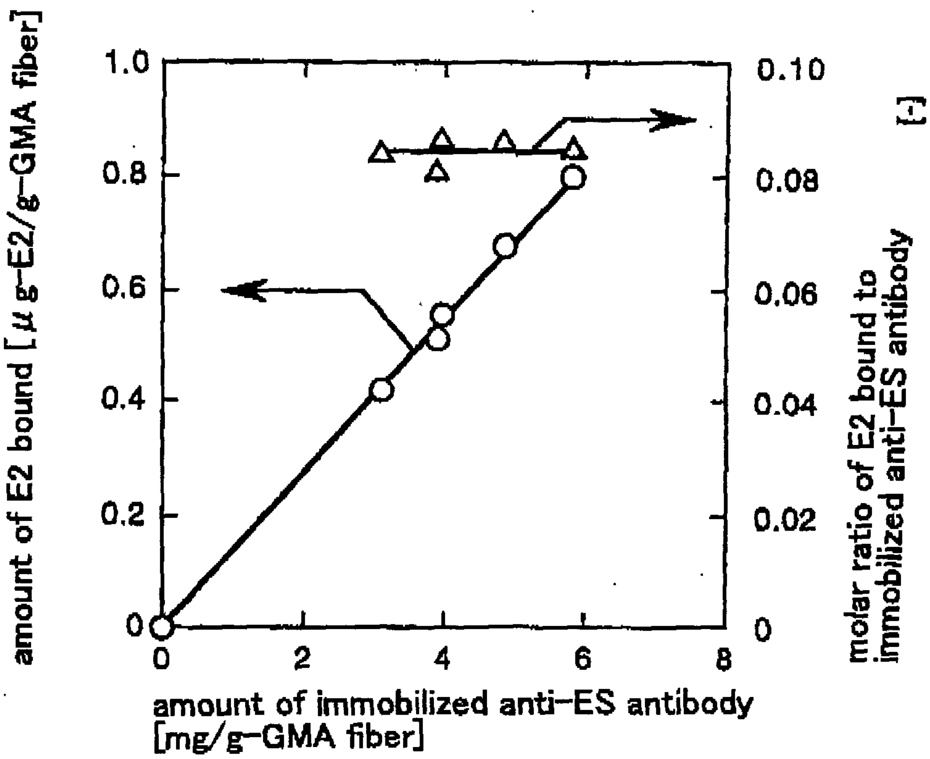
amount of E2 bound [μ g-E2/g-GMA fiber]
1.0
0.8
0.6
0.4
0.2
0
0
2
4
6
8
0.10
0.08
0.06
0.04
0.02
0
molar ratio of E2 bound to immobilized anti-ES antibody [-]
amount of immobilized anti-ES antibody [mg/g-GMA fiber]

FIG. 6

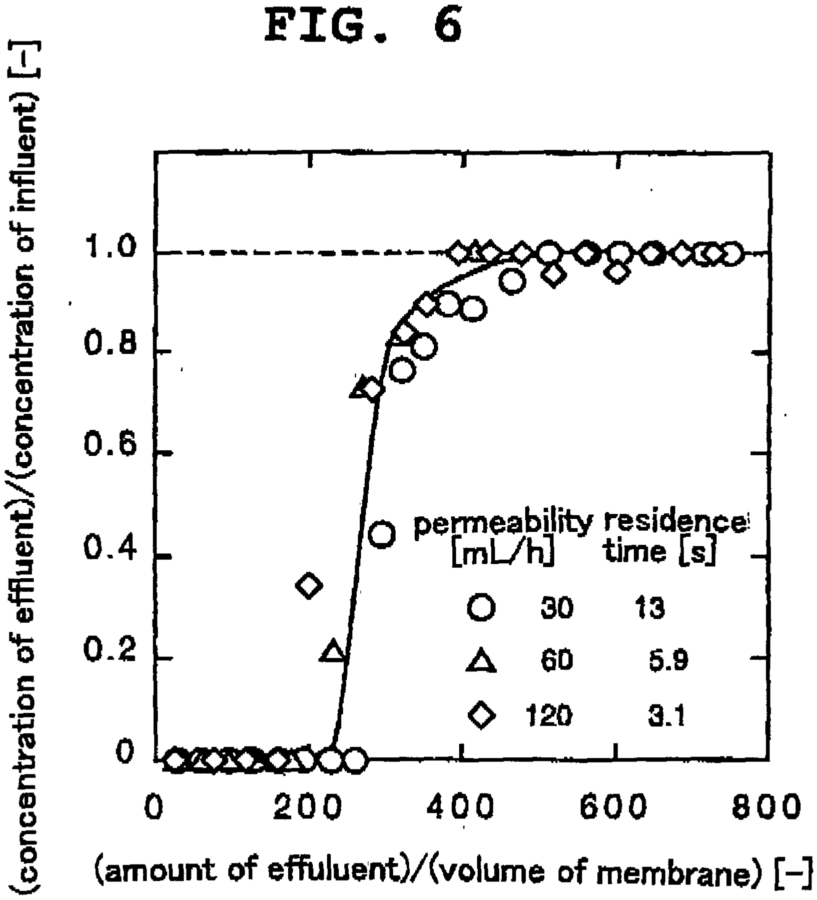
(concentration of effluent)/(concentration of influent) [-]
1.0
0.8
0.6
0.4
0.2
0
0
200
400
600
800
permeability [mL/h]
residence time [s]
30 13
60 5.9
120 3.1
(amount of effuluent)/(volume of membrane) [-]

FIG. 7

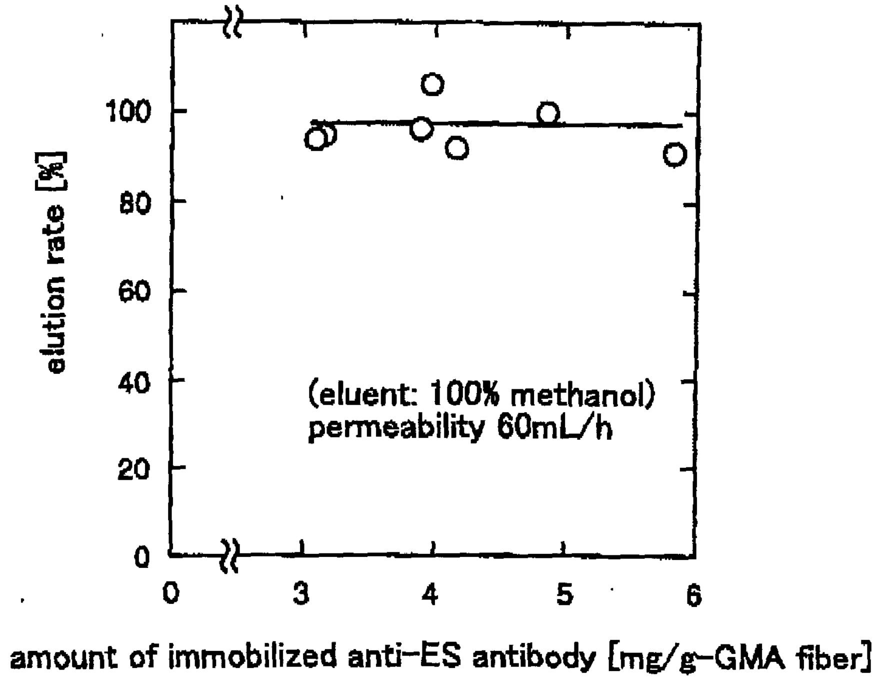
elution rate [%]
100
80
60
40
20
0
(eluent: 100% methanol)
permeability 60mL/h
0
3
4
5
6
amount of immobilized anti-ES antibody [mg/g-GMA fiber]

FIG. 8

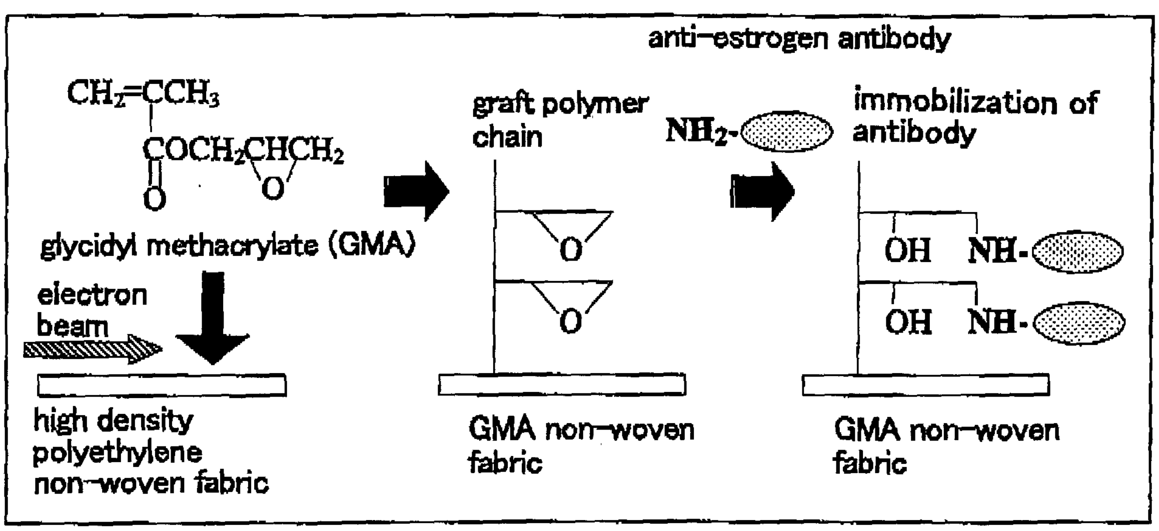
anti-estrogen antibody
glycidyl methacrylate (GMA)
electron beam
high density polyethylene non-woven fabric
graft polymer chain
GMA non-woven fabric
immobilization of antibody
GMA non-woven fabric

# FIG. 9

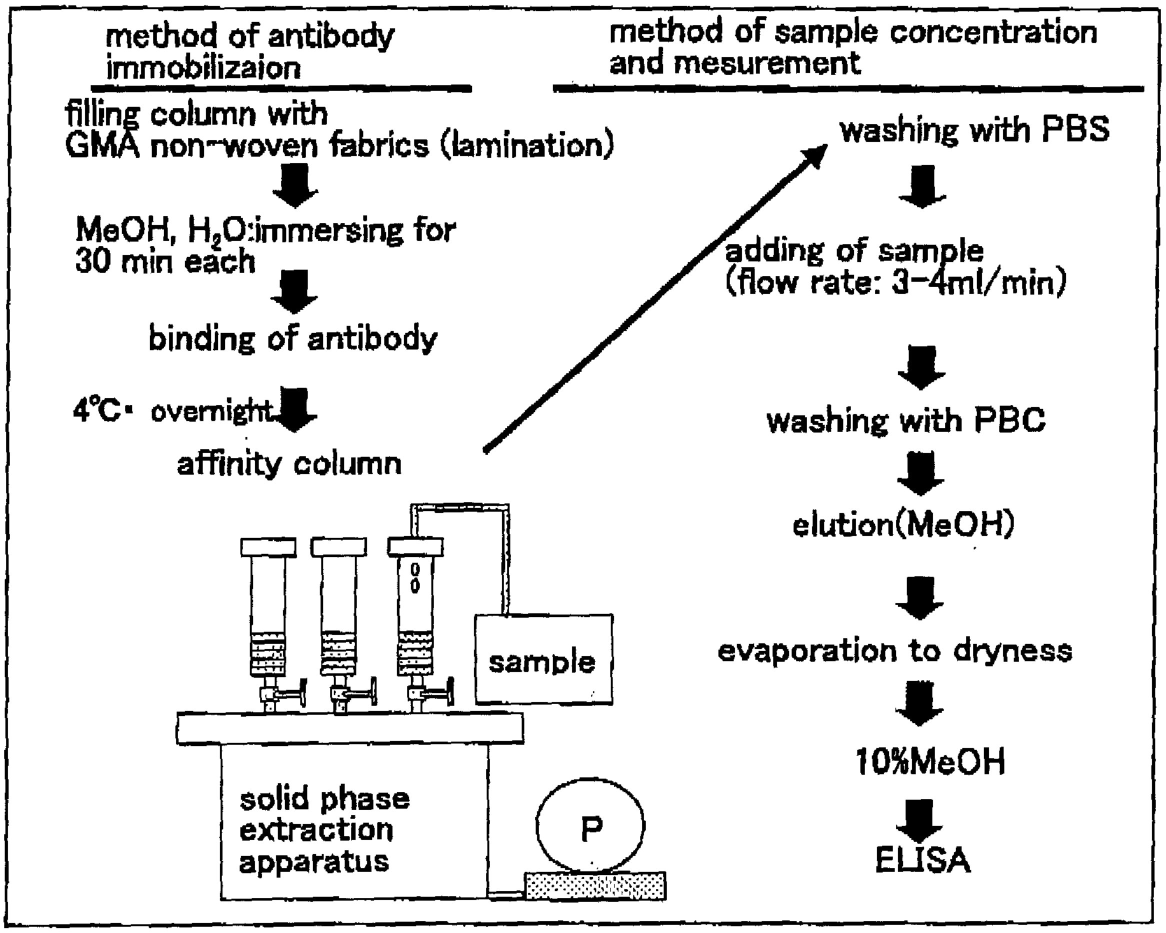
method of antibody immobilizaion
filling column with GMA non-woven fabrics (lamination)
MeOH, $H_2O$:immersing for 30 min each
binding of antibody
4°C· overnight
affinity column
sample
solid phase extraction apparatus
P
method of sample concentration and mesurement
washing with PBS
adding of sample (flow rate: 3-4ml/min)
washing with PBC
elution(MeOH)
evaporation to dryness
10%MeOH
ELISA

FIG. 10

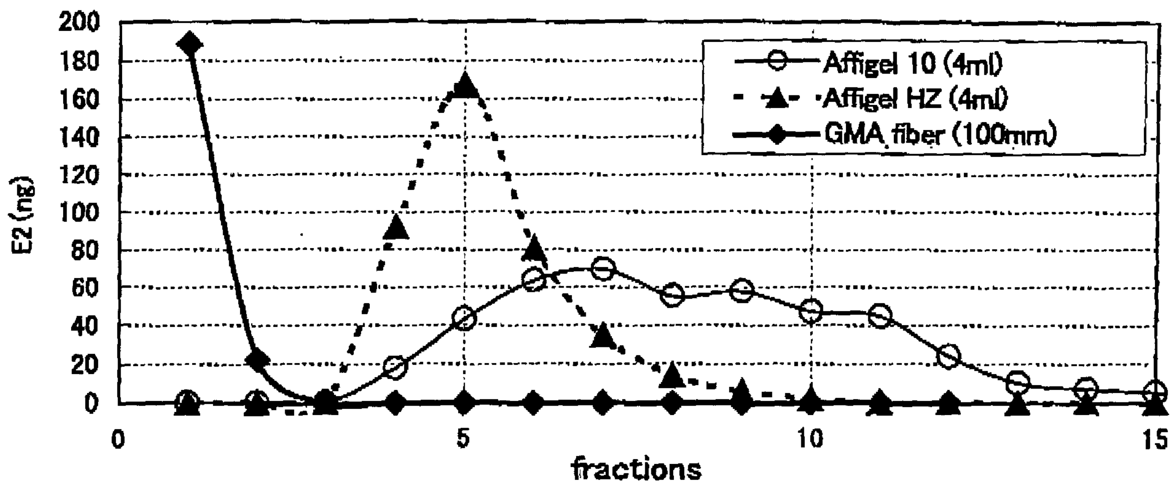


comparison of E2 elution pattern of each carrier

# FIG. 11

hapten

estriol-16-glucuronide

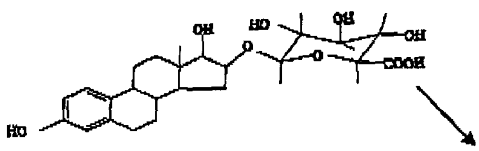

carrier protein

KLH (keyhole limpet hemocyanin)

peptide bond (immunogen)

immunization

rabbit

anti-estrogen antiserum

ammonium sulfate precipitation

·anion exchange resin

purified antibody

FIG. 12

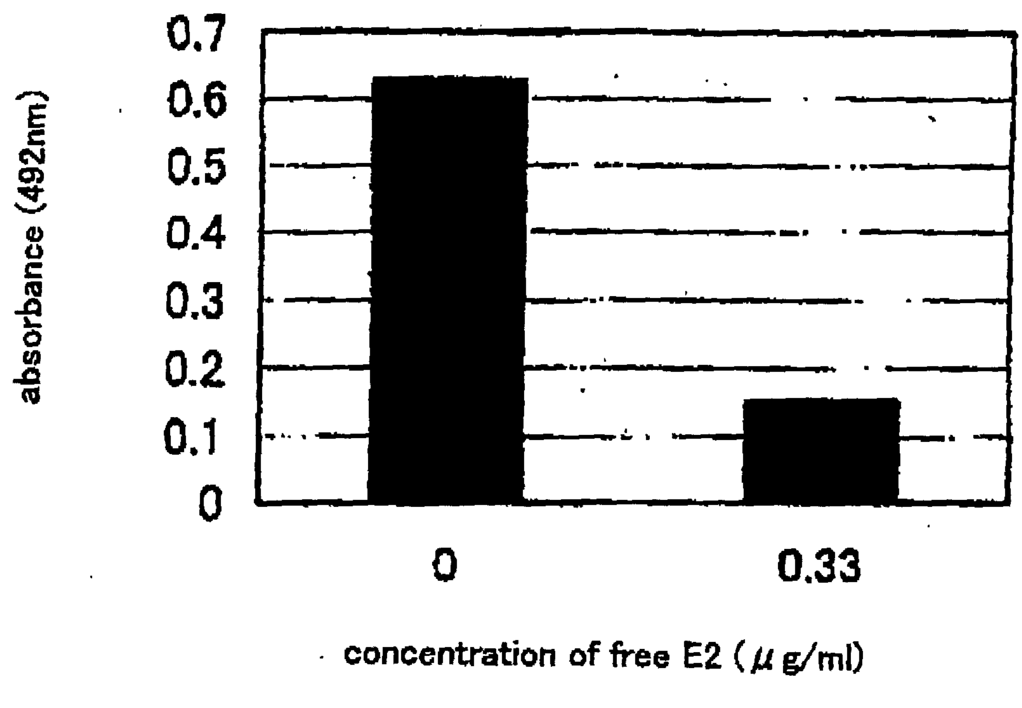
absorbance (492nm)
0.7
0.6
0.5
0.4
0.3
0.2
0.1
0
0
0.33
concentration of free E2 (μg/ml)

FIG. 13

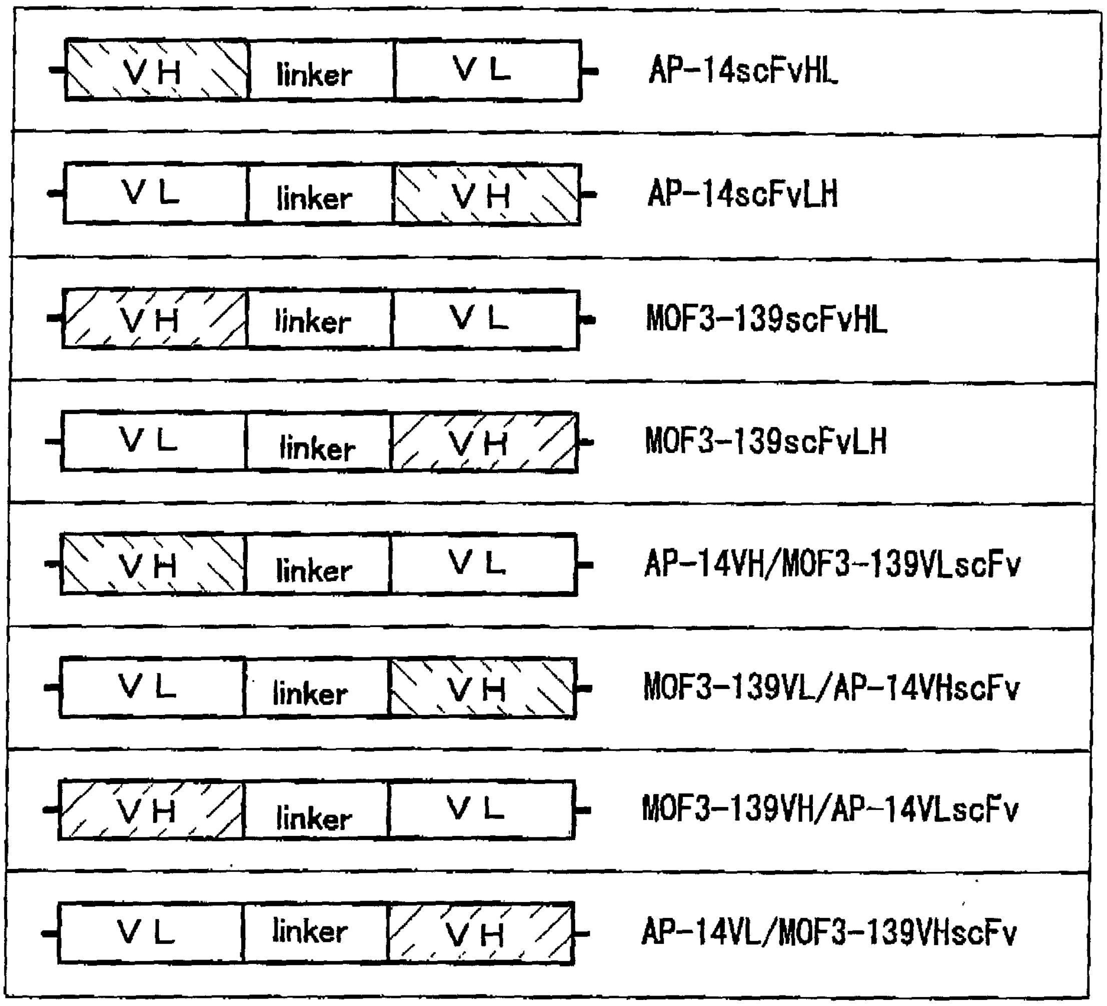
VH
linker
VL
AP-14scFvHL
VL
linker
VH
AP-14scFvLH
VH
linker
VL
MOF3-139scFvHL
VL
linker
VH
MOF3-139scFvLH
VH
linker
VL
AP-14VH/MOF3-139VLscFv
VL
linker
VH
MOF3-139VL/AP-14VHscFv
VH
linker
VL
MOF3-139VH/AP-14VLscFv
VL
linker
VH
AP-14VL/MOF3-139VHscFv

# FIG. 14

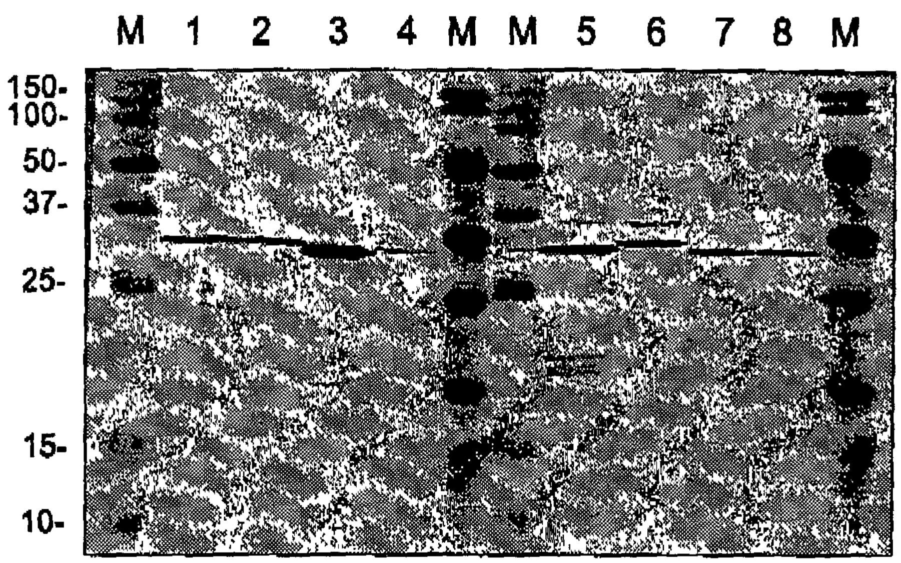


M; molecular weight marker,

1; AP-14scFvHL,
2; AP-14scFvLH,
3; MOF3-139scFvHL,
4; MOF3-139scFvLH,
5; MOF3-139VH/AP-14VLscFv,
6; AP-14VH/MOF3-139VLscFv,
7; AP-14VL/MOF3-139VHscFv,
8; MOF3-139VL/AP-14VHscFv.

FIG. 15

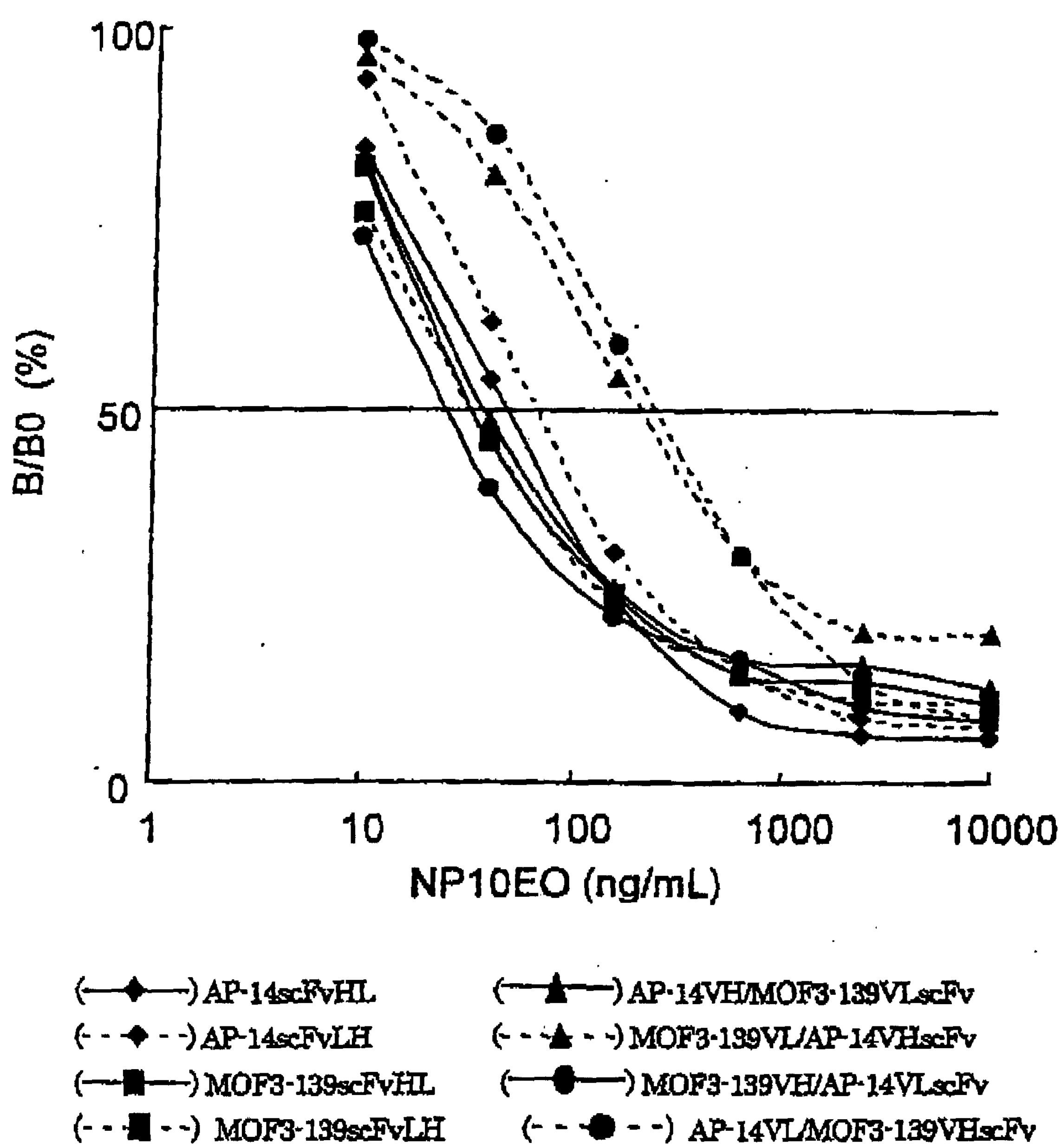

100
50
0
B/B0 (%)
1
10
100
1000
10000
NP10EO (ng/mL)
AP-14scFvHL
AP-14scFvLH
MOF3-139scFvHL
MOF3-139scFvLH
AP-14VH/MOF3-139VLscFv
MOF3-139VL/AP-14VHscFv
MOF3-139VH/AP-14VLscFv
AP-14VL/MOF3-139VHscFv

## INTERNATIONAL SEARCH REPORT

International application No.
PCT/JP03/06840

A. CLASSIFICATION OF SUBJECT MATTER
Int.Cl$^7$ B01J20/26, B01D71/82, 69/08, G01N30/48

According to International Patent Classification (IPC) or to both national classification and IPC

B. FIELDS SEARCHED

Minimum documentation searched (classification system followed by classification symbols)
Int.Cl$^7$ B01J20/26, B01D71/82, 69/08, G01N30/48

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | Akiko GOTO et al., "Hoshasen Graft Jugoho o Tekiyo Shita Immuno Affinity Chushutsu Zairyo no Sakusei", Hoshasen Kagaku Toronkai Koen Yoshisyu, Vol.43, pages 16 to 17, (2000) | 1-5,8-14<br>6,7 |
| Y | JP 8-29428 A (Masashi FUNAYAMA), 02 February, 1996 (02.02.96), Claims; page 5, column 8, lines 14 to 19; examples (Family: none) | 6,7 |
| A | JP 7-113799 A (Toyobo Co., Ltd.), 02 May, 1995 (02.05.95), Claims (Family: none) | 1-14 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>02 September, 2003 (02.09.03) | Date of mailing of the international search report<br>24 September, 2003 (24.09.03) |
| Name and mailing address of the ISA/<br>Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP03/06840

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-350597 A (Kubota Corp.), 19 December, 2000 (19.12.00), Claims (Family: none) | 1-14 |
| A | JP 2001-272400 A (Sekisui Chemical Co., Ltd.), 05 October, 2001 (05.10.01), Claims; page 6, column 9, lines 16 to 18 (Family: none) | 1-14 |

Form PCT/ISA/210 (continuation of second sheet) (July 1998)

SEQUENCE LISTING

```
<110>  Japan EnviroChemicals, Ltd.
       Environment Purification Research Institute Inc.

<120>  Separating materials, concentration methods and clean up methods for
environmental hormones

<130>  09556

<150>  JP 2002-159728
<151>  2002-05-31

<160>  55

<170>  PatentIn version 3.0

<210>  1
<211>  351
<212>  DNA
<213>  Mouse

<220>
<221>  CDS
<222>  (1)..(351)

<400>  1
gtg cag ctg cag cag tct ggg gct gaa ctg gct aga cct ggg gct tca        48
Val Gln Leu Gln Gln Ser Gly Ala Glu Leu Ala Arg Pro Gly Ala Ser
1               5                   10                  15

gtg aaa ttg tcc tgc aag gct tct gac tac acc ttt act aac tac tgg        96
Val Lys Leu Ser Cys Lys Ala Ser Asp Tyr Thr Phe Thr Asn Tyr Trp
            20                  25                  30

atg cag tgg ata aaa cag agg cct gga cag ggt ctg gaa tgg att ggg       144
Met Gln Trp Ile Lys Gln Arg Pro Gly Gln Gly Leu Glu Trp Ile Gly
        35                  40                  45

act att tct ctt gga aat ggt gat acc aga tac act cag aag ttc aag       192
Thr Ile Ser Leu Gly Asn Gly Asp Thr Arg Tyr Thr Gln Lys Phe Lys
    50                  55                  60

gac aag gcc aca ttg act gca gat aaa tcc tcc agt aca gtc tac atg       240
Asp Lys Ala Thr Leu Thr Ala Asp Lys Ser Ser Ser Thr Val Tyr Met
65                  70                  75                  80

caa ctc acc aac ttg gca tct gag gat tct gcg gtc tat tac tgt tca       288
Gln Leu Thr Asn Leu Ala Ser Glu Asp Ser Ala Val Tyr Tyr Cys Ser
                85                  90                  95

atc tat tcc ttc gtt agg ggc ccc gga ggt tgg ggc caa ggc acc act       336
Ile Tyr Ser Phe Val Arg Gly Pro Gly Gly Trp Gly Gln Gly Thr Thr
            100                 105                 110

ctc aca gtc tcc tcg                                                   351
Leu Thr Val Ser Ser
        115


<210>  2
<211>  117
<212>  PRT
```

<213> Mouse

<400> 2

```
Val Gln Leu Gln Gln Ser Gly Ala Glu Leu Ala Arg Pro Gly Ala Ser
1               5                   10                  15

Val Lys Leu Ser Cys Lys Ala Ser Asp Tyr Thr Phe Thr Asn Tyr Trp
            20                  25                  30

Met Gln Trp Ile Lys Gln Arg Pro Gly Gln Gly Leu Glu Trp Ile Gly
        35                  40                  45

Thr Ile Ser Leu Gly Asn Gly Asp Thr Arg Tyr Thr Gln Lys Phe Lys
    50                  55                  60

Asp Lys Ala Thr Leu Thr Ala Asp Lys Ser Ser Ser Thr Val Tyr Met
65                  70                  75                  80

Gln Leu Thr Asn Leu Ala Ser Glu Asp Ser Ala Val Tyr Tyr Cys Ser
                85                  90                  95

Ile Tyr Ser Phe Val Arg Gly Pro Gly Gly Trp Gly Gln Gly Thr Thr
            100                 105                 110

Leu Thr Val Ser Ser
        115
```

<210> 3
<211> 324
<212> DNA
<213> Mouse

<220>
<221> CDS
<222> (1)..(324)

<400> 3

```
gat atc gtg atg act cag tct ccc aca ttc ttg tct aca tca tta gga      48
Asp Ile Val Met Thr Gln Ser Pro Thr Phe Leu Ser Thr Ser Leu Gly
1               5                   10                  15

gac aaa atc acc atc acc tgc aag gcc agt cag gat gtg ggt ccc gct      96
Asp Lys Ile Thr Ile Thr Cys Lys Ala Ser Gln Asp Val Gly Pro Ala
            20                  25                  30

ata gcc tgg tat caa cag aca cca gga caa tct cct aga cta ctg att     144
Ile Ala Trp Tyr Gln Gln Thr Pro Gly Gln Ser Pro Arg Leu Leu Ile
        35                  40                  45

tat tgg aca tcc acc cgg cac act gga gtc cct gat cgc ttc aca ggc     192
Tyr Trp Thr Ser Thr Arg His Thr Gly Val Pro Asp Arg Phe Thr Gly
    50                  55                  60

agt gga tct ggg aca gat ttc act ctc tcc att ggc gat gtt cag tct     240
Ser Gly Ser Gly Thr Asp Phe Thr Leu Ser Ile Gly Asp Val Gln Ser
65                  70                  75                  80

gaa gac ttg aca gat tat ttc tgt cac caa tat cgc agc tat cca tac     288
Glu Asp Leu Thr Asp Tyr Phe Cys His Gln Tyr Arg Ser Tyr Pro Tyr
                85                  90                  95
```

```
aca ttc ggg ggg ggg acc aag ctg gaa ata aaa cgg                          324
Thr Phe Gly Gly Gly Thr Lys Leu Glu Ile Lys Arg
            100                 105


<210>  4
<211>  108
<212>  PRT
<213>  Mouse

<400>  4

Asp Ile Val Met Thr Gln Ser Pro Thr Phe Leu Ser Thr Ser Leu Gly
1               5                   10                  15

Asp Lys Ile Thr Ile Thr Cys Lys Ala Ser Gln Asp Val Gly Pro Ala
            20                  25                  30

Ile Ala Trp Tyr Gln Gln Thr Pro Gly Gln Ser Pro Arg Leu Leu Ile
        35                  40                  45

Tyr Trp Thr Ser Thr Arg His Thr Gly Val Pro Asp Arg Phe Thr Gly
    50                  55                  60

Ser Gly Ser Gly Thr Asp Phe Thr Leu Ser Ile Gly Asp Val Gln Ser
65                  70                  75                  80

Glu Asp Leu Thr Asp Tyr Phe Cys His Gln Tyr Arg Ser Tyr Pro Tyr
                85                  90                  95

Thr Phe Gly Gly Gly Thr Lys Leu Glu Ile Lys Arg
            100                 105


<210>  5
<211>  354
<212>  DNA
<213>  Mouse

<220>
<221>  CDS
<222>  (1)..(354)

<400>  5
atc cag ttg gtg cag tct gga cct gaa ctg aag aag cct gga gag aca       48
Ile Gln Leu Val Gln Ser Gly Pro Glu Leu Lys Lys Pro Gly Glu Thr
1               5                   10                  15

gtc aag atc tcc tgc aag gct tct ggg ttt acc ttc aga aac tat gga       96
Val Lys Ile Ser Cys Lys Ala Ser Gly Phe Thr Phe Arg Asn Tyr Gly
            20                  25                  30

atg aac tgg gtg agg cag tct cca gga gag ggt tta aag tgg atg ggc      144
Met Asn Trp Val Arg Gln Ser Pro Gly Glu Gly Leu Lys Trp Met Gly
        35                  40                  45

tgg ata aac acc tac act gga gag cca aca tat cct gat gcc ttc aag      192
Trp Ile Asn Thr Tyr Thr Gly Glu Pro Thr Tyr Pro Asp Ala Phe Lys
    50                  55                  60

gga cgg ttt gcc atc tct ctg gaa acc tct gcc agc act gcc tat ttg      240
Gly Arg Phe Ala Ile Ser Leu Glu Thr Ser Ala Ser Thr Ala Tyr Leu
65                  70                  75                  80
```

```
cag atc aac agc ctc aaa aat gag gac acg gct aca tat ttc tgt gca       288
Gln Ile Asn Ser Leu Lys Asn Glu Asp Thr Ala Thr Tyr Phe Cys Ala
                85                  90                  95

aga ggg gac tat agg tac cgt tcc ttt gac tac tgg ggc caa ggc acc       336
Arg Gly Asp Tyr Arg Tyr Arg Ser Phe Asp Tyr Trp Gly Gln Gly Thr
            100                 105                 110

act ctc aca gtc tcc tcg                                               354
Thr Leu Thr Val Ser Ser
        115


<210>  6
<211>  118
<212>  PRT
<213>  Mouse

<400>  6

Ile Gln Leu Val Gln Ser Gly Pro Glu Leu Lys Lys Pro Gly Glu Thr
1               5                   10                  15

Val Lys Ile Ser Cys Lys Ala Ser Gly Phe Thr Phe Arg Asn Tyr Gly
            20                  25                  30

Met Asn Trp Val Arg Gln Ser Pro Gly Glu Gly Leu Lys Trp Met Gly
        35                  40                  45

Trp Ile Asn Thr Tyr Thr Gly Glu Pro Thr Tyr Pro Asp Ala Phe Lys
    50                  55                  60

Gly Arg Phe Ala Ile Ser Leu Glu Thr Ser Ala Ser Thr Ala Tyr Leu
65                  70                  75                  80

Gln Ile Asn Ser Leu Lys Asn Glu Asp Thr Ala Thr Tyr Phe Cys Ala
                85                  90                  95

Arg Gly Asp Tyr Arg Tyr Arg Ser Phe Asp Tyr Trp Gly Gln Gly Thr
            100                 105                 110

Thr Leu Thr Val Ser Ser
        115


<210>  7
<211>  339
<212>  DNA
<213>  Mouse

<220>
<221>  CDS
<222>  (1)..(339)

<400>  7
gat gtt ttg atg acc caa act cca ctc act ttg tcg gtt acc att gga        48
Asp Val Leu Met Thr Gln Thr Pro Leu Thr Leu Ser Val Thr Ile Gly
1               5                   10                  15

caa cca gcc tcc atc tct tgc aag tca agt cag agc ctc tta aat agt        96
Gln Pro Ala Ser Ile Ser Cys Lys Ser Ser Gln Ser Leu Leu Asn Ser
            20                  25                  30
```

```
gat gga aag aca tat ttg cat tgg ttg tta cag agg cca ggc cag tct      144
Asp Gly Lys Thr Tyr Leu His Trp Leu Leu Gln Arg Pro Gly Gln Ser
        35                  40                  45

cca aag cgc cta atc tat ctg gtg tct aaa ctg gac tct gga gtc cct      192
Pro Lys Arg Leu Ile Tyr Leu Val Ser Lys Leu Asp Ser Gly Val Pro
    50                  55                  60

gac agg ttc tct ggc agt gga tca ggg aca gat ttc aca ctg aaa atc      240
Asp Arg Phe Ser Gly Ser Gly Ser Gly Thr Asp Phe Thr Leu Lys Ile
65                  70                  75                  80

agc aga gtg gag gct gag gat ttg gga gtt tat tat tgc tgg caa gtt      288
Ser Arg Val Glu Ala Glu Asp Leu Gly Val Tyr Tyr Cys Trp Gln Val
                85                  90                  95

aca cat ttt cct ctc acg ttc ggt gct ggg acc aag ctg gag ctg aaa      336
Thr His Phe Pro Leu Thr Phe Gly Ala Gly Thr Lys Leu Glu Leu Lys
            100                 105                 110

cgg                                                                  339
Arg
```

```
<210>  8
<211>  113
<212>  PRT
<213>  Mouse

<400>  8

Asp Val Leu Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asn Ser
            20                  25                  30

Asp Gly Lys Thr Tyr Leu His Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Val
                85                  90                  95

Thr His Phe Pro Leu Thr Phe Gly Ala Gly Thr Lys Leu Glu Leu Lys
            100                 105                 110

Arg
```

```
<210>  9
<211>  354
<212>  DNA
<213>  Mouse
```

<220>
<221> CDS
<222> (1)..(354)

<220>
<221> Unsure
<222> (13)..(13)
<223> n

<400> 9
```
gtt act ctg aaa nag tct ggc cct ggg ata ttg cag ccc tcc cag acc        48
Val Thr Leu Lys Xaa Ser Gly Pro Gly Ile Leu Gln Pro Ser Gln Thr
1               5                   10                  15

ctc agt ctg act tgt tct ttc tct ggg ttt tca ctg agc act tct gat        96
Leu Ser Leu Thr Cys Ser Phe Ser Gly Phe Ser Leu Ser Thr Ser Asp
            20                  25                  30

ctg ggt gtg agt tgg att cgt cag act tca gga aag ggt ctg gag tgg       144
Leu Gly Val Ser Trp Ile Arg Gln Thr Ser Gly Lys Gly Leu Glu Trp
        35                  40                  45

ctg gca cac att tac tgg aat gat gac aaa tat tat aat cca tcc ctg       192
Leu Ala His Ile Tyr Trp Asn Asp Asp Lys Tyr Tyr Asn Pro Ser Leu
    50                  55                  60

aag agc cgg ctc aca atc tcc aag gat acc tcc gga aac ctg gta ttc       240
Lys Ser Arg Leu Thr Ile Ser Lys Asp Thr Ser Gly Asn Leu Val Phe
65                  70                  75                  80

ctc agg atc acc agt gtg gac act gac gat tct gcc aca tac tat tgt       288
Leu Arg Ile Thr Ser Val Asp Thr Asp Asp Ser Ala Thr Tyr Tyr Cys
                85                  90                  95

gct cga agt cct ctc tac ggt agt ctt act tac tgg ggc caa ggg act       336
Ala Arg Ser Pro Leu Tyr Gly Ser Leu Thr Tyr Trp Gly Gln Gly Thr
            100                 105                 110

ctg gtc act gtc tct gcg                                               354
Leu Val Thr Val Ser Ala
        115
```

<210> 10
<211> 118
<212> PRT
<213> Mouse

<400> 10

```
Val Thr Leu Lys Xaa Ser Gly Pro Gly Ile Leu Gln Pro Ser Gln Thr
1               5                   10                  15

Leu Ser Leu Thr Cys Ser Phe Ser Gly Phe Ser Leu Ser Thr Ser Asp
            20                  25                  30

Leu Gly Val Ser Trp Ile Arg Gln Thr Ser Gly Lys Gly Leu Glu Trp
        35                  40                  45

Leu Ala His Ile Tyr Trp Asn Asp Asp Lys Tyr Tyr Asn Pro Ser Leu
    50                  55                  60

Lys Ser Arg Leu Thr Ile Ser Lys Asp Thr Ser Gly Asn Leu Val Phe
```

```
65                  70                  75                  80

Leu Arg Ile Thr Ser Val Asp Thr Asp Asp Ser Ala Thr Tyr Tyr Cys
                85                  90                  95

Ala Arg Ser Pro Leu Tyr Gly Ser Leu Thr Tyr Trp Gly Gln Gly Thr
            100                 105                 110

Leu Val Thr Val Ser Ala
        115


<210>  11
<211>  654
<212>  DNA
<213>  Mouse

<220>
<221>  CDS
<222>  (1)..(654)

<400>  11
gac att gtg atg tca cag tct cca tcc tcc ctg gct gtg tca gta gga        48
Asp Ile Val Met Ser Gln Ser Pro Ser Ser Leu Ala Val Ser Val Gly
1               5                   10                  15

gag aag gtc act ttg aac tgc aaa tcc agt cag agt ctg ctc aac agt        96
Glu Lys Val Thr Leu Asn Cys Lys Ser Ser Gln Ser Leu Leu Asn Ser
            20                  25                  30

aga acc cga aag aac tac ttg gct tgg tac cag cag aaa cca gga cag       144
Arg Thr Arg Lys Asn Tyr Leu Ala Trp Tyr Gln Gln Lys Pro Gly Gln
        35                  40                  45

tct cct aaa ctg ctg atc tct tgg gca tcc act agg gaa tct ggg gtc       192
Ser Pro Lys Leu Leu Ile Ser Trp Ala Ser Thr Arg Glu Ser Gly Val
    50                  55                  60

cct gat cgt ttc aca ggc agt gga tct ggg aca gat ttc act ctc acc       240
Pro Asp Arg Phe Thr Gly Ser Gly Ser Gly Thr Asp Phe Thr Leu Thr
65                  70                  75                  80

atc agc agt gtg cag gct gaa gac ctg gca gtt tat tac tgc aat caa       288
Ile Ser Ser Val Gln Ala Glu Asp Leu Ala Val Tyr Tyr Cys Asn Gln
                85                  90                  95

ttt tat tat cta ctc acg ttc ggt gct ggg acc aag ctg gag ctg aaa       336
Phe Tyr Tyr Leu Leu Thr Phe Gly Ala Gly Thr Lys Leu Glu Leu Lys
            100                 105                 110

cgg gct gat gct gca cca act gta tcc atc ttc cca cca tcc agt gag       384
Arg Ala Asp Ala Ala Pro Thr Val Ser Ile Phe Pro Pro Ser Ser Glu
        115                 120                 125

cag tta aca tct gga ggt gcc tca gtc gtg tgc ttc ttg aac aac ttc       432
Gln Leu Thr Ser Gly Gly Ala Ser Val Val Cys Phe Leu Asn Asn Phe
    130                 135                 140

tac ccc aaa gac atc aat gtc aag tgg aag att gat ggc agt gaa cga       480
Tyr Pro Lys Asp Ile Asn Val Lys Trp Lys Ile Asp Gly Ser Glu Arg
145                 150                 155                 160

caa aat ggc gtc ctg aac agt tgg act gat cag gac agc aaa gac agc       528
```

```
Gln Asn Gly Val Leu Asn Ser Trp Thr Asp Gln Asp Ser Lys Asp Ser
                165                 170                 175

acc tac agc atg agc agc acc ctc acg ttg acc aag gac gag tat gaa      576
Thr Tyr Ser Met Ser Ser Thr Leu Thr Leu Thr Lys Asp Glu Tyr Glu
            180                 185                 190

cga cat aac agc tat acc tgt gag gcc act cac aag aca tca act tca      624
Arg His Asn Ser Tyr Thr Cys Glu Ala Thr His Lys Thr Ser Thr Ser
        195                 200                 205

ccc att gtc aag agc ttc aac agg aat gag                              654
Pro Ile Val Lys Ser Phe Asn Arg Asn Glu
    210                 215


<210>  12
<211>  218
<212>  PRT
<213>  Mouse

<400>  12

Asp Ile Val Met Ser Gln Ser Pro Ser Ser Leu Ala Val Ser Val Gly
1               5                   10                  15

Glu Lys Val Thr Leu Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Ser Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Asn Gln
                85                  90                  95

Phe Tyr Tyr Leu Leu Thr Phe Gly Ala Gly Thr Lys Leu Glu Leu Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser
        195                 200                 205

Pro Ile Val Lys Ser Phe Asn Arg Asn Glu
```

```
    210                 215

<210>  13
<211>  355
<212>  DNA
<213>  Mouse

<220>
<221>  CDS
<222>  (1)..(354)

<400>  13
gtg cag gtg aag gag tca gga cct ggc ctg gtg gcg ccc tca cag agc        48
Val Gln Val Lys Glu Ser Gly Pro Gly Leu Val Ala Pro Ser Gln Ser
1               5                   10                  15

ctg tcc atc agt tgc act gtc tct ggg ttt cca tta acc agt tat ggt        96
Leu Ser Ile Ser Cys Thr Val Ser Gly Phe Pro Leu Thr Ser Tyr Gly
            20                  25                  30

gta cag tgg gtt cgc cag cct cca gga aag ggt ctg gag tgg ctg gga       144
Val Gln Trp Val Arg Gln Pro Pro Gly Lys Gly Leu Glu Trp Leu Gly
        35                  40                  45

gta ata tgg cct ggt gga agc aca aat tat aat tcg gct ctc atg tcc       192
Val Ile Trp Pro Gly Gly Ser Thr Asn Tyr Asn Ser Ala Leu Met Ser
    50                  55                  60

aga ctg agc atc agc aaa gac aac tcc aag agc caa gtt ttc tta aaa       240
Arg Leu Ser Ile Ser Lys Asp Asn Ser Lys Ser Gln Val Phe Leu Lys
65                  70                  75                  80

atg aac agt ctg caa act gat gac aca gcc atg tac tac tgt gcc gga       288
Met Asn Ser Leu Gln Thr Asp Asp Thr Ala Met Tyr Tyr Cys Ala Gly
                85                  90                  95

tgg ttc caa acg gga ccg agg cta ttt gct tac tgg ggc caa ggg act       336
Trp Phe Gln Thr Gly Pro Arg Leu Phe Ala Tyr Trp Gly Gln Gly Thr
            100                 105                 110

ctg gtc act gtc tct gcg a                                             355
Leu Val Thr Val Ser Ala
        115


<210>  14
<211>  118
<212>  PRT
<213>  Mouse

<400>  14

Val Gln Val Lys Glu Ser Gly Pro Gly Leu Val Ala Pro Ser Gln Ser
1               5                   10                  15

Leu Ser Ile Ser Cys Thr Val Ser Gly Phe Pro Leu Thr Ser Tyr Gly
            20                  25                  30

Val Gln Trp Val Arg Gln Pro Pro Gly Lys Gly Leu Glu Trp Leu Gly
        35                  40                  45

Val Ile Trp Pro Gly Gly Ser Thr Asn Tyr Asn Ser Ala Leu Met Ser
```

```
    50                  55                  60

Arg Leu Ser Ile Ser Lys Asp Asn Ser Lys Ser Gln Val Phe Leu Lys
65                  70                  75                  80

Met Asn Ser Leu Gln Thr Asp Asp Thr Ala Met Tyr Tyr Cys Ala Gly
                85                  90                  95

Trp Phe Gln Thr Gly Pro Arg Leu Phe Ala Tyr Trp Gly Gln Gly Thr
            100                 105                 110

Leu Val Thr Val Ser Ala
        115


<210>  15
<211>  321
<212>  DNA
<213>  Mouse

<220>
<221>  CDS
<222>  (1)..(321)

<220>
<221>  Unsure
<222>  (1)..(21)
<223>  n

<400>  15
nnn nnn nnn nnn nnn nnn nnn cca gcc tcc cta tct gca tct gtg gga       48
Asp Ile Gln Met Thr Gln Ser Pro Ala Ser Leu Ser Ala Ser Val Gly
1               5                   10                  15

gaa act gtc acc atc aca tgt cga gca agt gag aat att cac agt tat       96
Glu Thr Val Thr Ile Thr Cys Arg Ala Ser Glu Asn Ile His Ser Tyr
            20                  25                  30

tta gca tgg tat cag cag aaa cag gga aaa tct cct cag gtc ctg gtc      144
Leu Ala Trp Tyr Gln Gln Lys Gln Gly Lys Ser Pro Gln Val Leu Val
        35                  40                  45

tat aat gca aaa acc tta gga gaa ggt gtg tca tca agg ttc agt ggc      192
Tyr Asn Ala Lys Thr Leu Gly Glu Gly Val Ser Ser Arg Phe Ser Gly
    50                  55                  60

agt gga tca ggc aca cag ttt tct ctg aag atc aac agc ctg cag cct      240
Ser Gly Ser Gly Thr Gln Phe Ser Leu Lys Ile Asn Ser Leu Gln Pro
65                  70                  75                  80

gaa gat ttt ggg act tat tac tgt caa aat cat tat ggt agt ccg tgg      288
Glu Asp Phe Gly Thr Tyr Tyr Cys Gln Asn His Tyr Gly Ser Pro Trp
                85                  90                  95

acg ttc ggt gga ggc acc aag ctg gaa tca aaa                          321
Thr Phe Gly Gly Gly Thr Lys Leu Glu Ser Lys
            100                 105


<210>  16
<211>  107
<212>  PRT
<213>  Mouse
```

<400> 16

```
Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile His Ser Tyr
            20                  25                  30

Leu Ala Trp Tyr Gln Gln Lys Gln Gly Lys Ser Pro Gln Val Leu Val
        35                  40                  45

Tyr Asn Ala Lys Thr Leu Gly Glu Gly Val Ser Ser Arg Phe Ser Gly
    50                  55                  60

Ser Gly Ser Gly Thr Gln Phe Ser Leu Lys Ile Asn Ser Leu Gln Pro
65                  70                  75                  80

Glu Asp Phe Gly Thr Tyr Tyr Cys Gln Asn His Tyr Gly Ser Pro Trp
                85                  90                  95

Thr Phe Gly Gly Gly Thr Lys Leu Glu Ser Lys
            100                 105
```

<210> 17
<211> 375
<212> DNA
<213> Mouse

<220>
<221> CDS
<222> (1)..(375)

<400> 17

```
cag gtc cag ctc cag cag tct gca gct gaa ctg gca aga cct ggg gcc       48
Gln Val Gln Leu Gln Gln Ser Ala Ala Glu Leu Ala Arg Pro Gly Ala
1               5                   10                  15

tca gtg aag atg tcc tgc aag gct tct ggc tat aga ttt act agg tac       96
Ser Val Lys Met Ser Cys Lys Ala Ser Gly Tyr Arg Phe Thr Arg Tyr
            20                  25                  30

acg atg cac tgg gta aaa cag agg cct gga cag ggt ctg gaa tgg att      144
Thr Met His Trp Val Lys Gln Arg Pro Gly Gln Gly Leu Glu Trp Ile
        35                  40                  45

gga aac att aat cct agc agt gga gat act gag tac aat cag aag ttc      192
Gly Asn Ile Asn Pro Ser Ser Gly Asp Thr Glu Tyr Asn Gln Lys Phe
    50                  55                  60

aag gac aag acc aca ttg act gca gac aaa tcc tcc acc aca gcc tac      240
Lys Asp Lys Thr Thr Leu Thr Ala Asp Lys Ser Ser Thr Thr Ala Tyr
65                  70                  75                  80

atg cag ctg agc agc ctg aca tct gag gac tct gcg gtc tat tac tgt      288
Met Gln Leu Ser Ser Leu Thr Ser Glu Asp Ser Ala Val Tyr Tyr Cys
                85                  90                  95

gca acc cca att ttt tac tac ggt agt agg tac gtg agg tat gaa atg      336
Ala Thr Pro Ile Phe Tyr Tyr Gly Ser Arg Tyr Val Arg Tyr Glu Met
            100                 105                 110
```

```
gac tac tgg ggt caa gga acc tca gtc acc gtc tcc tca                 375
Asp Tyr Trp Gly Gln Gly Thr Ser Val Thr Val Ser Ser
        115                 120                 125


<210>  18
<211>  125
<212>  PRT
<213>  Mouse

<400>  18

Gln Val Gln Leu Gln Gln Ser Ala Ala Glu Leu Ala Arg Pro Gly Ala
1               5                   10                  15

Ser Val Lys Met Ser Cys Lys Ala Ser Gly Tyr Arg Phe Thr Arg Tyr
            20                  25                  30

Thr Met His Trp Val Lys Gln Arg Pro Gly Gln Gly Leu Glu Trp Ile
        35                  40                  45

Gly Asn Ile Asn Pro Ser Ser Gly Asp Thr Glu Tyr Asn Gln Lys Phe
    50                  55                  60

Lys Asp Lys Thr Thr Leu Thr Ala Asp Lys Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Ala Thr Pro Ile Phe Tyr Tyr Gly Ser Arg Tyr Val Arg Tyr Glu Met
            100                 105                 110

Asp Tyr Trp Gly Gln Gly Thr Ser Val Thr Val Ser Ser
        115                 120                 125


<210>  19
<211>  339
<212>  DNA
<213>  Mouse

<220>
<221>  CDS
<222>  (1)..(339)

<400>  19
gat att gtg atg acg cag gct gca ttc tcc aat cca gtc act ctt gga      48
Asp Ile Val Met Thr Gln Ala Ala Phe Ser Asn Pro Val Thr Leu Gly
1               5                   10                  15

aca tca act tcc atc tcc tgc agg tct act aag agt ctc cta cat agt      96
Thr Ser Thr Ser Ile Ser Cys Arg Ser Thr Lys Ser Leu Leu His Ser
            20                  25                  30

aat ggc atc act tat ttg tat tgg tat ctg cag aag cca ggc cag tct     144
Asn Gly Ile Thr Tyr Leu Tyr Trp Tyr Leu Gln Lys Pro Gly Gln Ser
        35                  40                  45

cct cag ctc ctg att tat cag atg tcc aac ctt gcc tca gga gtc cca     192
Pro Gln Leu Leu Ile Tyr Gln Met Ser Asn Leu Ala Ser Gly Val Pro
    50                  55                  60
```

```
gac agg ttc agt agc agt ggg tca gga act gat ttc aca ctg aga atc      240
Asp Arg Phe Ser Ser Ser Gly Ser Gly Thr Asp Phe Thr Leu Arg Ile
65                  70                  75                  80

agc aga gtg gag gct gag gat gtg ggt gtt tat tac tgt gct caa aat      288
Ser Arg Val Glu Ala Glu Asp Val Gly Val Tyr Tyr Cys Ala Gln Asn
                85                  90                  95

cta gaa ctt ccg tac acg ttc gga ggg ggg acc aag ctg gaa ata aaa      336
Leu Glu Leu Pro Tyr Thr Phe Gly Gly Gly Thr Lys Leu Glu Ile Lys
            100                 105                 110

cgg                                                                  339
Arg


<210>  20
<211>  113
<212>  PRT
<213>  Mouse

<400>  20

Asp Ile Val Met Thr Gln Ala Ala Phe Ser Asn Pro Val Thr Leu Gly
1               5                   10                  15

Thr Ser Thr Ser Ile Ser Cys Arg Ser Thr Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Ala Ser Gly Val Pro
    50                  55                  60

Asp Arg Phe Ser Ser Ser Gly Ser Gly Thr Asp Phe Thr Leu Arg Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Leu Glu Ile Lys
            100                 105                 110

Arg


<210>  21
<211>  363
<212>  DNA
<213>  Mouse

<220>
<221>  CDS
<222>  (1)..(363)

<400>  21
cag gtt cag ctg cag cag tct gga gct gag ctg atg aag cct ggg gcc       48
Gln Val Gln Leu Gln Gln Ser Gly Ala Glu Leu Met Lys Pro Gly Ala
1               5                   10                  15
```

```
tca gtg aag ata tcc tgc aag gtt act ggc tac aca ttc agg agc tac       96
Ser Val Lys Ile Ser Cys Lys Val Thr Gly Tyr Thr Phe Arg Ser Tyr
            20                  25                  30

tgg ata gag tgg gta aag cag agg cct gga cat ggc ctt gag tgg att      144
Trp Ile Glu Trp Val Lys Gln Arg Pro Gly His Gly Leu Glu Trp Ile
        35                  40                  45

gga gag att tta gtt gga agt ggt agt act aag tat aat gag aag ttc      192
Gly Glu Ile Leu Val Gly Ser Gly Ser Thr Lys Tyr Asn Glu Lys Phe
    50                  55                  60

aag ggc aag gcc aca atc act gca cag aca tcc tcc aat aca gta tac      240
Lys Gly Lys Ala Thr Ile Thr Ala Gln Thr Ser Ser Asn Thr Val Tyr
65                  70                  75                  80

atg caa ctc agc agc ctg aca tct gag gac tct gcc gtc tat tac tgt      288
Met Gln Leu Ser Ser Leu Thr Ser Glu Asp Ser Ala Val Tyr Tyr Cys
                85                  90                  95

gca aga cct ttc ttc ggt agt agg tac gac tac tct gac ttc tgg ggc      336
Ala Arg Pro Phe Phe Gly Ser Arg Tyr Asp Tyr Ser Asp Phe Trp Gly
            100                 105                 110

caa ggc acc act ctc aca gtc tcc tca                                  363
Gln Gly Thr Thr Leu Thr Val Ser Ser
        115                 120


<210>  22
<211>  121
<212>  PRT
<213>  Mouse

<400>  22

Gln Val Gln Leu Gln Gln Ser Gly Ala Glu Leu Met Lys Pro Gly Ala
1               5                   10                  15

Ser Val Lys Ile Ser Cys Lys Val Thr Gly Tyr Thr Phe Arg Ser Tyr
            20                  25                  30

Trp Ile Glu Trp Val Lys Gln Arg Pro Gly His Gly Leu Glu Trp Ile
        35                  40                  45

Gly Glu Ile Leu Val Gly Ser Gly Ser Thr Lys Tyr Asn Glu Lys Phe
    50                  55                  60

Lys Gly Lys Ala Thr Ile Thr Ala Gln Thr Ser Ser Asn Thr Val Tyr
65                  70                  75                  80

Met Gln Leu Ser Ser Leu Thr Ser Glu Asp Ser Ala Val Tyr Tyr Cys
                85                  90                  95

Ala Arg Pro Phe Phe Gly Ser Arg Tyr Asp Tyr Ser Asp Phe Trp Gly
            100                 105                 110

Gln Gly Thr Thr Leu Thr Val Ser Ser
        115                 120


<210>  23
<211>  339
```

```
<212>  DNA
<213>  Mouse

<220>
<221>  CDS
<222>  (1)..(339)

<400>  23
gat att gtg atg acg cag gct gca ttc tcc aat cca gtc act ctt gga        48
Asp Ile Val Met Thr Gln Ala Ala Phe Ser Asn Pro Val Thr Leu Gly
1               5                   10                  15

tca tca gct tcc atc tcc tgc agg tct agt aag agt ctc cta cat aga        96
Ser Ser Ala Ser Ile Ser Cys Arg Ser Ser Lys Ser Leu Leu His Arg
            20                  25                  30

aat ggc atc act tat ttg tat tgg tat ctg cag aag cca ggc cag tct       144
Asn Gly Ile Thr Tyr Leu Tyr Trp Tyr Leu Gln Lys Pro Gly Gln Ser
        35                  40                  45

cct cag gtc ctg att tat cag atg tcc aac ctt gcc tca gga gtc cca       192
Pro Gln Val Leu Ile Tyr Gln Met Ser Asn Leu Ala Ser Gly Val Pro
    50                  55                  60

gac agg ttc agt agc agt ggg tca gga act gat ttc aca ctg aga atc       240
Asp Arg Phe Ser Ser Ser Gly Ser Gly Thr Asp Phe Thr Leu Arg Ile
65                  70                  75                  80

agc aga gtg gag gct gag gat gtg ggt gtt tat tac tgt gct caa aat       288
Ser Arg Val Glu Ala Glu Asp Val Gly Val Tyr Tyr Cys Ala Gln Asn
                85                  90                  95

cta gaa ctt ccg tac acg ttc gga ggg ggg acc aag ctg gaa ata aaa       336
Leu Glu Leu Pro Tyr Thr Phe Gly Gly Gly Thr Lys Leu Glu Ile Lys
            100                 105                 110

cgg                                                                   339
Arg


<210>  24
<211>  113
<212>  PRT
<213>  Mouse

<400>  24

Asp Ile Val Met Thr Gln Ala Ala Phe Ser Asn Pro Val Thr Leu Gly
1               5                   10                  15

Ser Ser Ala Ser Ile Ser Cys Arg Ser Ser Lys Ser Leu Leu His Arg
            20                  25                  30

Asn Gly Ile Thr Tyr Leu Tyr Trp Tyr Leu Gln Lys Pro Gly Gln Ser
        35                  40                  45

Pro Gln Val Leu Ile Tyr Gln Met Ser Asn Leu Ala Ser Gly Val Pro
    50                  55                  60

Asp Arg Phe Ser Ser Ser Gly Ser Gly Thr Asp Phe Thr Leu Arg Ile
65                  70                  75                  80
```

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Leu Glu Ile Lys
            100                 105                 110

Arg

<210> 25
<211> 42
<212> DNA
<213> Artificial Sequence

<220>

<223> Oligonucleotide designed to act as primer for PCR.

<400> 25
ctatgcggcc cagccggcca tggtbcagct gcagcagtct gg 42

<210> 26
<211> 22
<212> DNA
<213> Artificial Sequence

<220>

<223> Oligonucleotide designed to act as primer for PCR.

<400> 26
accactctca cagtctcctc gg 22

<210> 27
<211> 32
<212> DNA
<213> Artificial Sequence

<220>

<223> Oligonucleotide designed to act as primer for PCR.

<400> 27
ctcagtctcc agayatcgtg atgacycagt ct 32

<210> 28
<211> 25
<212> DNA
<213> Artificial Sequence

<220>

<223> Oligonucleotide designed to act as primer for PCR.

<400> 28
gggaccaagc tggaaataaa acgga 25

<210> 29

<211> 43
<212> DNA
<213> Artificial Sequence

<220>

<223> Oligonucleotide designed to act as primer for PCR.

<400> 29
ctatgcggcc cagccggcca tgatccagtt ggtgcagtct gga 43

<210> 30
<211> 21
<212> DNA
<213> Artificial Sequence

<220>

<223> Oligonucleotide designed to act as primer for PCR.

<400> 30
cgaggagact gtgagagtgg t 21

<210> 31
<211> 32
<212> DNA
<213> Artificial Sequence

<220>

<223> Oligonucleotide designed to act as primer for PCR.

<400> 31
ctcagtctcc agatgttttg atgacccaaa ct 32

<210> 32
<211> 24
<212> DNA
<213> Artificial Sequence

<220>

<223> Oligonucleotide designed to act as primer for PCR.

<400> 32
ccgtttcagc tccagcttgg tccc 24

<210> 33
<211> 39
<212> DNA
<213> Artificial Sequence

<220>

<223> Oligonucleotide designed to act as primer for PCR.

<400> 33
ctatgcggcc cagccggccc aggttactct raaasartc 39

<210> 34
<211> 21
<212> DNA
<213> Artificial Sequence

<220>

<223> Oligonucleotide designed to act as primer for PCR.

<400> 34
cgcagagaca gtgaccagag t 21

<210> 35
<211> 31
<212> DNA
<213> Artificial Sequence

<220>

<223> Oligonucleotide designed to act as primer for PCR.

<400> 35
ctcagtctcc agacattgtg atgtcacagt c 31

<210> 36
<211> 32
<212> DNA
<213> Artificial Sequence

<220>

<223> Oligonucleotide designed to act as primer for PCR.

<400> 36
gcggccgcct cattcctgtt gaagctcttg ac 32

<210> 37
<211> 43
<212> DNA
<213> Artificial Sequence

<220>

<223> Oligonucleotide designed to act as primer for PCR.

<400> 37
ctatgcggcc cagccggcca tggtgcaggt gaaggagtca gga 43

<210> 38
<211> 21
<212> DNA
<213> Artificial Sequence

<220>

<223> Oligonucleotide designed to act as primer for PCR.

<400> 38

cgaggagacg gtgactgagg t 21

<210> 39
<211> 31
<212> DNA
<213> Artificial Sequence

<220>

<223> Oligonucleotide designed to act as primer for PCR.

<400> 39
ctcagtctcc agayattcag atgaydcagt c 31

<210> 40
<211> 24
<212> DNA
<213> Artificial Sequence

<220>

<223> Oligonucleotide designed to act as primer for PCR.

<400> 40
ccgtttgatt tccagcttgg tgcc 24

<210> 41
<211> 24
<212> DNA
<213> Artificial Sequence

<220>

<223> Oligonucleotide designed to act as primer for PCR.

<400> 41
gacagatggg ggtgtcgttt tggc 24

<210> 42
<211> 26
<212> DNA
<213> Artificial Sequence

<220>

<223> Oligonucleotide designed to act as primer for PCR.

<400> 42
ggaggaacca gttgtatctc cacacc 26

<210> 43
<211> 28
<212> DNA
<213> Artificial Sequence

<220>

<223> Oligonucleotide designed to act as primer for PCR.

<400> 43
agatggatac agttggtgca gcatcagc 28

<210> 44
<211> 29
<212> DNA
<213> Artificial Sequence

<220>

<223> Oligonucleotide designed to act as primer for PCR.

<400> 44
ccatggatca ggtccagctc cagcagtct 29

<210> 45
<211> 52
<212> DNA
<213> Artificial Sequence

<220>

<223> Oligonucleotide designed to act as primer for PCR.

<400> 45
agagccacct ccgcctgaac cgcctccacc tgaggagacg gtgactgagg tt 52

<210> 46
<211> 52
<212> DNA
<213> Artificial Sequence

<220>

<223> Oligonucleotide designed to act as primer for PCR.

<400> 46
ggcggaggtg gctctggcgg tggcggatcg gatattgtga tgacgcaggc tg 52

<210> 47
<211> 28
<212> DNA
<213> Artificial Sequence

<220>

<223> Oligonucleotide designed to act as primer for PCR.

<400> 47
gctagcccgt tttatttcca gcttggtc 28

<210> 48
<211> 29
<212> DNA
<213> Artificial Sequence

<220>

<223> Oligonucleotide designed to act as primer for PCR.

<400> 48
ccatggatca ggttcagctg cagcagtct 29

<210> 49
<211> 53
<212> DNA
<213> Artificial Sequence

<220>

<223> Oligonucleotide designed to act as primer for PCR.

<400> 49
agagccacct ccgcctgaac cgcctccacc tgaggagact gtgagagtgg tgc 53

<210> 50
<211> 30
<212> DNA
<213> Artificial Sequence

<220>

<223> Oligonucleotide designed to act as primer for PCR.

<400> 50
ccatggatga tattgtgatg acgcaggctg 30

<210> 51
<211> 52
<212> DNA
<213> Artificial Sequence

<220>

<223> Oligonucleotide designed to act as primer for PCR.

<400> 51
agagccacct ccgcctgaac cgcctccacc ccgttttatt tccagcttgg tc 52

<210> 52
<211> 51
<212> DNA
<213> Artificial Sequence

<220>

<223> Oligonucleotide designed to act as primer for PCR.

<400> 52
ggcggaggtg gctctggcgg tggcggatcg caggtccagc tccagcagtc t 51

<210> 53
<211> 28
<212> DNA
<213> Artificial Sequence

```
<220>

<223> Oligonucleotide designed to act as primer for PCR.

<400>  53
gctagctgag gagacggtga ctgaggtt                                  28


<210>  54
<211>  51
<212>  DNA
<213>  Artificial Sequence

<220>

<223> Oligonucleotide designed to act as primer for PCR.

<400>  54
ggcggaggtg gctctggcgg tggcggatcg caggtccagc tccagcagtc t        51


<210>  55
<211>  29
<212>  DNA
<213>  Artificial Sequence

<220>

<223> Oligonucleotide designed to act as primer for PCR.

<400>  55
gctagctgag gagactgtga gagtggtgc                                 29
```